(19) Europäisches Patentamt

European Patent Office

Office européen des brevets

(11) **EP 3 680 252 A1**

(12) **EUROPEAN PATENT APPLICATION**
published in accordance with Art. 153(4) EPC

(43) Date of publication:
**15.07.2020 Bulletin 2020/29**

(21) Application number: **17927999.7**

(22) Date of filing: **02.10.2017**

(51) Int Cl.:
***C07K 16/18*** *(2006.01)*

(86) International application number:
**PCT/MX2017/000115**

(87) International publication number:
**WO 2019/070108 (11.04.2019 Gazette 2019/15)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(71) Applicant: **Laboratorios Silanes, S.A. de C.V.
C.P. 03100 Ciudad de Mexico (MX)**

(72) Inventors:
• **GONZÁLEZ CANUDAS, Jorge Alejandro
03100 Ciudad de México (MX)**

• **GARCÍA UBBELOHDE, Walter Jacob
03100 Ciudad de México (MX)**
• **ROSAS ROMERO, Gabriel
03100 Ciudad de México (MX)**
• **RIVERA MARTÍNEZ, Vicente
03100 Ciudad de México (MX)**

(74) Representative: **Arth, Hans-Lothar
ABK Patent Attorneys
Jasminweg 9
14052 Berlin (DE)**

(54) **HIGH-PERFORMANCE PROCESS FOR PRODUCING POISON-ANTIDOTES USING F(AB')2 ANTIBODY FRAGMENTS**

(57) The present invention relates to the field of products of biological origin based on fragmentation of antibodies that are used for passive immunization to neutralize toxins present in poisonous animal venoms, specifically it refers to a biological product where the active ingredient is a combination of $F(ab')_2$ antibody fragments and the process to obtain thereof with a higher yield and better quality in terms of purity and neutralizing capacity of such toxins. Its purification from hyperimmune plasma by salting out without cresol, thermocoagulation and dialysis and applying ultrafiltration techniques with specific parameters is described in order to obtain $F(ab')_2$ fragments with purities greater than 95%.

**EP 3 680 252 A1**

**Description**

**FIELD OF THE INVENTION**

The present invention refers to the field of biotechnology based on the fragmentation of antibodies used in passive immunization vaccines to neutralize toxins present in the venoms of poisonous animals; it specifically refers to a biological product in which the active principle is a combination of $F(ab')_2$ antibody fragments and the process to obtain thereof with high purity and potency.

**BACKGROUND**

Snakebites and scorpion stings are medical emergencies in many countries where these animals are found. The people most affected are agricultural workers and children from food-producing countries. It is hard to estimate the real world-wide incidence of snakebites. It has been reported that there are 5 million snakebites every year, resulting in 2.5 million poisonings, 125,000 deaths and maybe three times the permanent sequels world-wide, (Chippaux, 1998).

The mortality incidence is particularly high in Africa, Asia, South America and New Guinea. In India alone, there can be up to 50,000 deaths by snakebites every year. Most of the people affected by snakebites don't seek treatment in a hospital, they would rather turn to traditional remedies, so the number of accidents and deaths associated is underestimated due to the lack of these records, (Gutierrez, et al. 2006, Fox, et al. 2006).

Some studies have begun to reveal the real burden of the mortality of snakebites, for example, in the oriental Terai region in Nepal, there were 162 deaths by snakebite for every 100,000 inhabitants every year (Sharma, 2004), and in a region in Nigeria, the incidence of snakebites was of 497 for every 100,000 people every year, with a mortality rate of 12.2% (Warrell and Amett, 1976).

A study made in Malumfashi, Nigeria, demonstrated that there were 40-50 cases of snakebites, with 4 deaths for every 100,000 people every year; 19% of the affected developed persistent sequels and only 8.5% sought hospital treatment; in the Kilifi district on the coast of Kenya, 68% of snakebite victims consulted a local "muganga" (medical warlock), only 27% went to the hospital and 36% developed permanent sequels (Pugh, *et al.,* 1980; Snow, 1994).

The global problem is much greater than what has been recorded, besides, the burden of human suffering attributable to these poisonings implies a greater impact to public health. It is estimated that there are 2 million poisonings every year in the Sub Saharan Africa, where most of the victims are children young agricultural workers, many of which acquire permanent physical or psychological consequences; many of the people affected are agricultural workers (food producers) whose families depend on their activity. These accidents have an economic impact on the agricultural communities in these countries. In the Sub Saharan Africa, snake and scorpion poisonings are still an unsolved public health issue due to the crisis in antivenom production.

In North America, human encounters with snakes and scorpions occur, leading to more than 25,000 calls a year to toxicology centers. A significant number of these events will continue to happen world-wide, independently from the geographical practice area. Passive immunization, which refers to the administration of antibodies to protect against infections or toxins, was experimentally proved by various investigators, among which stands out Albert Calmette, who protected rabbits against cobra venom by administering them with antibodies in the form of antiserum and hour before (Calmette, 1896). Passive immunization is nowadays used to treat infections in a successful way, for example, against rabies or Ebola, and also poisoning.

At this point, only Fab and $F(ab')_2$ fragments have been commercialized for passive immunization, although various clinical and preclinical candidates have been generated using scFv, VH or VL human domains, humanized camelids' VHH domains, and IgNAR individual domains. A new antibody fragment scaffold has also been recently constructed from the $CH_2$ domain from a human IgG. Furthermore, various strategies have been used to improve the natural short half-life of antibody fragments, including PEGylation, the use of the repetition of peptide sequences, polysialization, albumin or IgG fusions or bonds, among other approaches.

The functionality of an antibody is defined by the variable chains it contains, regardless of whether or not it contains its constant regions, hence the interest in producing and isolating only the $F(ab')_2$ fragments. On the other hand, the Fc fragment comprises antigenic determinants of the antibody, so that administering complete antibodies generated in some other animal to a patient, generates an immune response against those determinant antigenics, giving rise to various adverse secondary responses that can even be an anaphylactic shock. These problems are significantly reduced by previously digesting the antibodies with papain or pepsin and administering only the resulting purified Fab or $F(ab')_2$ fragments.

The use of Fab or $F(ab')_2$ fragments finds another advantage in what is known as the volume of distribution concept, which is nothing but the volume of the body in which a determined pharmaceutical is dissolved, this volume can refer only to circulating blood, as is the case of the IgGs, or can include a major part of the bodily water in the case of the fragments. Thus, Fab and $F(ab')_2$ fragments by having a higher body volume, they can access to neutralize toxins housed in various tissues, not only in blood, and can even break through the hematoencephalic barrier in both ways and can be used to neutralize or remove neuro-toxins. Particularly, the use of F(ab')$_2$ fragments has an advantage over Fab fragments, in which F(ab')$_2$ fragments have higher retention time in the organism because they have double the molecular weight, apart from conserving the capacity to precipitate the antigen in physiological conditions, they also have a size that allows them to access a distribution volume sufficient for treatment purposes.

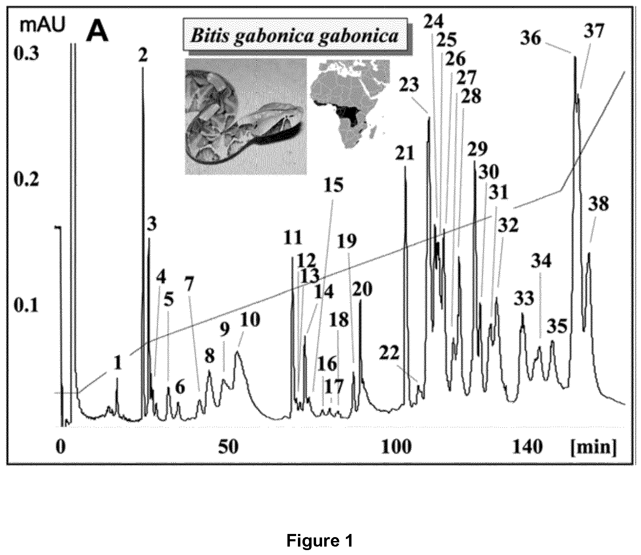

By having the same principal characteristics as antibodies, F(ab')$_2$ fragments can have the same applications as antibodies, with the added advantage of lacking the Fc fragment, which makes it less probable for the patient's organism to recognize them as foreign, having a higher tolerance to their application and reducing the chances of secondary reactions (humoral reaction to the Fc or Arthus reaction and the activation of the complement system), this is particularly useful for prolonged treatments like the ones applied in autoimmune diseases. As is evident, it is important to consider the differences between the venoms of snake species that are remote geographically and phylogenetically in the processes of F(ab')$_2$ fragment purification. Venoms between species differ in effects, so Mitchell and Reichert (1886) found significant differences in the concentrations of globulins and peptones in rattlesnake venoms (*Crotalus adamanteus*), water moccasin (*Agkistrodon piscivorus*) and cobra (*Naja naja*). They also found that these poisons differed in their pharmacological effects and in the changes in the tissues produced. Wolfenden (1886) reported on the chemical differences between cobra and Rusell's viper (*Vipera russellii*) venoms, concluding that they depended on changes in protein molecules. Even today the composition of snake poisons has not been adequately defined, the components of poisons are classified by their pharmacological effects such as: neurotoxins, hemorrhages, cytolysins, etc. It soon became clear that the composition of snake venoms could not be adequately defined by the biochemical techniques of the day, and the venom components were generally classified by their pharmacological effects such as neurotoxins, hemorrhains, cytolysins, etc. As an example, Figure 1 shows the complexity in terms of components that the venom can reach, in this case of the species *Bitis gabonica gabonica.* From the immunogenic properties of snake venom and the development of bacterial antitoxins, the therapeutic antisera of snake venoms were developed on a more or less empirical basis with an imperfect understanding of the nature and complexity of the venoms.

As with the viperine species venoms, scorpion venoms are also a complex mixture of a wide variety of molecules and play an important role in the defense and capture of prey. As Hainen and Gorini da Veiga (2011) have referred, more than 1500 species of scorpions have been identified, each producing a different type of poison; it is estimated that each venom is composed of 50-100 different toxic polypeptides (Lourenço, 1994 and Possani et al., 2000). In contrast to spider and snake venoms, scorpion venom generally shows low levels of enzyme activity (Gwee et al., 2002). They contain mucopolysaccharides, phospholipases, hyaluronidases, protease inhibitors, low molecular weight molecules such as serotonin and histamine, histamine releasing peptides, inorganic salts, mucus and many basic small proteins called neurotoxic peptides (Martin-Eauclaire and Couraud, 1995, Müller, 1993) and Simard and Watt, 1990). Of the approximately 1500 species of scorpions, only a few have been studied, this is an indicator of the need to provide antidotes to meet global demand.

As an example of the complexity, the toxins of the poison of the species *Tityus serrulatus,* (considered to be most dangerous of the South American scorpions and responsible for most of the fatal cases), were analyzed by radioimmunoassays, which show that in addition to their complexity, they have very diverse patterns of antigenic reactivity, even some antigens fail to be detected by sera, which gives us an idea of how complex the immune response can be, De Lima, et al. (1993).

Spider venoms are also a complex mixture of proteins, polypeptides, neurotoxins, nucleic acids, free amino acids, inorganic salts and monoamines that cause various effects. Heinen, A.B. Gorini da Veiga / Toxicon 57 (2011) 497-511 in vertebrates and invertebrates (Jackson and Parks, 1989, Ori and Ikeda, 1998, Schanbacher et al., 1973). For example, funnel web spiders (Atrax robustus), one of the genera with some of the most poisonous species, produce more than 1000 peptides, as revealed by mass spectrometry analysis of their venom. A gross estimate of 500 different toxins for each spider venom would give us a total of 19,000,000 toxins for the approximately 38,000 known spider species. In the field of antivenom design and manufacturing, such biochemical complexity of most venoms is a variable to consider. In the case of obtaining antibody fragments that effectively neutralize complex venoms, it is still necessary to start from antibodies derived from hyperimmune plasma, keeping the greatest amount of such antibodies with their different affinities for the various antigen toxins to have more likeliness to neutralize effectively. Hyperimmune plasma means plasma that contains extremely high levels of antibodies and for this invention it specifically refers to the presence of high concentrations of polyclonal antibodies in the plasma generated by immunization with venoms or derivatives of poisonous species, to immunize, horses or sheep are generally used. The process of the present invention promotes the generation and obtaining of the F(ab')$_2$ fragments of the effectively neutralizing antibodies, although the complexity of the poisons could hinder their generation and separation or fractionation and at the same time obtain them free of molecules that are not F(ab')$_2$ and that are irrelevant or even undesirable (such as pyrogens among other toxins) for the neutralization mechanism. It is important to consider that the use of recombinant (such as scFv) or monoclonal antibodies of other variants of recombinant antibodies is not the best alternative for neutralization of complex venoms (of unknown composition), since it involves the isolation of such venom components (antigens and/or antigenic peptides) to apply molecular biology techniques to obtain specific antibodies that have previously been recognized as effective neutralizers, and from there either the variable domains (VH, VL) or the scFv design, of humanized or hybrid antibodies, etc. These techniques are not ideal for commercial scale production because of their laboriousness and cost, in addition to the loss of large amounts of the antibodies of interest. F(ab')$_2$ polyclonal fragments are still the most efficient to neutralize the toxicity of poisons. It is important to preserve the original polyclonality since many of the truly neutralizing antibodies of toxic activity may be represented in the hyperimmune serum but not in very high concentrations, this occurs in two situations: when the poison is very complex in terms of components and the antigen is not very well represented in terms of concentration and when antigenic determinants are encrypted. When the poison is very complex in terms of components, and toxic antigens are found but in low representation, so a high production of neutralizing antibodies is not achieved. When the antigenic determinants are encrypted and only during the processing of the antigen that is carried out in antigen presenting cells (APCs), within which the antigens are processed starting with their degradation in cytosolic proteasomes (in APCs) random peptides are generated that are presented in the membranes by major histocompatibility complex molecules to cause lymphocyte responses, among these responses is the expansion of memory cells and the generation of high affinity antibodies by the antigen. The responses come to light before the immune system so that the production of very effective antibodies to neutralize the toxic effects will be achieved. The leading immunological response in the generation of high affinity antibodies is that derived from B cells; B lymphocytes are considered as professional antigen presenting cells (APCs) despite their primary role in humoral immunity. It is generally clear that B cells process and present specific and nonspecific antigens differently, the presentation of specific antigen through the B cell antigen receptor occurs with very high efficacy and it is associated with the activation of B cells, resulting in the activation of related T cells. In contrast, the presentation of non-specific antigen by B cells is minimized and dissociated from the activation of B cells. As a result, B cells inactivate T cells that recognize non-specific antigenic epitopes presented by B cells, or induce differentiation or expansion of regulatory T cells. These mechanisms serve to ensure the effective production of high affinity antigen specific antibodies but minimize the production of non-specific antibodies and autoantibodies (Chen and Jensen, 2008). From the polyclonal antibodies that are produced in the hyperimmunized animal, only a percentage is truly neutralizing and during the obtaining of F(ab')$_2$ from plasma a great loss of them occurs, among which the most important will be fragments with low percentage of representation, and it will be for this reason, more likely to be lost, which will consequently lower or lose the ability to neutralize the venom when it comes to neutralizing antigenic determinants or epitopes on which the toxic biological activity of the venom antigen depends. With respect to the state of the art closest to the invention, the most direct antecedent is US Patent 6,709,655, therefore throughout the description an intensive comparative analysis of the purification processes is made. The protein purification techniques described in the state of art such as electrophoresis, chromatographic separation (such as molecular exclusion chromatography, ion and affinity exchange and high performance liquid chromatography (HPLC)), among others, are ideal for obtaining minimal or relatively discrete amounts, in fact, there are countless articles that describe methods of protein purification that can produce from a few micrograms to a few hundred milligrams of very pure protein products. However, these processes are very difficult to scale without implying high production costs, which limits their use for industrial level protein production. Several approaches to antibody production and fragments have been reported in the literature, with affinity for venoms. For example, in patents US 4,849,352 and US 8,048,414 of Sullivan et al, the production of Fab or F(ab')$_2$ fragments is described by digesting antibodies, present in hyperimmune serum, with papain or pepsin immobilized in a matrix of polyacrylamide and the production of F(ab')$_2$ fragments. To purify these fragments, they use a second affinity chromatography where the antigen (the antigens present in the venom) is fixed and thus retain the molecules of F(ab) or F(ab')$_2$ that are attracted by the antigen (venom) immobilized in a polyacrylamide matrix, obtaining Fab or F(ab')$_2$ fragments. Nonetheless the purity with which the required fragments are obtained, for a large-scale commercial production of antibody fragment preparations with the method described in the Sullivan patents is extremely expensive both for the use of immobilized enzymes for digestion and for the use of immobilized antigens for purification. In addition, although an antigenic sieve may be useful for the production of antibody fragments against pure substances, this method is not feasible to purify antibodies against poisons that are mixtures of a large amount and variety of toxins, since it would require excessive time consumption and resources. To be able to isolate all the F(ab')$_2$ molecules capable of neutralizing all the antigens present in the venom, it would be necessary not only to perform multiple chromatographs but to know all the antigens, which to date has not been easy to distinguish, all in regard to complex venoms as commonly occurs with the venoms of snake and scorpion species. With the method of Sullivan *et al* described in patents US 4,849,352 and US 8,048,414 the neutralizing quality of the antivenoms can even be compromised. Another approach that is important to review is that shown in US Patent 5,733,742 in which Landon et al, claims a process for producing Fab fragments from whole blood in a sterile medium, where the whole blood contacts the papain enzyme directly, preferably purified, either free or immobilized, removing cell debris by centrifugation, separating and recovering the fragments to subsequently purify them, preferably by immunoaffinity. Again, Landon uses purified antigens which, unlike venoms, can easily be linked to supports in order to obtain a sieve for the purification of the Fab of interest, and never uses or discusses the method to obtain Fab fragments against antigens that are mixtures of many substances, such as venoms, in addition to working only with papain and chymopapain and does not discuss the possibility of using pepsin. In the reports of Landon and Jones (2002 and 2003) a protocol is described to obtain F(ab')$_2$ fragments from sheep serum that consists of digestion with pepsin in a reaction mixture at pH 3.5 that keeps the pH raised at 6 with a piperazine buffer, centrifuges at 2750 g and removes the precipitate or filter with a fiberglass filter, from this solution separates the low molecular weight components ($\leq$ 13kDa) by means of tangential flow diafiltration in polyethersulfone membrane with a molecular weight cut of 30 kDa, in this step it is not possible to eliminate the pepsin, which is necessary so that no aggregates of the digestion products are formed, so an ion exchange chromatography is carried out, thus removing all acid contaminants including pepsin. With Landon's procedure, no salting out is performed, but the digestion product goes to ultrafiltration. The need to use a chromatographic method to remove the enzyme makes this process difficult to scale to industrial production because of the high costs involved.

Landon J. et al. (2014) also describe a process to purify F(ab')$_2$ and Fab antivenoms from IgG from sheep hyperimmune serum, in order to minimize the degradation of immunoglobulins, all serum proteins are precipitated, including albumin, with caprylic acid, the immunoglobulins are not precipitated, then the digestion to obtain the F(ab')$_2$ or Fab is performed, with the addition of pepsin or papain, respectively. With this method there is also contamination with albumin that is optimized by modifying mixing and temperature times, and degradation products that are removed by diafiltration are also present, the acid impurities present also require removal through an ion exchange column. The use of caprylic acid, either simultaneously with digestion or after digestion, reduces digestion time (from 24 hours to 4 hours under the conditions reported) without reducing production of F(ab')$_2$ too much. An additional approach to producing Fab fragments is that of US Patent 4,814,433 in which Fredrickson discloses a method for obtaining Fab free of papain, since he observes that by digesting the antibodies with this enzyme, hybrid compounds of the papain linked by disulfide bridges with any of the fragments resulting from digestion remain contaminants in the solution, from which later papain can continue digesting and degrading the fragments obtained. To solve the problem, anti-papain antibodies are used, which capture the enzyme's hybrid compounds. Subsequently, the fragments are purified by passing the solution through a column with protein A where the Fc fragments and hybrid compounds are retained. This problem that occurs in digestion with papain has not been reported to occur with pepsin digestion. Some traditional methods involve digestion with pepsin and precipitation of the fraction of the fragments with ammonium or sodium sulfates, but a pre-separation of the antibodies by sulfate precipitation is usually handled first and then the digestion of the antibody fraction. However, with this protocol large losses of biological activity have been reported in the resulting fragments along with a high content of intact antibodies and other contaminants. Other modalities of immunized plasma immunoglobulin processing are described in Chinese patents CN 103864930 and CN 101816789, however, both include chromatographic processes that do not represent a favorable cost-benefit balance. Chinese application CN 103864930 refers to obtaining F(ab')$_2$ fragments of antibodies that resist *Buthus martensii karsch* scorpion venom and is based on obtaining immunized horse plasma with this venom and the fragments are obtained and purified by enzymolysis and salting out, and by exchange column they obtain the active F(ab')$_2$ fragments with high purity, the selected peak is purified by desalination column or by ultrafiltration, sterilized by filtration and finally the product is lyophilized. Chinese application CN 101816789 refers to the obtaining of F(ab')$_2$ fragments of viperidae anti-venom antibodies, it is based on obtaining immunized horse plasma with this venom and immunoglobulins are obtained by salting out, centrifugal dialysis is used, F(ab')$_2$ fragments are subsequently obtained by enzymolysis and purified by hydrophobic column, they are passed through ultrafiltration in a 8000 to 10000 Da membrane or by ultrafiltration by desalination membrane and 0.22$\mu$m sterilization filter, and freeze-dried at the end. Enzymatic digestion in the production of F(ab')$_2$ in itself leads to the loss of activity of some F(ab')$_2$ molecules and therefore to the loss of potency of the resulting F(ab')$_2$ fraction compared to undigested IgG. This loss of activity can be explained by the aggressive low pH conditions necessary for digestion, but also by the nonspecific digestion of IgG that becomes evident with incubation at longer reaction times. Numerous digestion conditions have been described which vary in terms of the type and concentration of enzyme and digestion time, and in the state of art it can be seen that the process is not well standardized. Pepsin is the only enzyme capable of completely cleaving IgG to yield F(ab')$_2$. As reported in the state of art documents, after enzymatic digestion, several stages of purification are normally required by a variety of methods, to ensure high purity and efficacy of products with minimal side effects and minimal loss of the molecules of interest. Purification can be carried out by salting out (saline precipitation), using caprylic acid, ammonium sulfate or sodium sulfate. The simultaneous or consecutive use of thermocoagulation during this stage is also reported in the prior technique. In the process of US Patent 6,709,655 the process of purification of the active principle based on precipitation and dialysis is described and where the thermocoagulation step is also essential and it is mentioned it follows after the first precipitation with ammonium sulfate is finished. The thermocoagulation that is performed at 54° C facilitates the precipitation of serum proteins that are not digested such as albumin and fibrinogen, however these temperatures can also damage a certain percentage of the immunoglobulin fragments that were generated during enzymatic digestion, so by eliminating it, more intact proteins of interest are conserved (F(ab')$_2$ fragments), so eliminating their application represents an outstanding advantage to the technique. Ultrafiltration is a filtration technique that uses membranes to separate different types of solids and liquids (separates particles with a diameter of 0.1 to 0.001 $\mu$m (1,000 to 20,000 Da), by applying a driving force, pressure is always applied, but the concentration and electric potential also influences as a driving force. With ultrafiltration the flow crosses the surface of the membrane in a parallel manner and is not perpendicular as with ordinary filtration, so that an accumulation occurs on the surface of the membrane, thus separating two liquid phases, one depleted in solute (ultrafiltrate or permeate) and one enriched in solute (retained) (Garavito, 1995). On the other hand, diafiltration is a modification of ultrafiltration in which water is added to the feed in order to facilitate the permeate of some components through the membrane, the added water is recirculated in the process so that it reduces the concentration of soluble permeate components and increases the concentration of retained components. On the contrary, nanofiltration, is a membrane filtration process that occurs by the application of pressure, where low molecular weight solutes (approximately in the range of 1000 daltons) are retained, while others, such as salts, can pass totally or partially, through the membrane with the filtrate. Ultrafiltration has become an important technique in the treatment of water and industrial effluents; it can be used directly in the production of drinking water thanks to its ability to retain bacteria and viruses. In some other cases it can be used as pretreatment before nanofiltration or reverse osmosis. In the case of the agri-food industry, it is in the treatment of milk that ultrafiltration has been used in particular for the concentration of whey. In the surface treatment industries, ultrafiltration is used for the regeneration of cataphoresis paint baths used in the automobile industry. Ultrafiltration can also be used to separate oil-in-water emulsions. In the field of biochemistry, ultrafiltration serves to separate and concentrate enzymes, viruses or active ingredients that serve to manufacture vaccines, which is the technical field that concerns to us in the present invention. As it is known in the technique, the operation of ultrafiltration and its variants is a unit operation ideally designed and used for the separation and concentration of particles and not so much of molecular classes; for the most exquisite separation of sizes and molecular types the unit operations that are used in the state of the art are primarily chromatographic techniques, since so far they have been the only techniques that have allowed obtaining high purity for biotechnological or biological products. Regularly, ultrafiltration and its variants are applied in the separation of products of not much added value or where an exquisite separation of molecular fractions is not so relevant (in the dairy industry for example), in order to increase the speed of production, the use of higher flows and pressures to perform the purification operation tends to happen, when such parameters are raised there may not be such a precise separation, but this will not have an important impact on the product consumer's health, however for the separation of molecular fractions in biotechnological products as used for the present invention, the application of ultrafiltration techniques becomes a technical problem that requires experimental design involving an inventive level. In the present invention, ultrafiltration has been redirected in a novel and inventive way towards a high performance process for production, obtaining and purification of antibodies and compositions comprising thereof; particularly, antibody fragments and compositions comprising thereof; more particularly, $F(ab')_2$ fragments and compositions comprising thereof, capable of neutralizing venoms from non-human hyperimmune mammal plasma. The present invention also presents data on the optimal conditions of digestion, that is, where the higher productivity of $F(ab')_2$ is achieved, which also represents a significant advance in production methods based on digestion of antibodies with pepsin. Patent ES 2549690 describes the obtaining of antibody derivatives with a tangential flow diafiltration step, using a 50 kDa membrane for said diafiltration; in addition, judging by the figures of the chromatograms, a degree of purity comparable to that obtained for the present invention is not achieved, as described below; actually, it is not possible to eliminate the low molecular weight components (below 15 kDa) at the same levels as those obtained with the process of the present invention. Said patent does not show evidence of yields or potency data of the antivenoms obtained. With this it is evident that it is not enough to implement any ultrafiltration protocol, but that the design of the protocol and the appropriate parameters is required. Other purification techniques are, of course, chromatographic ones, such as ion exchange chromatography and affinity chromatography, but we already described their disadvantages for the field of the invention. In general, salt precipitation (salting-out), which is also used to separate and purify subsequently by dialysis, is low cost, but results in low antibody recovery; the salting-out process is difficult to scale in sterile conditions and gives poor performance and purity. In addition, it may be associated with large losses in antibody activity and/or aggregate formation. Alternatively, precipitation with caprylic acid may increase the yield of IgG, but the process takes a long time and does not eradicate bacterial contamination producing endotoxins (Landon J. *et al.* (2014)). In the state of art it is also reported that dialysis is effective to purify $F(ab')_2$ but cannot completely remove pepsin, its presence can affect the stability of antibody fragments so to eliminate it ion exchange chromatography is used, as already mentioned (Landon J., *et al* (2003)). The main advantage of ultrafiltration processes over conventional bioseparation processes is the high performance of the product. However, despite the extensive use of ultrafiltration in processes such as diafiltration and concentration, the potential for its use in protein fractionation has not been so widely exploited in the biotechnology industry. This is mainly due to two reasons: one is the lack of understanding of the separation mechanisms involved in ultrafiltration, surprisingly, it is still considered by many to be a mere screening process where size is the only criterion for the separation of the solute. There is a myth that a difference of eight to ten times the size of the solute is essential for the satisfactory separation of the solute by ultrafiltration. But this is not the case since the size of the solute is only one of the many factors responsible for the separation. Solute-solute interactions, solute-membrane interactions, concentration polarization, the predominant mode of solute transport (i.e., convective or diffusive) are among several factors that may be responsible for protein fractionation. Another reason for the low exploitation of these methods is that the research work on the application of ultrafiltration in the area of protein fractionation has been on the separation of simulated protein mixtures; while this has led to a better understanding of protein transport mechanisms and separation, the lack of application-based research has kept ultrafiltration in the blind spot of potential users. The fact is that ultrafiltration processes can be precisely tuned to achieve high productivity and product purity at the same time. The increase in the demand for polyclonal antivenoms implies the development of purification processes of the antibodies and their feasible active fragments on a large scale and efficiently. The production technique, based on the separation or fractionation, which is proposed with the present invention, has a high product yield at low process cost; it is certainly beneficial for the biotechnology industry, particularly in the field of antivenoms based on polyclonal antibodies or on its active fragments. In the present invention experimental development is described working with very complex mixtures of biological origin such as hyperimmune sera (Ghosh, and Cui, 2000) and ultrafiltration is applied based on an exhaustive experimental work, with the invention, it has been possible to optimize the separation of the molecules of interest. For example, the current method for the preparation of $F(ab')_2$ anti-rabies serum approved by the WHO is based on the approach described by Pope (1938; 1939 a; 1939 b) and Harms, briefly: pepsin digestion (30 min, 30° C, pH 3.2) of crude hyperimmune equine plasma is followed by a heat denaturation stage (1h at 55° C, pH 4.3) to precipitate most of non-IgG and by an additional addition of ammonium sulfate (salting-out), to precipitate the IgG including $F(ab')_2$. Due to the salt precipitation stage, the process results in $F(ab')_2$ products of comparatively low yield and low purity. Although patent US 6,709,655 exceeds the quality of the final product, it does not cease to rely on salt precipitation in addition to consuming a lot of time. The final product is also perfectible as we will see later in this description. The objective of the invention is to provide a new process suitable for industrial production of antivenoms, based on the obtaining and digestion of immunoglobulins and subsequent purification of the $F(ab')_2$ fragment of such immunoglobulins, they are obtained from equine crude hyperimmune plasma with the objective of obtaining a better performance than with the process approved by the WHO, and obtaining an active product, more efficient and pure than those obtained by the processes described in the closest state of art (US PAT 6,709,655, of Roodt, AR et al 2010). The development of the process was the result of years of research, described here are: activity tests of $F(ab')_2$ fragments, comparative analysis of productivity or performance, and comparative analysis of purity. A method for obtaining $F(ab')_2$ antibodies from serum or hyperimmune plasma as a source of antibodies, which uses the above principle is described in patents US 8,512,706, US 6,709,655, US 7,485,303 and US 8,075,893, in which the source of antibodies with pepsin is contacted, to subsequently carry out two precipitations with ammonium sulfate and subsequent purification steps. The present invention provides a composition of $F(ab')_2$ antibody fragments with remarkable biological activity and purity. With the process of the present invention, the biological activity and the purity of the final products are increased, in addition, it is also a process of better performance, so that man hours are saved and production costs and times are decreased. During the development of the invention, objectives were set up aimed at eliminating steps that could affect the productivity of the process, likewise work was carried out on the known process by modifying parameters so that productivity and product quality were also increased in both potential and in purity, minimizing the possibility that adverse reactions occur during use as an antivenom.

Another technology where the use of $F(ab')_2$ is intended is described in US Patent 7,537,916 by Jones et. al, where $F(ab')_2$ fragments are produced in a recombinant manner, so for the production of antivenoms the problem of the need for polyclonality or multivalence conservation required to neutralize complex poisons is not resolved. In the closest state of art there are also antivenoms manufactured by the NAVPC company from hyperimmune horse plasma and based on obtaining and purifying the fraction of $F(ab')_2$ fragments of antibodies, which differ in its final properties. Judging by the refrigeration requirement to store it (4 ± 2° C and in the dark), and by the description of their products, these are not lyophilized, which makes them susceptible to being degraded at room temperature and exposed to light. In addition, their products contain a high concentration of cresol (maximum 3.5 mg per vial of 0.5 to 1 ml). About the process for obtaining them, it is reported that immunization begins with horses with increased doses of the venom, obtaining high titer serum and purifying with different stages of saline precipitation and refined by digestion with pepsin, $F(ab')_2$ fragments are purified by adsorbent gels and multistage filtrations followed by dilution for the required potency. These products are displayed as high purity. The general processing steps of these antivenoms indicate that the novelty of the claimed invention is not affected. As can be derived from the background described herein, the present invention contrasts in many elements with the processes and with similar products belonging to the closest state of art. The present invention relates to a high performance process for the production of antivenoms from $F(ab')_2$ fragments of IgG immunoglobulins; its use is an alternative to the use of IgGs, although as mentioned, smaller variants of IgGs have been developed trying to improve their bioavailability, tissue penetrability, and solubility; even so, the polyclonal $F(ab')_2$ fragments are still the most efficient to neutralize the toxicity of venoms, the present invention is due to the complexity of the venoms, which contributes to its effectiveness.

## SUMMARY

In summary, the present invention provides a production and obtaining process of antivenoms based on $F(ab')_2$ antibodies fragments from plasma immunoglobulins, designed to confer passive immunization, neutralizing the toxins of the poisons for which they were manufactured. Moreover, in the present invention the parameters of the unit operation used allow obtaining pure F(ab')$_2$ fragments. In one embodiment, chromatography analysis of the F(ab')$_2$ fragments shows unique peaks of said fragments allowing to observe the absence of other types of fragments or biological material, such as proteins and Fab fragments, which makes this invention unique for obtaining pure F(ab')$_2$ fragments. In another embodiment, the present invention has the advantage of separating and at the same time purifying particles very close in molecular weight using parameters and ranges of working conditions in the unit operation, which otherwise could not be separated or purified using the conditions, parameters and working materials for biological materials, including the pore size of the ultrafiltration membrane, used for the manufacture of antibody fragments. In another embodiment and unlike the prior art, the ultrafiltration process of the present invention also allows to increase the yield of obtaining antibody fragments, particularly, of F(ab')$_2$ antibody fragments. In another embodiment, the high performance process of the present invention makes it possible to obtain and observe by chromatography a single peak of F(ab')$_2$ fragments, whose only presence in the absence of other types of particles and/or contaminating fragments, makes the composition comprising F(ab')$_2$ antibody fragments obtained through the present invention, a composition much more effective than those existing in the state of the art obtained by traditional dialysis and conventional ultrafiltration techniques. Therefore, the high-performance process of production and obtaining novel and inventive antivenoms shown herein allows to purify the F(ab')$_2$ fragments without reducing their potency as demonstrated by the titers and potency of the fragments by traditional routes compared to those obtained by the process described herein. The high yields observed improve effectiveness since it is linked to potency. In another embodiment, the present invention also presents data on optimal digestion conditions, where the highest productivity of F(ab')$_2$ is achieved. The contribution of the obtaining process in the present invention is as important as the quality of hyperimmune plasmas (in terms of concentration of titers). The most direct antecedent of the technology of the present invention is US Patent 6,709,655 (hereinafter Patent 655) whereby an intensive comparative analysis of the process is made throughout the description. The present invention offers a product whose quality is due to the process and is therefore distinctive from the state of art products, they are also an alternative of safe antivenoms and of the quality required by the highest standards.

## BRIEF DESCRIPTION OF THE FIGURES

**Figure 1:** Separation by reverse phase chromatography of the components that comprise the venom of the species Bitis gabonica gabonica. Calvete, JJ et al (2007).

**Figure 2:** Timeline of the development of antibodies-based antivenoms.

**Figure 3: A:** Chromatogram of the supernatant after precipitation with ammonium sulfate (digested antiviperine plasma + process water + pepsin + ammonium sulfate). **B:** Chromatogram of the clarified supernatant (digested antiviperine plasma + process water + pepsin + ammonium sulfate); Clarification I and II.

**Figure 4: Plasma: (a)** Chromatogram of the antiviperine plasma, **(b)** Chromatogram of the prepared plasma (digested antiviperine plasma + process water), Preparation of the plasma. **Digestion: (c)** Chromatogram of the mixture (digested antiviperine plasma + process water + pepsin), End of digestion. **Precipitation: (d)** Chromatogram of the precipitate (digested antiviperine plasma + process water + ammonium sulfate).

**Clarification: (e)** Clarified chromatogram (digested antiviperine plasma + process water + ammonium sulfate). **Ultrafiltration** (4 repetitions): **(f, h, j, l)** Chromatograms of ultrafiltrate I (solution of fragments F(ab')$_2$, Tangential filtration I by 30 kDa cassestte, **(g, i, k, m)** Chromatogram of ultrafiltrate II (solution of fragments F(ab')$_2$, Tangential filtration II by 50 kDa cassestte.

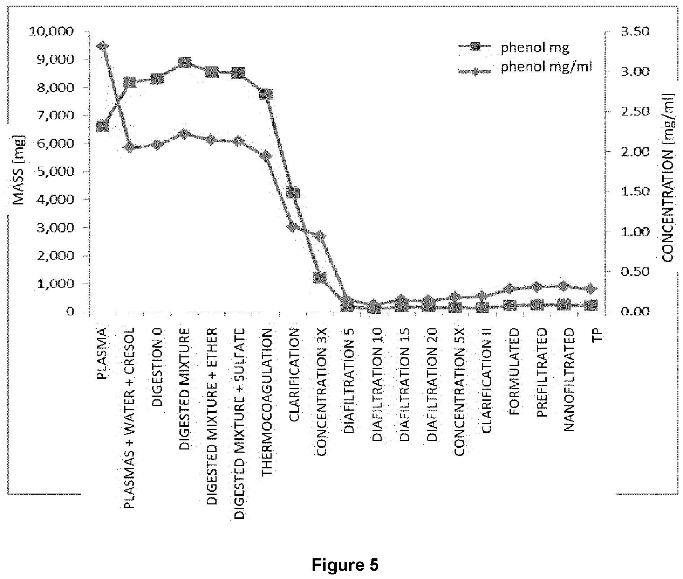

**Figure 5:** Quantification of phenol from antiviperine samples in the different production process stages and obtention of F(ab')$_2$ fragments at pilot plant level from an antiviperine plasma where the hollow-fiber system was applied in the tangential filtration stage with a 30 kDa cut size with washes with process water.

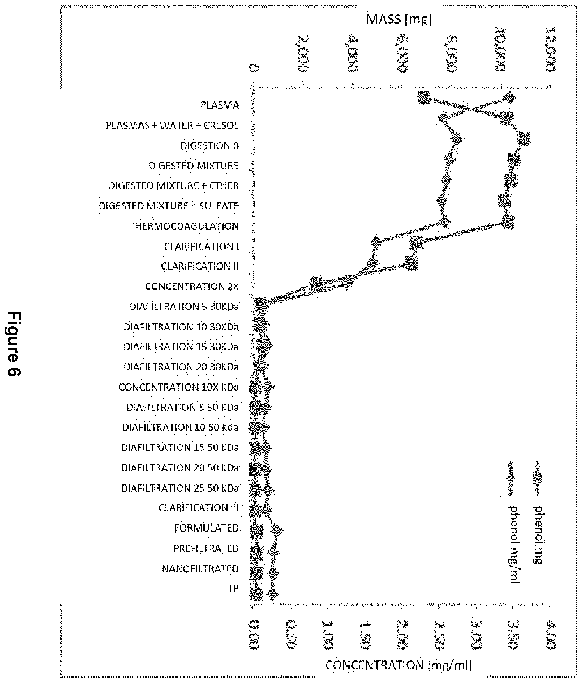

**Figure 6:** Quantification of phenol/cresol in the samples of the different production process stages and obtention of F(ab')$_2$ fragments at pilot plant level from an antiviperine plasma where the cassette system was applied in the tangential filtration stage with a 30 kDa cut size with 0.9% sodium chloride washes and 50 kDa with washes with process water.

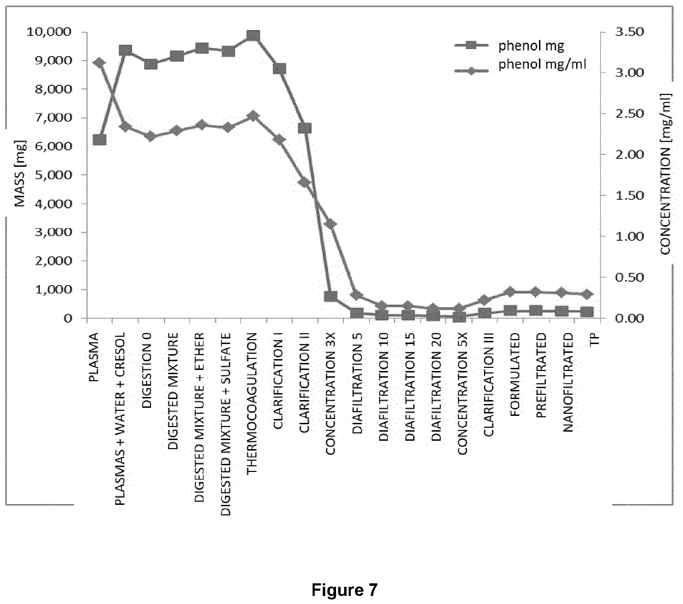

**Figure 7:** Quantification of phenol in the samples of the different production process stages and obtention of F(ab')$_2$ fragments at pilot plant level from an anti-scorpion plasma where the hollow-fiber system was applied in the tangential filtration stage with a 30 kDa cut size with washes with process water (20 washes).

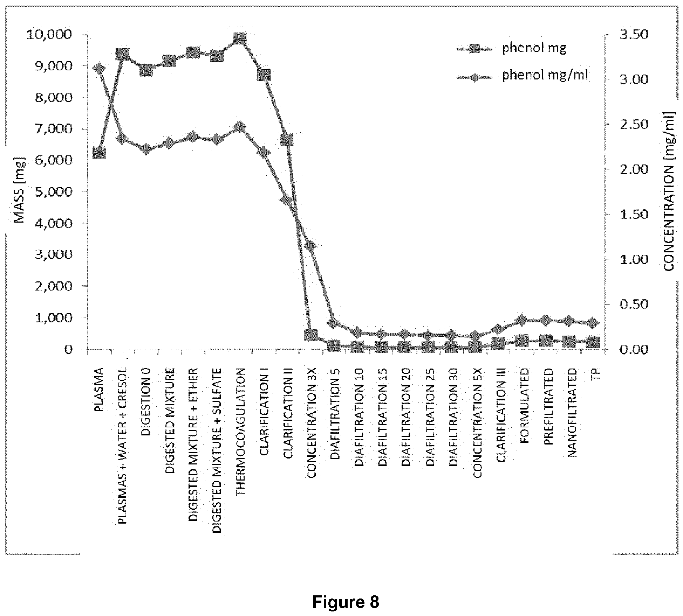

**Figure 8:** Quantification of phenol of the samples of the different production process stages and obtention of F(ab')$_2$ fragments at pilot plant level from an anti-scorpion plasma where the hollow-fiber system was applied in the tangential filtration stage with a 30 kDa cut size with washes with process water (30 washes).

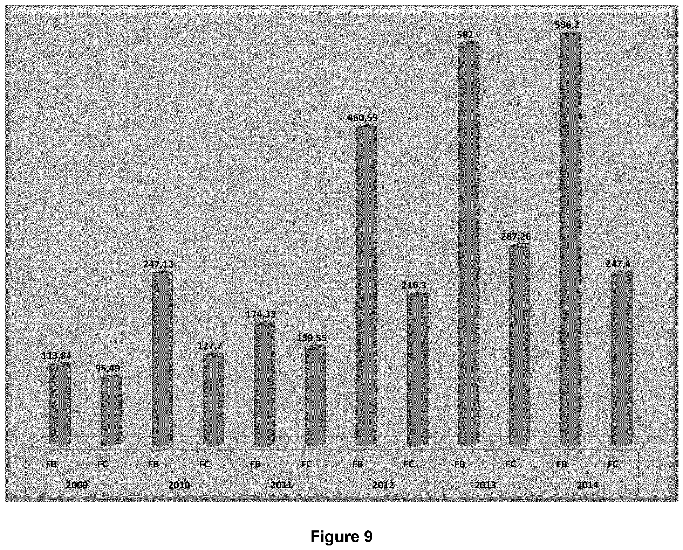

**Figure 9:** Antibody titer in plasma expressed as LD$_{50}$ Neut/ml, 2009 to 2014 annual averages, Viperidae Group I. Induced-bite obtained venoms from two viper species were used. FB: Specific fraction for *Bothrops* and FC: Specific fraction for *Crotalus.*

## DETAILED DESCRIPTION OF THE INVENTION

The increase in the demand for polyclonal antivenoms implies the development of purification processes of the antibodies and their feasible active fragments on a large scale and efficiently. Most of the antivenoms for passive immunization are efficient in their neutralizing capacity, but the production process is considered not only tedious but also long and not efficient enough, in addition, the use of these antivenoms may be associated with adverse effects. Over time the production processes have been improved thanks to advances in the fields of immunization and analytical biochemistry, resulting in more appropriate products in pharmacokinetics and pharmacodynamics, improving their specific activity and reducing their side effects, including anaphylactic reaction.

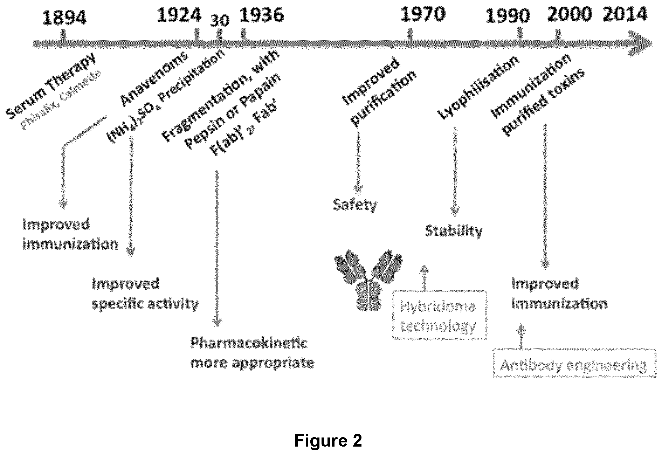

Visualizing the development of antivenoms over time in Figure 2, it is possible to identify that the principles of the technology remain the same as 115 years ago, however, various improvements have been made based on techniques such as antibody fragmentation, protein purification, etc. However, despite the discovery of hybridoma technologies and antibody engineering, there haven't been stronger innovations. At the moment, the development using these techniques has only occurred in scientific research, but they have not reached the market (Alvarenga, et al., 2014) (Williams, et al., 2011).

One of the factors that define the processes to obtain antivenoms with the ability to neutralize them efficiently is to obtain the poisons of the different species as explained below: animal poisons have evolved quickly and effectively together with the defense mechanisms presented by the relationship between prey and predators, poisons provide defense against predators and also help capture prey, which results in a large repertoire of molecules that bind to specific targets, and of which not enough is known yet to design specific neutralizing molecules for all the toxic molecules existing in the mixture of proteins that make up a poison.

The present invention relates to a high-performance process for obtaining an antidote against poisonings caused by poisonous species venoms, based on immunoglobulins of animal origin. The antidotes of the invention are characterized by their efficiency, they neutralize the toxic molecules even those of wide repertoire as required by some venoms and are characterized by their purity as described below.

The immunoglobulins or antibodies from which the antivenoms of the invention are obtained are polyclonal and are of biological origin, derived from hyperimmune plasma, so they need to be fractionated from the plasma and purified; the purity is conferred by the process of the invention and the potency is conferred both by the obtaining process and by the quality of the hyperimmune plasmas from which the total immunoglobulins are processed, said quality is referred to by the antibody titer and its neutralizing capacity.

The quality of hyperimmune plasmas in turn depends on the quality of the venoms used to immunize the horse and it also depends on immunization schemes, of course on the animal's health condition. In Example 1 a basic immunization scheme for carrying out the present invention is described and is illustrative and not limiting.

Commonly the hyperimmune plasma is derived from equines, from the immunoglobulins present therein the F(ab')$_2$ fragments are obtained, and these possess neutralizing capacity of virtually all the toxins present in the venoms. With the process of the invention, a higher yield is obtained since the loss of relevant protein (immunoglobulins processed from hyperimmune plasma) is avoided.

Patent 655 describes a method for obtaining F(ab')$_2$ antibodies from serum or hyperimmune plasma as a source of antibodies that is contacted with pepsin, then two protein precipitations are carried out with ammonium sulfate followed by dialysis and clarification steps. Table 1 describes 17 steps for this method. Under this technique, F(ab')$_2$ antibodies with a remarkable biological activity and purity are obtained, but as we have said, they are improvable characteristics as for example the process. The 17-step process of Table 1, is the direct background of the present invention, currently, the increased quality in the quality of the venoms used to immunize and obtain hyperimmune plasma, and better immunization schemes, have allowed to have plasma with better antibody titers, which encourages the development of new processes that are more efficient in order to obtain F(ab')$_2$ fragments. In our research laboratories, new processing steps have been developed to make antivenom production more efficient. All these changes are reflected in time; data on the quality of the plasma and the way it is processed are presented below, which are reflected in purity, productivity and potency of antivenoms. The approximate manufacturing time using the 17 steps is 52 days. Patent 655 describes, as an alternative to dialysis the use of ultrafiltration, however, this step is only a suggestion without presenting any experimental basis, so from this document it is not possible to anticipate its performance, in addition, experimentation and design of parameters are necessary so that a quality product is obtained from the method and also maximizes the performance. It should be clarified that this enumeration of 17 steps of the process implies one or more unit operations. In order to compare the process of the invention with that of Table 1, the steps were classified so that they could be comparable.

| Table 1: Process to obtain F(ab')$_2$ fragments from plasma or hyperimmune serum *. * Patent US 6,709,655 | |
|---|---|
| 1 | Preparation of pepsin |
| 2 | Mixture of hyperimmune blood plasma in the reactor. * * |
| 3 | Enzymatic digestion with Pepsin at 20-23° C pH 3.2 for one hour with agitation at 15-minute intervals and stopping the reaction when adding NaOH |
| 4 | 1st Precipitation of unwanted proteins. Thermocoagulation, raising the temperature to 54° C. 21% w/v ammonium sulfate. Let stand 30 minutes and then leave the mixture at a temperature from 8 to 4° C for a stand space of 2 hours. |
| 5 | Clarification of the digested mixture (soluble Fraction of F(ab')$_2$) 1 $\mu$ and 0.4 $\mu$, 7-plate system Wastes that contain the precipitate of unwanted protein fragments A partially purified F(ab')$_2$ solution is obtained. |
| 6 | 2nd Precipitation of F(ab')$_2$ from the previous solution. 32-38% w/v ammonium sulfate. Stand time: 12 hrs. |
| 7 | Centrifuged for recovery of the precipitate. |
| 8 | Obtaining paste, (involves dissolving at a stand time from 12 to 20 hours at 2-8° C) |
| 9 | Decantation of the product and removing of sediment containing waste: low molecular weight components and salts |
| 10 | Product clarification (0.2$\mu$) |
| 11 | Dialysis (from 8 to 10 days). |
| 12 | Product clarification (0.2 $\mu$) |
| 13 | Formulation of the product bulk (glycine, sodium chloride, sucrose). |
|  | Nanofiltration of the Product Bulk (50 nm) |
|  | Sterile Terminal Filtration of the Product Bulk |
|  | Filling the Product Bulk |
| 17 | Lyophilization |
|  | Final product (samples are reconstituted by batch for analysis certificates) |

** US Patent 6,709,655 does not mention that phenol, cresol and/or ethyl ether is added to the plasma before its purification processing, but by regulation the use of this type of preservatives is implicit in the process.

Ethyl ether is used in a very low concentration, and it has no relevance to the quality of the final product since it is easily removed during purification, its presence is not required to be specified by regulation.

As detailed below, the process of the invention differs in the process of the closest state of the art in several aspects: With the process of the invention only 12 steps equivalent to 17 of the previous process are applied (see tables 1 and 21). One of the determinations that were made was on the enzymatic digestion process, this as it is appreciated, is carried out with different parameters, the optimal conditions found during the development of the invention indicate that the digestion is optimally performed at a temperature of 37 + 1° C and with a pH of 3.5 + 0.1 for 90 minutes. Another difference that is marked as an object of the invention is that for the processing of hyperimmune plasmas, only one saline precipitation process (or salting out, with ammonium sulfate) is required and this does not require the addition of cresol or ethyl ether, in addition, it is done without standing time, the time it takes for the salt to be dissolved for precipitation to occur is enough and immediately proceed to the next step (Table 21). With respect to ethyl ether, it should be clarified that, it is already used in very low concentration and has no relevance to the quality of the final product since it is easily removed during purification, its presence is not required to be specified by regulation. Another of the differences described and supported for the process of the invention is that during the processing it is possible to remove the thermocoagulation process that is applied during the time the saline precipitation takes place. A marked difference with the process of the invention refers to the incorporation of ultrafiltration (UF); some special embodiments thereof are described below, ultrafiltration replaces the second precipitation with ammonium sulfate and it also replaces the long and risky dialysis process, which also implies a risk of contamination. The type and parameters of ultrafiltration determine the obtained degree of purity (greater than 95%). Among the advantages of applying the aforementioned changes are the yields and potencies that are now increased as explained: by the nature of the new process it is possible to purify in the same series two batches of equine plasma, which before only one could be processed, but regardless of this ease of activities, with the process of the invention, starting from a batch of plasma equivalent in volume, the 12-step process results in a greater number of vials of the final product and with increased potencies; the factors that influenced the higher yields and potencies are described in detail below. The purification process of the invention removes cresol and phenol, thanks to ultrafiltration. Ultrafiltration with the elements and parameters as established in the present invention guarantees the purification, desalination and concentration of the $F(ab')_2$ fragments, all at visibly advantageous levels with respect to what is reported in the prior art. Since the dialysis process described in Table 1, the regulatory acceptable levels of cresol/phenol were already met, when ultrafiltration was applied, the concentration in the finished product was further reduced. The result of this process is to obtain unprecedented yields, an antivenom quality of $F(ab')_2$ with power and purity also unprecedented in its kind. Only by replacing double precipitation and dialysis with the ultrafiltration operation, the total manufacturing time of a finished product is approximately 18 days. As we have mentioned, during the development of the invention, objectives were set to eliminate steps or change steps that ultimately improve the productivity (performance) of the process and the quality of the final product, as well as work on the known process: the one described in US Patent 6,709,655. Modifications were made at all levels, from the modification of parameters to a radical change in the way the material is processed. The combination of everything resulted in a more efficient process and superior product quality in potency, stability and purity, minimizing the possibility that adverse reactions occur during its use as an antivenom. The steps described below independently, corresponding to the process of the invention, are those that report the mentioned advantages. This was analytically tested during experimental trials.

**Removal of the thermocoagulation step that follows the enzymatic digestion, and first Salting-Out and removal of the second Salting-Out**

Thermocoagulation eases the precipitation of serum proteins that are not digested such as albumin and fibrinogen, however, this can damage the immunoglobulin fragments that were generated during enzymatic digestion, thereby eliminating it enables that more proteins of interest are kept intact ($F(ab')_2$ fragments), in this sense, the process of the invention which dispenses with thermocoagulation consequently has better performance. The results differences with and without thermocoagulation and without the second Salting-Out are described in detail below. In the US Patent 6,706,655 (hereinafter 955) it is disclosed that thermocoagulation occurs after digestion when the product is subjected to a temperature of 54° C, for 30 minutes at stand time; the thermocoagulation occurs simultaneously at the first precipitation with ammonium sulfate and then the mixture is passed at a temperature from 8 to 4° C for a period from 2 to 24 hours also at stand time, then the supernatant is recovered by decantation and clarified by passing it through filters of 12, 8 and 4 $\mu$m, this is carried out with the 7-plate system. It is worth mentioning that said system no longer meets the regulatory requirements, at this stage 50% of cresol/phenol was removed. According to the description of the process of patent 955, the clarified supernatant is added again with 35% ammonium sulfate, thus the plasma is subjected to a second precipitation after adjustment of the pH to approximately 6.8, here it is required a stand time for 12 hrs. Subsequently, the soluble fraction is recovered and centrifuged again, the obtained paste is recovered, it contains the $F(ab')_2$ fragments, it is solubilized and allowed to stand from 12 to 20 hours at 2-8° C. The sediment is removed since it contains low molecular weight components and salts, the recovered supernatant is the one that contains the specific $F(ab')_2$ fragments against venom for which it was generated during immunization. The product is clarified at 0.2 $\mu$m and the dialysis process is carried out with a duration of 52 days, and the risk of contamination is also increased. With the process of the invention all these steps, from the thermocoagulation and clarification of the digested mixture to the dialysis, are removed, that is, the thermocoagulation, the second precipitation and the dialysis are no longer carried out, resulting in less loss of the as mentioned, the risk of contamination is decreased, also it implies a considerable saving of time, and hours/man, therefore the production costs also decrease considerably. The following results support these conclusions. It is important to note that in these tests it was not compared with the state-of-the-art process that was described (patent 955), (double saline precipitation and thermocoagulation). The purpose of these tests was to evaluate the effect of the thermocoagulation itself already with the alternative ultrafiltration conditions, so that we compare the operation of the tangential filtration of the hollow-fiber system with a 30 kDa cut size with the tangential filtration system by cassette with a 30 kDa cut size followed by 50 kDa. The cassette is a plate and structure device favored by the ease of scaling from the laboratory to small pilot plants, it is the holder of the ultrafiltration system. The hollow-fiber system is arranged as a module with several small diameter tubes or fibers (0.6 to 2 mm), the solution to filter flows through the open fiber cores and the percolated liquid is collected in a cartridge surrounding the fibers. The HPLC analysis of antiviperine plasma samples from the different stages of a production and obtaining process of $F(ab')_2$ fragments was performed with the application of cresol, ethyl ether and thermocoagulation, making an enzymatic digestion at a temperature from 37° C $\pm$ 2° C and applying the operation of the tangential filtration of the hollow-fiber system with a 30 kDa cut size. Clarifications I and II are described in Example 5. The hollow-fiber UF is technically described in Example 6. On the other hand, HPLC analysis of antiviperine plasma samples from different stages of a production and obtaining process of $F(ab')_2$ fragments at the pilot level was performed, and it was performed without the application of cresol, ethyl ether and thermocoagulation, making an enzymatic digestion at a temperature of 37° C $\pm$ 2° C and replacing the operation of tangential filtration of the hollow-fiber system with a 30 kDa cut size by the cassette system with a 30 kDa-50 kDa cut size. The UF with cassettes is technically described in Examples 7 and 8. The results are shown in Tables 2 and 3.

Table 2. Characterization of the production and obtaining process of $F(ab')_2$ fragments at the pilot level, dispensing with the application of cresol, ethyl ether and thermocoagulation, carrying out an enzymatic digestion at a temperature of 37° C $\pm$ 2° C and replacing the tangential filtration operation of the hollow-fiber system with a 30 kDa cut size by the cassette system with a 30 kDa - 50 kDa cut size.

| STAGE | | SAMPLE - PRODUCT | CODE | PURITY [%] | | PROTEIN | |
|---|---|---|---|---|---|---|---|
| | | | | $F(ab')_2$ | Fab | [mg/mL] | [mg/mL$_{Plasma}$] |
| Plasma | Raw material | Plasma | A050814 | 14.39 | ND | 15.97 | 2.30 |
| Prepared plasma | Plasma preparation | Plasma + Water for injection | B050814 | 14.10 | ND | 27.43 | 7.74 |
| Digestion | Time 0 h | Plasma (digested) + Water for injection + Pepsin | C050814 | 21.60 | 3.42 | 28.89 | 12.48 |
| Digestion | Time 6 h | Plasma (digested) + Water for injection + Pepsin | O050814 | 23.23 | ND | 12.45 | 5.78 |
| Digestion | Stop | Digested mixture (Plasma + Water for injection + Pepsin) | P050814 | 23.63 | ND | 12.35 | 5.84 |
| Precipitation | Salting - out | Digested mixture (Plasma + Water for injection + Pepsin + ammonium sulfate) | Q050814 | 22.25 | ND | 5.57 | 2.48 |
| Clarification I and II | Clarification precipitate | Digested mixture (Plasma + Water for injection + Pepsin + ammonium sulfate) | R050814 | 12.95 | ND | 3.13 | 0.81 |
| Tangential filtration | Concentration - Diafiltration - Concentration | Product in NaCl 9 g/L | C070814 | 97.99 | ND | 25.13 | 4.92 |
| Tangential filtration | Diafiltration | Product in NaCl 9 g/L | C080814 | 98.16 | ND | 18.88 | 3.71 |

Table 3. Characterization of the production and obtaining process of F(ab')$_2$ fragments at the pilot level with the application of cresol, ethyl ether and thermocoagulation, carrying out an enzymatic digestion at a temperature of 37° C $\pm$ 2° C and applying the tangential filtration operation of the hollow-fiber system with a 30 kDa cut size.

| STAGE | | SAMPLE - PRODUCT | CODE | PURITY [%] | | PROTEIN | |
|---|---|---|---|---|---|---|---|
| | | | | F (ab')$_2$ | Fab | [mg/mL] | [mg/mL$_{Plasma}$] |
| Plasma | Raw material | Plasma | A201014 | 13.53 | ND | 88.82 | 12.02 |
| Prepared plasma | Plasma preparation | Plasma + Water for injection + cresol | B201014 | 13.12 | ND | 41.69 | 10.94 |
| Digestion | Time 0 h | Plasma (digested) + Water for injection + cresol + Pepsin | C201014 | 29.04 | 1.66 | 22.14 | 12.86 |
| Digestion | Time 6 h | Digested mixture (Plasma + Water for injection + cresol + Pepsin) | D201014 | 21.34 | 1.54 | 20.37 | 8.69 |
| Digestion | Stop | Digested mixture (Plasma + Water for injection + cresol + Pepsin + ether | E201014 | 20.49 | 1.73 | 23.08 | 9.46 |
| Precipitation | Salting - out | Digested mixture (Plasma + Water for injection + Pepsin + cresol + ether + ammonium sulfate) | F201014 | 27.12 | ND | 7.21 | 3.91 |
| Precipitation | Thermocoagulation | Digested mixture (Plasma + Water for injection + Pepsin + cresol + ether + ammonium sulfate) | G201014 | 18.78 | 0.61 | 4.57 | 1.72 |
| Clarification I and II | Precipitate | Digested mixture (Plasma + Water for injection + Pepsin + cresol + ether + ammonium sulfate) | A211014 | 17.7 | 0.57 | 4.54 | 1.61 |
| Tangential filtration | Concentration | Product in NaCl 9 g/L | G2111014 | 94.65 | 1.55 | 9.68 | 3.66 |

According to the results of the production and obtaining process of F(ab')$_2$ fragments at the pilot plant level, without the application of cresol, ethyl ether and thermocoagulation, making an enzymatic digestion at a temperature of 37° C $\pm$ 2° C and replacing the tangential filtration operation of the hollow-fiber system with a 30 kDa cut size by the cassette system with a 30 kDa - 50 kDa cut size shows obtaining a concentrate with a purity of 98.16% of F(ab')$_2$ and an undetectable level of Fab according to HPLC analysis while in the production and obtaining process of F(ab')$_2$ fragments at the pilot plant level with the application of cresol, ethyl ether and thermocoagulation, making an enzymatic digestion at a temperature of 37° C $\pm$ 2° C and applying the tangential filtration operation of the hollow-fiber system with a 30 kDa cut size shows obtaining a concentrate with a purity of 94.65% of F(ab')$_2$ and a detectable level of 1.55% of Fab. It is important to note that results of the stages of clarification III, formulation, nanofiltration, prefiltration and sterile filtration with respect to the solution of F(ab')$_2$ fragments are not shown since in these stages the purities of F(ab')$_2$ and Fab concentrates are kept constant and the formation of Fab fragments is not promoted either. Examples 11 and 12 describe the processing for these steps.

**Removal of the second precipitation with ammonium sulfate and the dialysis process**

At the end stage of the digestion with pepsin, for the process of the invention this reaction is stopped by adjusting the pH to 4.2 ± 0.2 with 5N NaOH and precipitation is carried out by adding ammonium sulfate while maintaining the stirring. The data in tables 2 and 3 indicate that the fraction of F(ab')$_2$ is now obtained by an ultrafiltration process that is applied immediately after the first and single saline precipitation; the data obtained confirm that with the application of ultrafiltration with the process of the invention the F(ab')$_2$ molecules are purified, without the need to precipitate them with a second salting-out process; this had implications not only in the economy of the process by the simple fact of removing this step but in a better recovery of the molecules of interest.

As for the amount of ammonium sulfate used, the invention proposes to use 21 to 25 kg of ammonium sulfate for a batch of 60 liters of plasma (35% w/v). In contrast, with the 17-step process (double precipitation) for a batch of 60 liters of plasma, 33 to 34 kg of ammonium sulfate was used for each precipitation (55% w/v). As we mentioned, this change not only implies the simple saving of salt, but it is related to the optimization of the solvation process to favor precipitation only of unwanted molecules and simultaneously keep in dissolution F(ab')$_2$ molecules.

As a result of the removal of the second precipitation with ammonium sulfate that was carried out to precipitate the F(ab')$_2$ molecules, the need to dialyze is eliminated; instead of these steps, ultrafiltration systems were used, which in principle it was proposed to use the 30 kDa hollow-fiber format, then the 30 kDa and 50 kDa cassette format was proposed and we were able to make comparisons regarding purity and yields.

Due to the dialysis process, the obtained antivenom batches have resulted in varying degrees of purity, the variability in batches is normal and expected, but the content of F(ab')$_2$ for this 17-step process can have very high percentages of shrinkage, as exemplified in Table 4, from among the indicated batches, it is seen that even the obtained F(ab')$_2$ fraction can be representing only 44% and the low molecular weight components can occupy 55% in the freshly dialyzed fraction.

**Table 4 ANTIVENOMS DERIVED FROM DIALYSIS IN CELLOPHANE MEMBRANE (Patent process, 955)**

ESA: Equine serum albumin
IgGT: immunoglobulin G subtype T
IgG: immunoglobulin G
LMWC: Low Molecular Weight Components
HMWC: High Molecular Weight Components

| ANTIVENOM | BATCH No. | mg/ml | HMWC | F(ab')$_2$ | Fab | LMWC | ESA % ($\leq$) | IgGT % | IgG % | Total IgGs % |
|---|---|---|---|---|---|---|---|---|---|---|
| Anti-arachnid | B-3B-08 | 7.95 | 0.36 | 44.17 | 0.00 | 55.47 | 0.5 | 1.0 | 0.5 | 1.5 |
| Anti-arachnid | B-7L-08 | 13.55 | 1.92 | 74.06 | 5.83 | 18.18 | 0.5 | 1.0 | 0.5 | 1.5 |
| Antiviperine | B-5F-04 | 42.48 | 6.35 | 88.57 | 3.66 | 1.43 | 0.5 | 1.5 | 1.5 | 3.0 |
| Antiviperine | B-0A-06 | 4.28 | 2.30 | 78.87 | 6.67 | 12.16 | 0.5 | 1.5 | 0.5 | 2.0 |
| Antiviperine | B-6L-05 | 14.07 | 4.37 | 90.87 | 2.60 | 2.17 | 0.5 | 0.5 | 0.5 | 1.0 |
| Anti-coral sanke | B-7L-07 | 34.91 | 2.40 | 66.59 | 10.68 | 20.34 | 0.5 | 1.0 | 0.5 | 1.5 |
| Anti-arachnid | B-0H-01 | 7.25 | 1.73 | 92.62 | 2.05 | 3.61 | 0.5 | 1.0 | 5.0 | 6.0 |
| Anti-scorpion | B-0B-03 | 9.38 | 7.32 | 80.18 | 3.28 | 9.22 | 0.5 | 0.5 | 1.5 | 2.0 |
| Anti-coral sanke | B-2D-06 | 30.63 | 2.56 | 77.94 | 10.70 | 8.81 | 0.5 | 0.5 | 0.5 | 1.0 |
| Anti-scorpion | B-0J-08 | 8.21 | 1.77 | 78.48 | 5.01 | 14.74 | 0.5 | 2.0 | 1.0 | 3.0 |
| Anti-scorpion | B-1L-02 | 4.42 | 0.62 | 91.58 | 2.24 | 5.57 | 0.5 | 1.0 | 2.0 | 3.0 |
| Antiviperine | B-2G-06 | 5.34 | 0.74 | 87.82 | 0.00 | 11.44 | 0.5 | 1.5 | 0.5 | 2.0 |

(continued)

| ANTIVENOM | BATCH No. | mg/ml | HMWC | F(ab')$_2$ | Fab | LMWC | % ($\leq$) | % | % | % |
|---|---|---|---|---|---|---|---|---|---|---|
| Antiviperine | B-2G-13 | 8.92 | 0.66 | 77.86 | 0.00 | 21.47 | 0.5 | 1.0 | 0.5 | 1.5 |
| Anti-arachnid | B-4C-15 | 3.65 | 5.77 | 80.94 | 5.40 | 7.89 | 0.5 | 3.0 | 0.5 | 3.5 |
| Antiviperine | B-3A-01 | 18.14 | 0.26 | 67.76 | 0.00 | 31.98 | 0.5 | 1.5 | 0.5 | 2.0 |
| Antiviperine | B-3K-05 | 15.57 | 0.17 | 74.33 | 0.00 | 25.50 | 0.5 | 1.0 | 0.5 | 1.5 |
| Antiviperine | B-4B-11 | 13.14 | 0.67 | 70.81 | 1.01 | 27.51 | 0.5 | 0.5 | 0.5 | 1.0 |
| | AVERAGE | | 2.35 | 77.85 | 3.48 | 16.32 | 0.50 | 1.18 | 1.00 | 2.18 |
| | STANDARD DEVIATION | | 2.26 | 11.85 | 3.51 | 13.59 | 0.00 | 0.64 | 1.13 | 1.25 |
| | VARIATION COEFFICIENT (%) | | 96.19 | 15.22 | 100.84 | 83.25 | 0.00 | 54.05 | 113.19 | 57.40 |
| | MIN | | 0.17 | 44.17 | 0.00 | 1.43 | 0.50 | 0.50 | 0.50 | 1.00 |
| | MAX | | 7.32 | 92.62 | 10.70 | 55.47 | 0.50 | 3.00 | 5.00 | 6.00 |

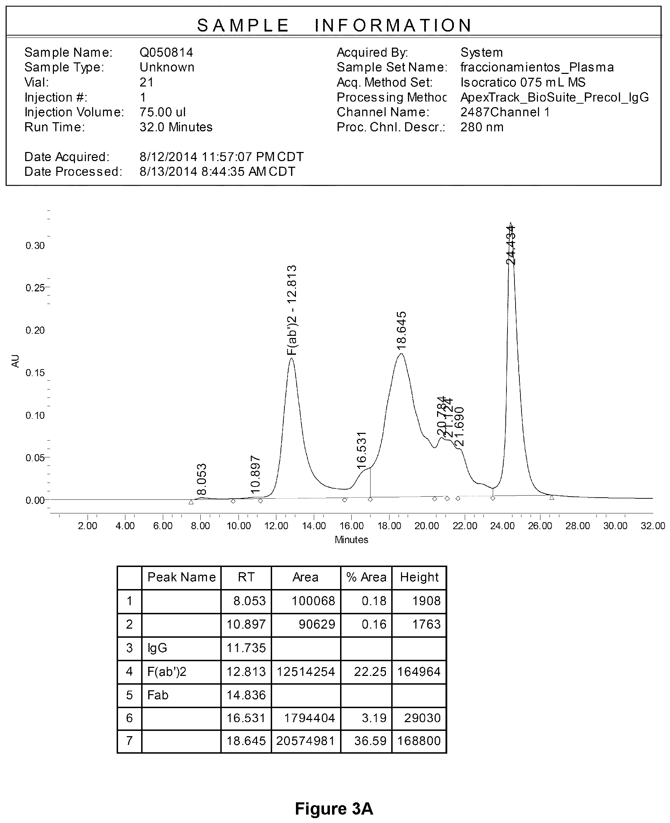

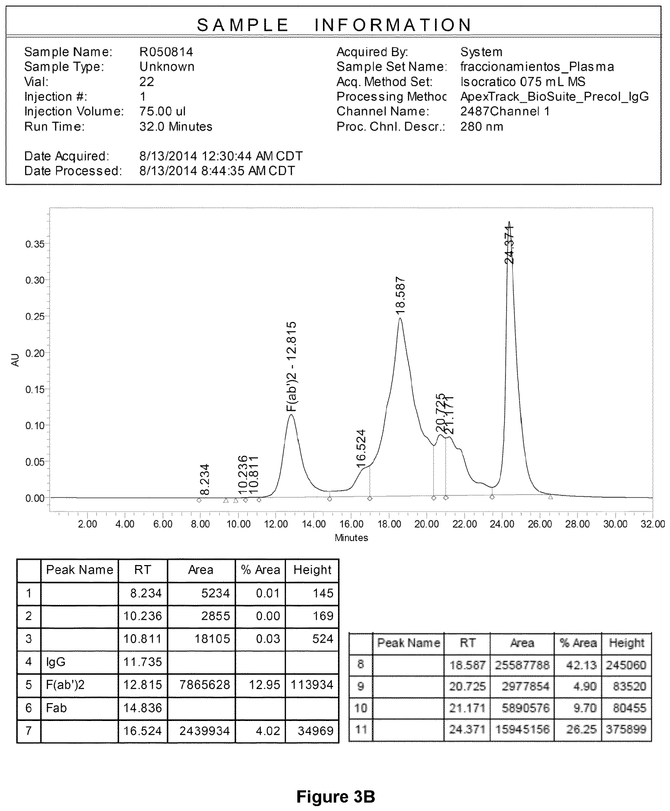

For the process of the invention, precipitation with $(NH_4)_2SO_4$ (Salting-out) implies precisely the saline precipitation of unwanted proteins, albumin and hemoglobin that are in high concentration precipitate, in the supernatant F(ab')$_2$ molecules are still soluble, so that once the ammonium sulfate is solubilized, the entire mixture is immediately subjected to the clarification step (I and II). The first one is carried out in a membrane with a pore from 8.0 to 20.0 $\mu$m, the second clarified is carried out with a 0.2 $\mu$m nominal pore membrane. The clarification step I and II removes large particles and helps for a better ultrafiltration performance, ensuring that the membranes are not damaged. It is important to note that, according to the observations when working with the process of the invention, it is necessary that salting out and clarification I and II be carried out immediately. In the 17-step process, it is even recommended the stand time of the mixture to promote precipitation (a stand period of two hours in the first precipitation and 12 hours in the second), but we have verified that these stand periods far from benefit the yield, according to our observation, the stand times cause loss or decline of F(ab')$_2$. An HPLC chromatogram of the sample of a plasma batch for the antiviperine product, digested with pepsin and precipitated with ammonium sulfate before and after clarification I and II is presented in Figure 3A. In the 17-step process the clarification of the newly digested mixture was carried out with the 7-plate system, with the process of the invention these clarifications I and II are used with an interval from 8 to 20 $\mu$m, an HPLC chromatogram of this step is shown in figure 3B for a sample of an antiviperine product batch. As we have mentioned, the 7-plate system is currently not acceptable in terms of regulations as it has a very high potential for contamination of the filtrates. After clarification I and II, ultrafiltration continues.

**Ultrafiltration or Tangential Filtration with hollow-fiber**

In the patent process 955 after obtaining the tablet of F(ab')$_2$ a long dialysis process is continued that involved at least 8 to 10 days for a 60-liter plasma batch. With the process of the invention this step is no longer necessary and consequently the series of steps derived from said dialysis process are saved: the recovery of the soluble fraction, its centrifugation to concentrate the soluble fraction and then dissolve at stand time during 12 to 20 hrs and decant; the precipitate had to undergo another clarification by 0.2 $\mu$m and then proceeded to dialysis that involved 40 days per batch only for dialysis and a third clarification of the dialysate by 0.2 $\mu$m. During the development of the invention, ultrafiltration or tangential filtration with hollow-fiber of 30 kDa was implemented; it saves the second precipitation and the steps described for dialysis. As proof of the performance of the ultrafiltration process, the results of 2 antivenom batches (batches B-3K-21 and B-3K-11) both processed with tangential filtration with 30 kDa hollow-fiber are presented as examples (see tables 5 and 6). These tables correspond to the analysis certificates for such batches, where the effects of automated ultrafiltration using 30 kDa hollow-fiber filtration can be compared.

**Table 5: DATA OF THE ANALYSIS CERTIFICATE FOR ANTI-SCORPION POLIVALENT FABOTHERAPY BATCH B-3K-21**
**30 kDa Hollow-Fiber Filtration**

Finished product. Code Number: 100152
**Amount: 26 536 Pieces**

(continued)

| Determinations | Specifications | Results |
|---|---|---|
| Description of the lyophilized | Powdery appearance or of a porous, fragile solid, white to light yellow. | Fragile porous white solid. |
| Description of the reconstituted | Clear or slightly opalescent and colorless or slightly yellow liquid. Free of foreign particles in suspension. Diluent: B3E11. | Slightly opalescent and colorless liquid. Free of foreign particles in suspension. |
| Identity tests | | |
| Identity of origin | Meets the specifications of Identity Test corresponding to the species of origin of the serum. | Comply |
| Specific identity | Specifically neutralizes the venom of scorpions of the *Centruroides* genus. | Comply |
| Specific identity (ELISA method) | It is determined by recognition of specific antigens compared to a negative control. | Comply |
| Sterility | Meets the requirements | Comply |
| Safety | At the end of the test period the animals must survive without presenting significant signs of toxicity or weight loss. | Comply |
| Pyrogens | The sum of the temperature increase of three rabbits should not be greater than 1.2° C. | 0.2° C |
| Potency | No less than 150 $LD_{50}$ neutralizers of scorpion venom | 219,57 $LD_{50}$ Neu/Fco |
| Moisture | Maximum 3.0% (m/v) | 1.7420 % |
| Proteins * | Not more than 10.0 % (m/v) | 0.171 % |
| Osmolarity | Minimum 240 mOsmol / kg after dilution when applying | 733 mOsmol/kg |
| Purity and molecular size distribution (by molecular exclusion HPLC) | | |
| F(ab')$_2$ | Not less than 90% | 91.28 % |
| LMWC | No more than 10% (According to the Mexican Pharmacopeia). | 4.92 % |
| Purity (by SDS Page in reducing conditions) | | ns) |
| Albumin | Not more than 0.5 % | < 0.5 % |
| IgG | No more than 5.0 % | < 5.0 % |
| pH | 6.0 and 7.0 at 25° C $\pm$ 2° C | 6.58 to 24.8° C |
| Cresol | No more than 0.41 mg/vial | 0.0055 mg/vial |
| Reconstitution | No more than 35 s | 11.15 s |

**Table 6: DATA OF THE ANALYSIS CERTIFICATE FOR ANTI-SCORPION POLIVALENT FABOTHERAPY. BATHC B-3K-11**
**30 kDa Hollow-Fiber Filtration**

Finished product. Code Number: 100152
Manufacturing date: F.I. 27/Feb/13. F.T. 10/Ene/14, F.L.L. 23/Oct/13.
Expiration Date: Oct/17.
**Amount: 27 277 Pieces**

(continued)

| Determinations | Specifications | Results |
|---|---|---|
| Description of the lyophilized | Powdery appearance or of a porous, fragile solid, white to light yellow. | Fragile porous white solid. |
| Description of the reconstituted | Clear or slightly opalescent and colorless or slightly yellow liquid. Free of foreign particles in suspension. Diluent: B3E11. | Slightly opalescent and colorless liquid. Free of foreign particles in suspension. |
| Identity tests | | |
| Identity of origin | Meets the specifications of Identity Test corresponding to the species of origin of the serum. | Comply |
| Specific identity | Specifically neutralizes the venom of scorpions of the *Centruroides* genus. | Comply |
| Specific identity (ELISA method) | It is determined by recognition of specific antigens compared to a negative control. | Comply |
| Sterility | Meets the requirements | Comply |
| Safety | At the end of the test period the animals must survive without presenting significant signs of toxicity or weight loss. | Comply |
| Pyrogens | The sum of the temperature increase of three rabbits should not be greater than 1.2°C. | 0.3°C |
| Potency | No less than 150 $LD_{50}$ neutralizers of scorpion venom | 217.41 $LD_{50}$ Neu/Fco |
| Moisture | Maximum 3.0% (m/v) | 1.7190 % |
| Proteins * | Not more than 10.0 % (m/v) | 0.210 % |
| Osmolarity | Minimum 240 mOsmol / kg after dilution when applying | 757 mOsmol/kg |
| Purity and molecular size distribution (by molecular exclusion HPLC) | | |
| $F(ab')_2$ | Not less than 90% | 97.88 % |
| LMWC | No more than 10% (According to the Mexican Pharmacopeia). | 0.25 % |
| Purity (by SDS Page in reducing conditions) | | |
| Albumin | Not more than 0.5 % | < 0.5 % |
| IgG | No more than 5.0 % | < 3.0 % |
| pH | 6.0 and 7.0 at 25° C $\pm$ 2° C | 6.81 at 25.2 °C |
| Cresol | No more than 0.41 mg/vial | 0.168 mg/vial |
| Reconstitution | No more than 35 s | 11.52 s |

To compare the advantages of the ultrafiltration implementation, the result of 2 batches of antivenoms (anti-scorpion batches B-2C-17 and B-3J-011) both processed by the dialysis method (17 steps) described in the US patent 6,709,655 are also presented as an example, (see tables 7 and 8).

**Table 7: DATA OF THE ANALYSIS CERTIFICATE FOR ANTI-SCORPION POLIVALENT FABOTHERAPY BATCH B-2C-17 PROCESSED BY DIALYSIS**

Finished product. Code Number: 100341

(continued)

**Table 7: DATA OF THE ANALYSIS CERTIFICATE FOR ANTI-SCORPION POLIVALENT FABOTHERAPY BATCH B-2C-17**

**PROCESSED BY DIALYSIS**

Manufacturing date: F.I. 15/Jun/11. F.T. 19/Abr/12, F.L.L. 28/Mar/12.

Expiration Date: Mar/14

**Amount: 1 670 Pieces**

| Determinations | Specifications | Results |
|---|---|---|
| Description of the lyophilized | Powdery appearance or of a porous, fragile solid, white to light yellow and free of foreign particles | Fragile porous white solid. Free of foreign particles. |
| Description of the reconstituted | Clear or slightly opalescent and colorless or slightly yellow liquid. | Slightly opalescent and colorless liquid. |
| Specific identity (ELISA method) | Meets the requirements | Comply |
| Pyrogens | Meets the requirements | 0.6° C |
| Potency | No less than 150 $LD_{50}$ neutralizers of scorpion venom | 217.19 $LD_{50}$ Neu/vial |
| Moisture | Maximum 3.0% (m/v) | 0.8702 % |
| Proteins | Not more than 120 mg/vial | 9.336 mg/vial |
| Sulfates | Not more than 1.7 mg/vial | < 1.7 mg/vial |
| Sucrose | 4.3 to 38.3 mg/vial | 17 mg/vial |
| Sodium Chloride | 45-80 mg/vial | 78 mg/vial |
| Borates | Not more than 1.0 mg/vial | 0.368 mg/vial |
| Glycine | 6.6 to 94.9 mg/vial | 64 mg/vial |
| Osmolarity | 240-800 mOsmol/kg | 688 mOsmol/kg |
| Purity and molecular size distribution (by molecular exclusion HPLC) | | |
| F(ab) | Not more than 7% | ND |
| F(ab')$_2$ | No less than 85% | 96.51 % |
| LMWC | Not more than 3% | 1.30 % |
| HMWC | Not more than 5% | 2.46 % |
| Purity (by SDS Page in reducing conditions) | | |
| Albumin | Not more than 0.5 % | < 0.5 % |
| IgG | Not more than 5.0 % | < 3.0 % |
| pH | 6.0 and 7.0 at 25° C $\pm$ 2° C | 6.81 at 25.2° C |
| Cresol | Not more than 0.41 mg/vial | 0.168 mg/vial |
| Reconstitution | Not more than 35 s | 11.52 s |

**Table 8: DATA OF THE ANALYSIS CERTIFICATE FOR ANTI-SCORPION POLIVALENT FABOTHERAPY BATCH B-3J-01**
**PROCESSED BY DIALYSIS**

Finished product. Code Number: 100341

Manufacturing date: F.I. 03/Jul/13. F.T. 03/Sep/13, F.L.L. 24/Sep/13.

Expiration Date: Sep/15.

(continued)

**Table 8: DATA OF THE ANALYSIS CERTIFICATE FOR ANTI-SCORPION POLIVALENT FABOTHERAPY BATCH B-3J-01**

**PROCESSED BY DIALYSIS**

**Amount: 1 648 Pieces**

| Determinations | Specifications | Resultados |
| --- | --- | --- |
| Description of the lyophilized | Powdery appearance or of a porous, fragile solid, white to light yellow. | Fragile porous white solid. |
| Description of the reconstituted | Clear or slightly opalescent and colorless or slightly yellow liquid. Free of foreign particles in suspension. | Slightly opalescent and colorless liquid. Free of foreign particles in suspension. |
| Specific identity (ELISA method) | Meets the requirements | Comply |
| Pyrogens | Meets the requirements | 0.2° C |
| Potency | No less than 150 $LD_{50}$ neutralizers of scorpion venom | 219.67 $LD_{50}$ Neu/vial |
| Moisture | Maximum 3.0% (m/v) | 2.0131 % |
| Proteins | Not more than 120 mg/vial | 5.28 mg/vial |
| Sulfates | Not more than 1.7 mg/vial | < 1.7 mg/vial |
| Sucrose | 4.3 to 38.3 mg/vial | 32.84 mg/vial |
| Sodium Chloride | 45-80 mg/vial | 66.56 mg/vial |
| Borates | Not more than 1.0 mg/vial | < 0.0012 mg/vial |
| Glycine | 6.6 to 94.9 mg/vial | 75.70 mg/vial |
| Osmolarity | 240-800 mOsmol/kg | 764 mOsmol/kg |
| Purity and molecular size distribution (by molecular exclusion HPLC) | | |
| ND | Not more than 7% | 2.86 % |
| $F(ab')_2$ | No less than 85% | 91.32 % |
| LMWC | Not more than 3% | 1.30 % |
| HMWC | Not more than 5% | 3.15 % |
| Purity (by SDS Page in reducing conditions) | | |
| Albumin | Not more than 0.5 % | < 0.5 % |
| IgG | Not more than 5.0 % | < 5.9 % |
| pH | 6.0 and 7.0 at 25° C $\pm$ 2° C | 6.68 at 25.4° C |
| Cresol | Not more than 0.41 mg/vial | 0.1392 mg/vial |
| Reconstitution | Not more than 35 s | 11.38 s |
| Pepsin | Not more than 160 ng/vial | 9.91 ng/vial |

The potency data included in Tables 5 to 8 corresponds to that adjusted to meet the average neutralizing doses required per vial or bottle, which is why they do not differ greatly from each other; therefore, to compare the potencies, the potency data corresponding to the same 4 batches are included in tables 9 and 10, but just purified, that is, before the setting of the potencies; the 4 batches come from the same volume of plasma. The data in tables 9 and 10 corresponds to processing of the same volume of plasma, 60 liters per batch (that is, for each of the batches: B-3K-21, B-3K-11, B-2C-17 and B-3J-01).

**Table 9. Anti-scorpion batches processed by dialysis (17 steps of table 1)**

| Batches of finished product | Concentrate | Potency of the concentrate $LD_{50}$Neut/mL | Plasma liters | Number of pieces (for 60 liters of plasma) |
|---|---|---|---|---|
| B-2C-17 | B-1F-06 | 110.64 | 60 | 1,670 |
| B-3J-01 | B-3G-02 | 156.7 | 60 | 1,648 |

**Table 10. Anti-scorpion batches processed by saline precipitation and ultrafiltration with 30 kDa hollow-fiber**

| Batches of finished product | Concentrate (Batches comprising it) | Average potency of the concentrate $LD_{50}$Neut/mL | Average plasma liters | Number of pieces (for 60 liters of plasma) |
|---|---|---|---|---|
| B-3K-21 | B-3C-08 B-3C-09 | 698.42 | 60 | 25,631 |
| B-3K-11 | B-3B-27 B-3B-25 | 699.23 | 60 | 27,061 |

Regarding the comparison of yields, tables 9 and 10 reiterate the number of pieces obtained and are comparable since, as mentioned, they are obtained from the processing of the same volume of plasma; this time the data has been averaged to ease the comparison. The difference in the number of pieces (vials) obtained is very remarkable, 25,631 and 27,061 pieces (average 26,346) for the process with 30 kDa hollow-fiber ultrafiltration, against 1,670 and 1,648 pieces (average 1,659) for the dialysis process, so the process for obtaining antivenoms is more than 15 times more productive, or at least 15 times more productive. It is important to note that these data from 4 batches are presented as examples, but the results have been consistent as can be demonstrated in the batch production records obtained from years of manufacture. Likewise, the details of the procedures have been kept confidential and have not been exposed to publications. In the analysis certificates it will be seen that the data of the high molecular weight components HMWC and Fab are no longer recorded in the batches of the invention, since by the process they reach not detectable levels by high performance liquid chromatography (HPLC). The $F(ab')_2$ and the low molecular weight components (LMWC) must be not less than 90% and not more than 10% respectively, this is according to the guidelines of the Mexican Pharmacopoeia (FEUM[0]). The response by pyrogens is comparable and is also in accordance with the pharmacopeia requirements (FEUM 2011[1]). The potency is comparable in all batches of tables 5 to 8, since this is required for regulatory purposes, this potency is adjusted to not less than 150 $LD_{50}$ of scorpion venom per bottle (FEUM 2011[2]) and is the potency of the finished product; however, to compare the differences in the potencies obtained by virtue of both processes, the potency data must be considered before such adjustment, that is, the potency data of the concentrated product, which is when it has just been obtained from the obtaining process. Tables 9 and 10 show these data and the differences are remarkable: For anti-scorpion batches B-3K-21, B-3K-11 (obtained with the method of the invention), the potencies of the concentrated product were 698.42 and 699.23 $LD_{50}$Neut/mL respectively. For anti-scorpion batches B-2C-17, B-3J-01 (obtained with the method of 17 steps) the potencies of the concentrated product were 110.64 and 156.7 $LD_{50}$Neut/mL. Judging by the examples presented, the potency is increased by at least 6-fold. Another indirect way to see the differences in potencies is seen by comparing the amount of specific protein in the Finished Product (after adjusting the lethal neutralizing dose). The information in tables 5 to 8 handles different units for protein concentration (percentages or in mg/vial), so that the concentrations of protein in mg/vial for the 4 batches of the finished anti-scorpion product are shown in Tables 11 and 12. It is noteworthy that more protein is required when processing by 17 steps, indicating that the process of the invention has greater performance.

Table 11: Anti-scorpion batches produced by double precipitation and dialysis (17 steps from table 1)

| BATCH | PROTEIN AMOUNT |
|---|---|
| B-2C-17 | 9.336 mg/Vial |
| B-3J-01 | 5.28 mg/Vial |

(continued)

| Table 12: Anti-scorpion batches produced by a single precipitation and UF with 30 kDa hollow-fiber | |
|---|---|
| BATCH | PROTEIN AMOUNT |
| B-3K-21 | 3.83 mg/Vial |
| B-3K-11 | 4.83 mg/Vial |

To finish characterizing the implementation of the ultrafiltration by 30 kDa hollow-fiber, in table 13 the levels of the different components are shown, the degree of purity of $F(ab')_2$ is at an average of 95.35 in this representative sample of antivenom batches.

**Table 13. Analysis of the results corresponding to the TANGENTIAL FILTRATION CHARACTERIZATION PROTOCOL (HOLLOW-FIBER SYSTEM WITH A 30 kDa CUT SIZE) APPLIED TO THE PRODUCTION AND OBTAINING PROCESS OF F(ab')2 FRAGMENTS.**

| Code | Purity % | | Protein | | $\tau$ (PA) |
|---|---|---|---|---|---|
| | $F(ab')_2$ | Fab | mg/ml | mg/ml plasma | |
| A061014 | 97.06 | 2.38 | 9.11 | 3.54 | 3.88 |
| A071014 | 97.30 | 2.03 | 12.84 | 5.00 | 5.17 |
| B071014 | 96.52 | 2.64 | 13.72 | 5.30 | 3.88 |
| A081014 | 96.08 | 2.43 | 16.15 | 6.21 | 3.88 |
| A151014 | 98.68 | ND | 19.52 | 7.70 | 3.88 |
| G211014 | 94.65 | 1.55 | 9.68 | 3.66 | 3.88 |
| 1311014 | 87.16 | 4.38 | 8.27 | 2.88 | 3.88 |
| AVERAGE | 95.35 | 2.57 | 12.76 | 4.90 | 4.06 |

**Tangential ultrafiltration with cassettes**

Specifying in the fact of replacing the tangential filtration operation of the hollow-fiber system with a 30 kDa cut size by tangential filtration with cassette with a 30 kDa and 50 kDa cut size, the purpose is to obtain a product with a higher purity without loss of potency, during the development of the invention we found the range of parameters required to avoid protein fragmentation so that a more efficient process was achieved, by minimizing the loss of $F(ab')_2$ proteins. The important thing is not in the unit operation itself, but in the ranges in the specific parameters found, which allow a higher degree of purity, so much that there is no detectable Fab, that is, the change to cassettes also implied a change of difference of low pressure since the differences of higher pressure supposes the damage of the proteins, (mostly irrelevant) that generate fragments which can be confused with Fab by molecular weight, but it was possible to obtain the appropriate pressure and flow parameters, which are managed in relatively low ranges to avoid the formation of Fab fragments. The shear stress Tau ($\tau$) parameter is the value that we present as a determinant to achieve a purity of 95% (Ahrer, et al 2006). Furthermore, with ultrafiltration separation only with 30 kDa or only with 50 kDa, Fab removal is not achieved. With the purpose of contrasting the processes, in table 14 we present purity data obtained using the ultrafiltration system with the option of 30 and 50 kDa cassettes, with which we verify that 95% purities are achieved on average. Table 14 shows 8 tests passing the sample through 30 kDa (a sample code is given) and consecutively through 50 kDa (another sample code is given). It is noteworthy that the removal of F(ab) is definitely achieved after filtration by 50 kDa. The parameters of feed flow, Flux, tangential pressure and $\Delta$P were used as appropriate.

**Table 14: analysis of the results corresponding to the tangential filtration characterization protocol (cassette system with a 30 kDa and 50 kDa cut size) applied to the production and obtaining process of F(ab')2 fragments. ND: Not Detectable**

| ASSAY | CUT SIZE kDa | Purity % Protein | | | | τ (Pa) |
|---|---|---|---|---|---|---|
| | | F(ab')$_2$ | Fab | mg/ml | mg/ml plasma | |
| 1 | 30 | 89.69 | 4.58 | 11.74 | 2.11 | 2.05 |
| | 50 | 96.3 | ND | 12.96 | 2.5 | 0 |
| 2 | 30 | 96.19 | 1.73 | 5.59 | 1.77 | 2.05 |
| | 50 | 97.48 | ND | 1.96 | 0.64 | 0 |
| 3 | 30 | 97.99 | ND | 25.13 | 4.92 | 2.05 |
| | 50 | 98.16 | ND | 18.88 | 3.71 | 0 |
| 4 | 30 | 99.3 | ND | 14.37 | 2.85 | 2.05 |
| | 50 | 99.31 | ND | 12.05 | 2.39 | 0 |
| 5 | 30 | 99.01 | ND | 9.74 | 1.93 | 2.05 |
| | 50 | 99.13 | ND | 9.05 | 1.79 | 0 |
| 6 | 30 | 99.06 | ND | 15.51 | 3.07 | 2.05 |
| | 50 | 99.12 | ND | 13.6 | 2.7 | 0 |
| 7 | 30 | 97.58 | 0.95 | 34.74 | 6.78 | 2.05 |
| | 50 | 98.83 | ND | 15.81 | 3.13 | 0 |
| 8 | 30 | 91.98 | 3.26 | 14.41 | 2.65 | 2.05 |
| | 50 | 98.68 | ND | 12.09 | 2.39 | 0 |

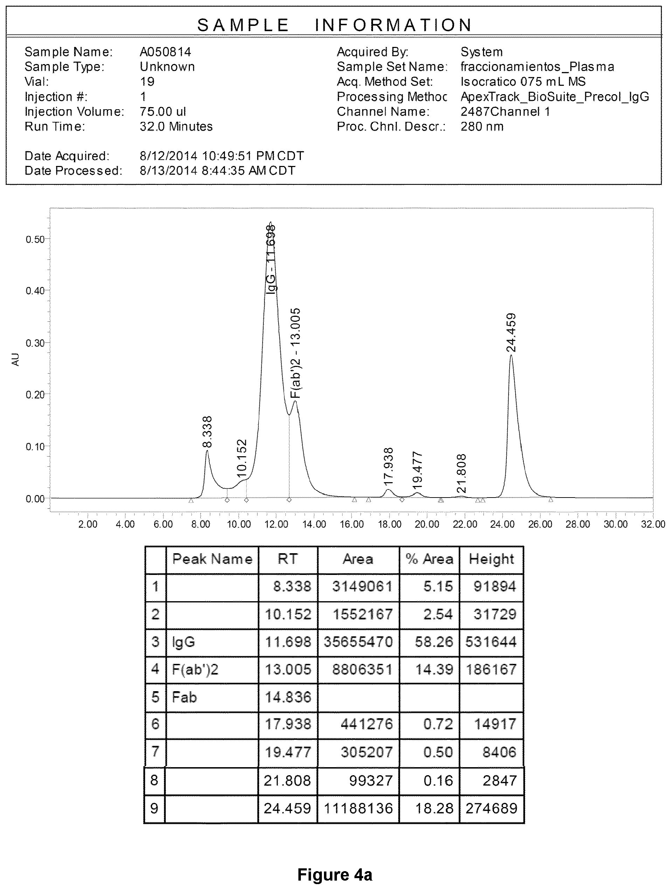

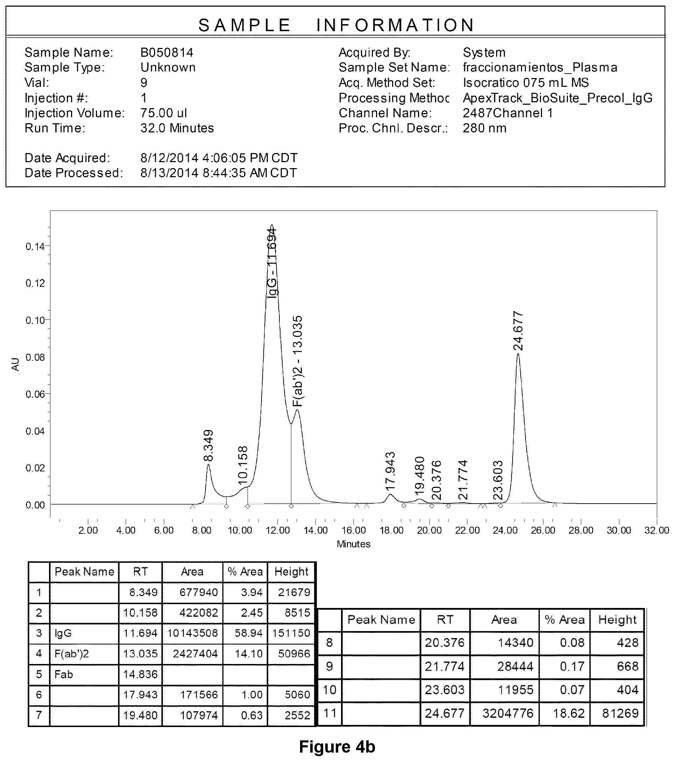

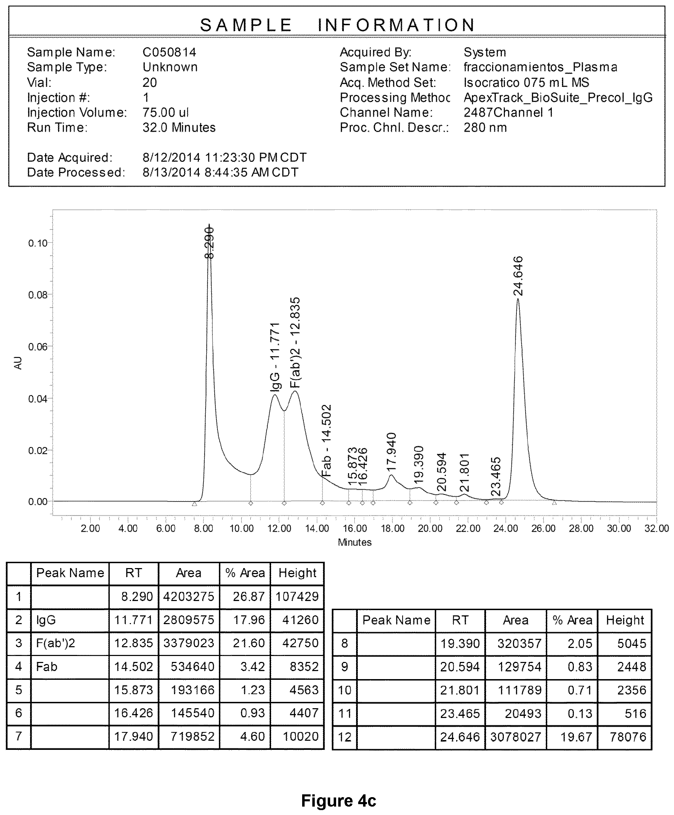

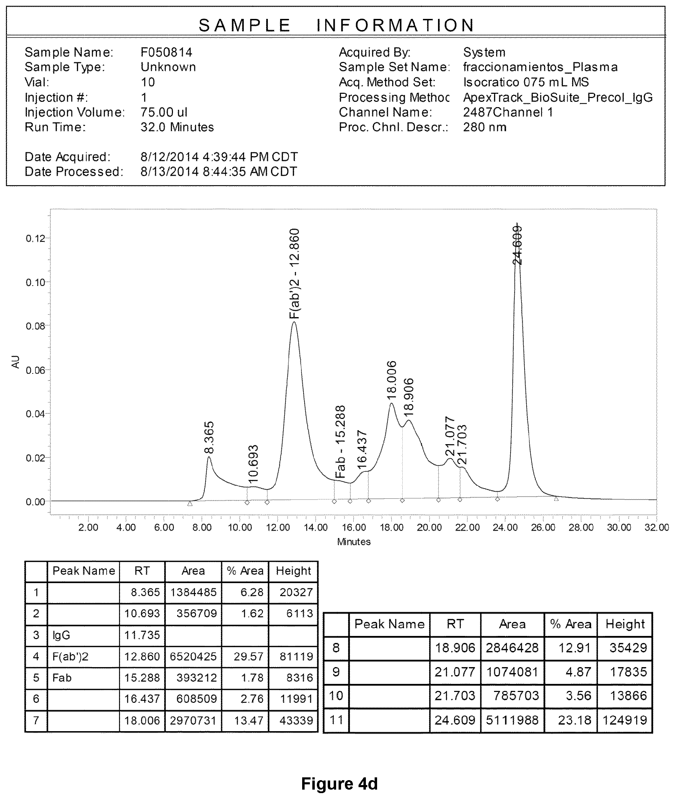

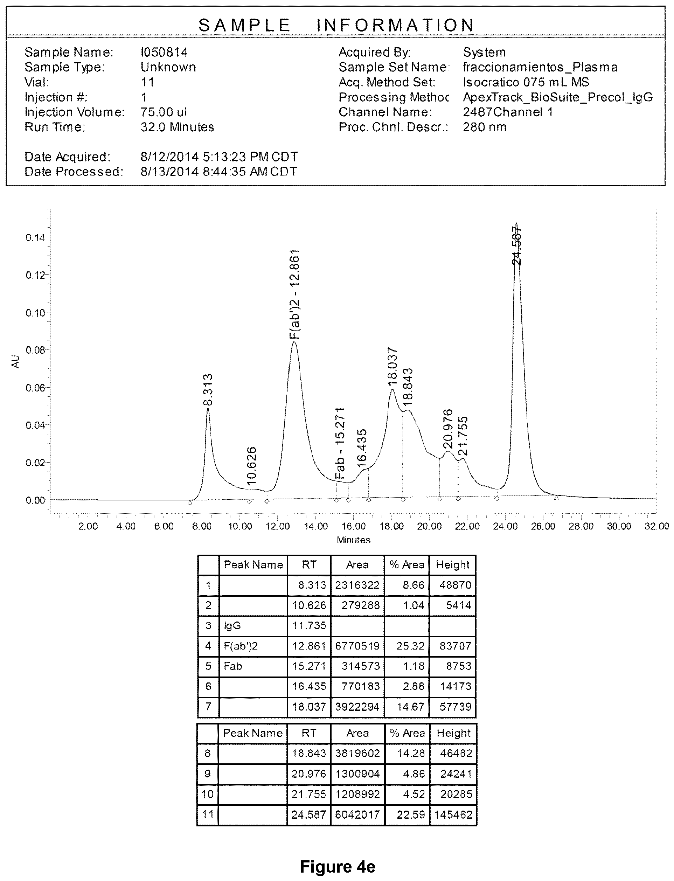

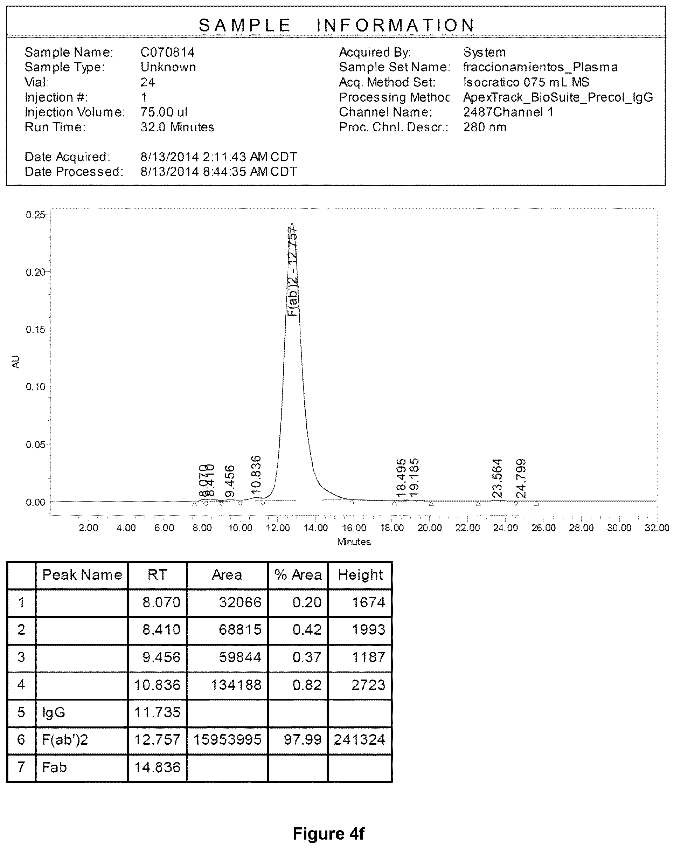

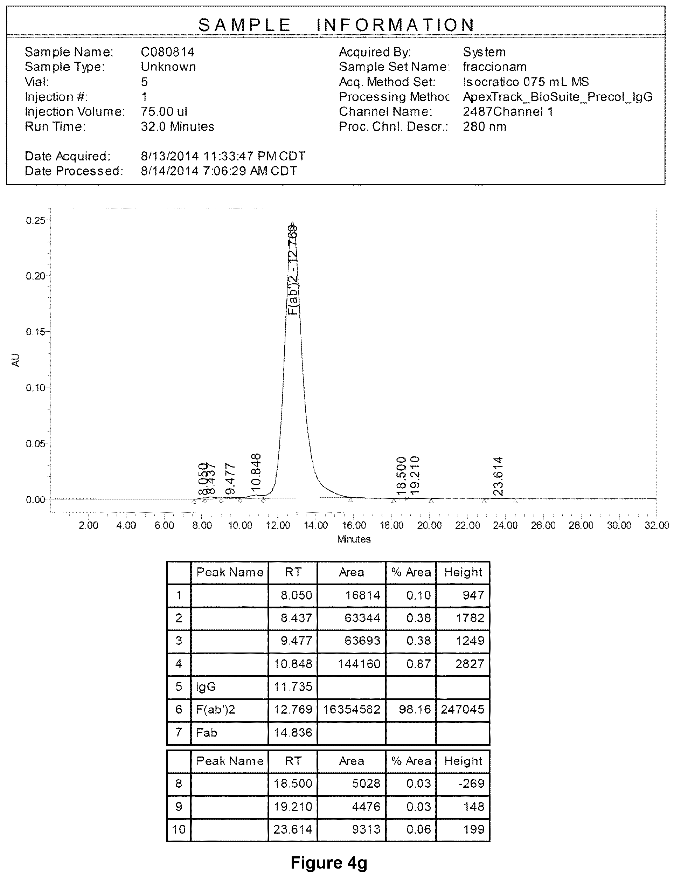

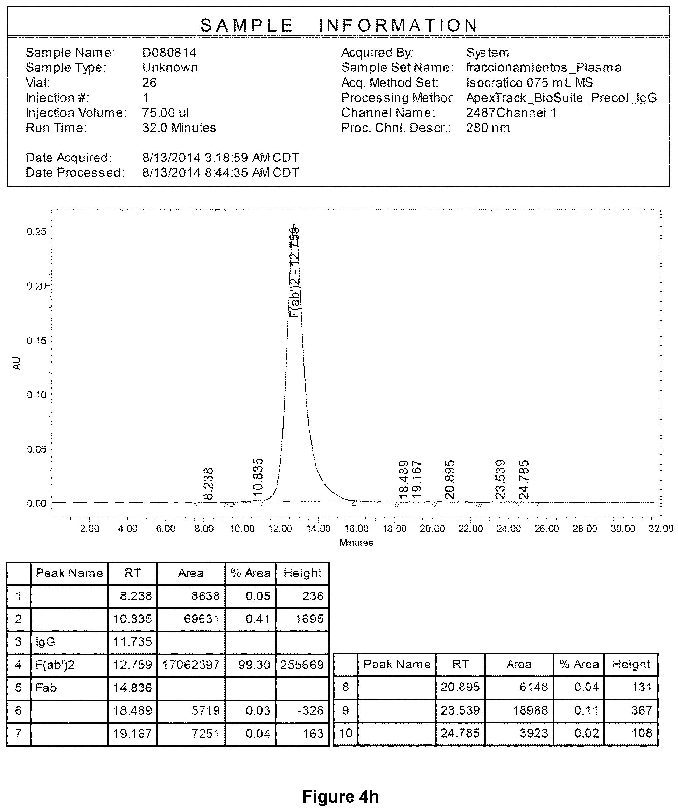

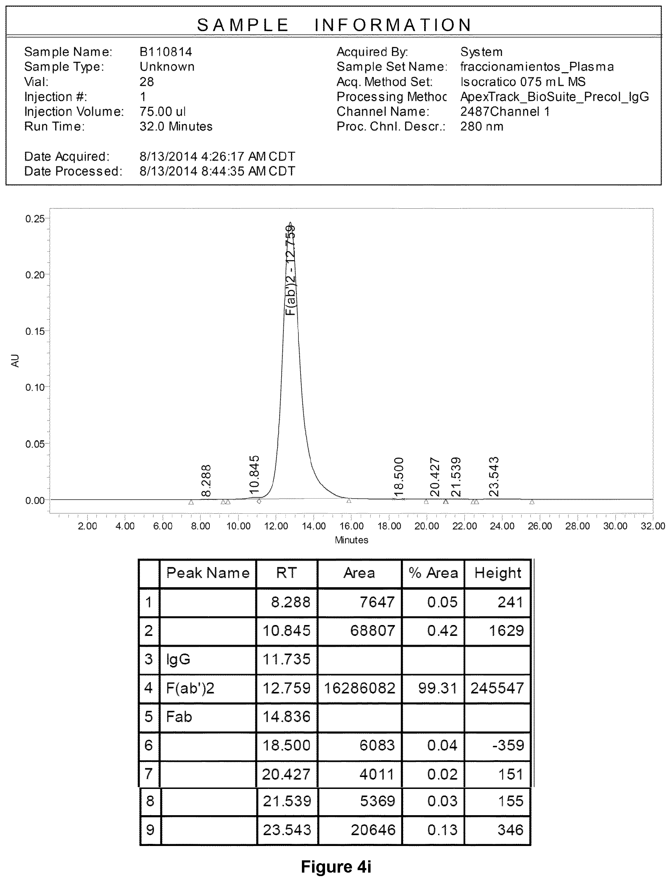

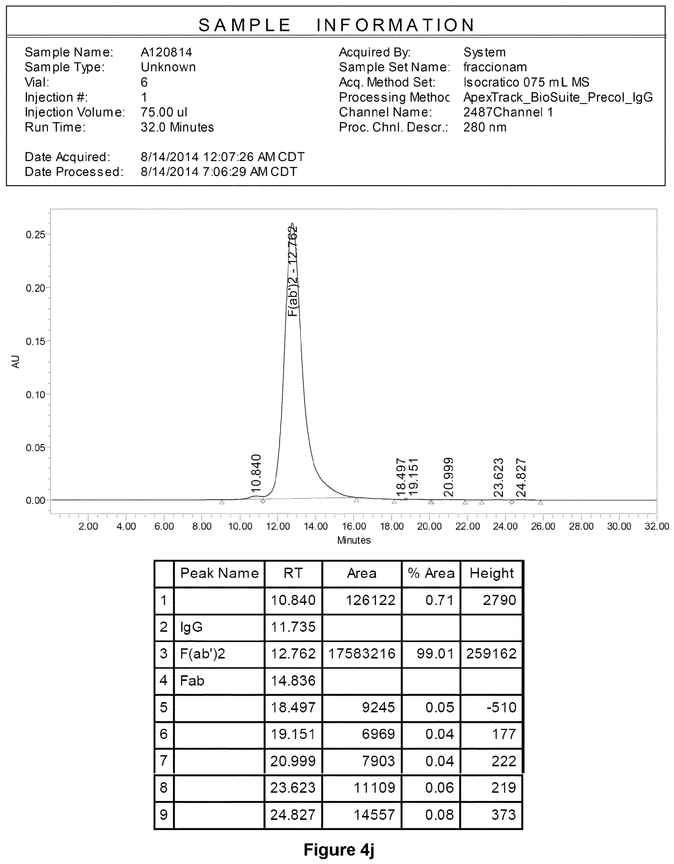

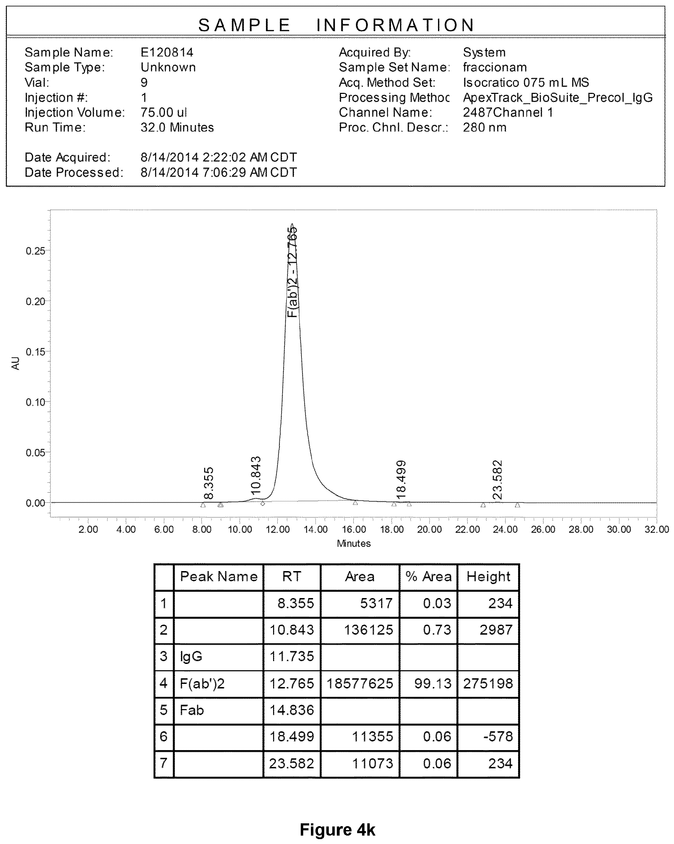

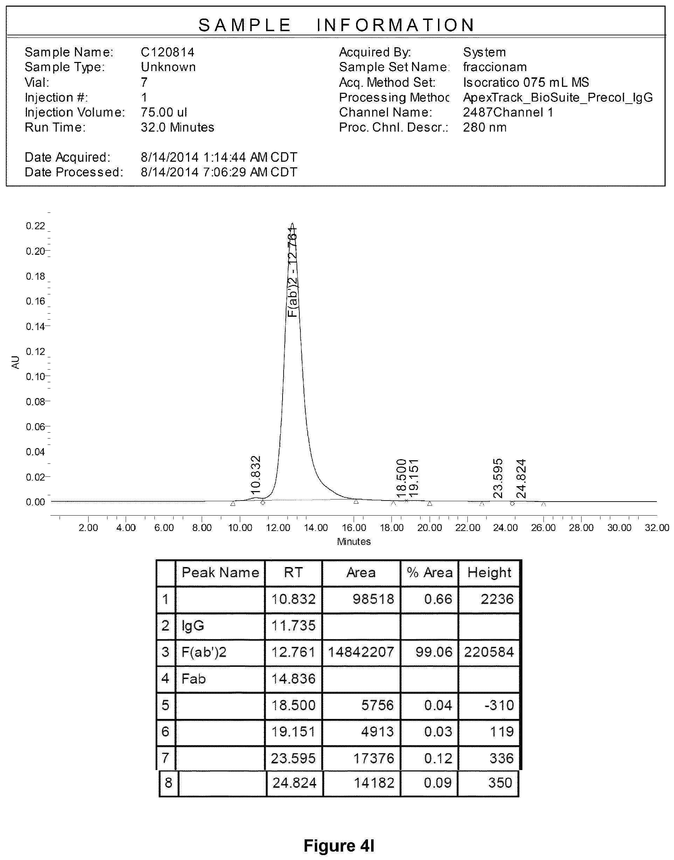

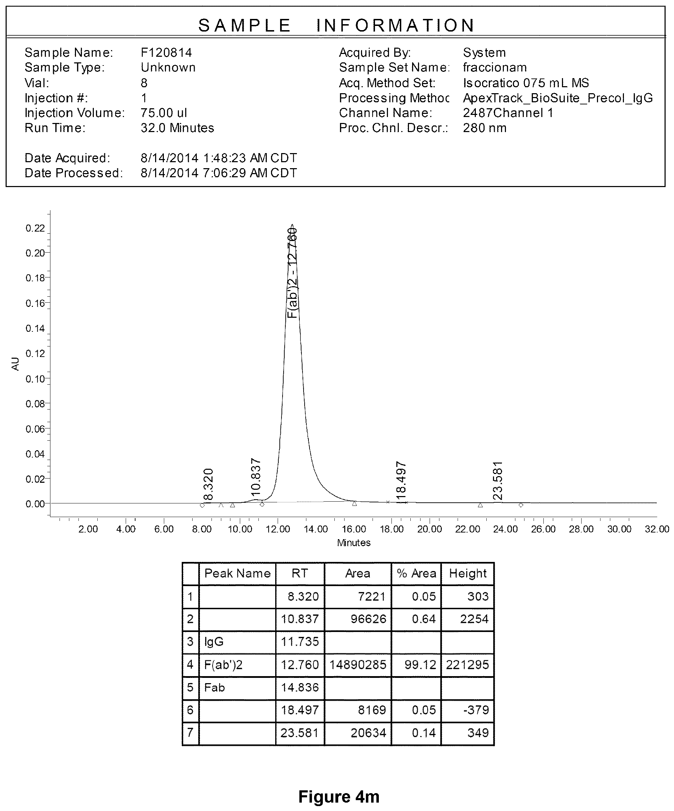

The ultrafiltration of the invention is carried out from the clarification obtained from the previous step, tangential filtration is performed. During the development of the invention, the tangential filtration operation of the hollow-fiber system with a 30 kDa cut size was replaced by the cassette system with a 30 kDa - 50 kDa cut size; purity was favored and it was also observed that F(ab')2 protein fragmentation is reduced so the cassette system offers greater performance. The appropriate parameters for using 30 and 50 kDa cassettes are described below, surprisingly the final product not only reaches the expected quality but exceeds it. In the HPLC chromatograms of Figure 4, results of the analysis of unprocessed plasma 4(a) and 4(b) are shown, and then digested 4 (c), and finally after being subjected to a 30 kDa cassette and then 50 kDa, in order to check the reproducibility of the process, 4 repetitions of this tangential filtration stage are shown for different samples (figures 4(f) to 4(m). Chromatograms 4(a) to 4(k) refer to the process of obtaining antivenoms (taking as an example a batch of a product designed as anti-snake (or antiviperine), using ultrafiltration with cassettes of 30 and then 50 kDa. Chromatograms 4(c) to 4(h) are the result of digestion kinetics for 6 hours. The chromatogram (i) is the result of precipitation with ammonium sulfate and without thermocoagulation. The chromatogram (j) is the clarified product. The chromatograms 4(k) and 4(r) refer to three repetitions of passage by 30 kDa and 5 0 kDa cassettes.

Table 15 presents results corresponding to the complete production and obtaining process of F(ab')$_2$ fragments, so it is representative of its characterization, this process was carried out at the pilot plant level. It was performed from plasma and without the application of cresol, ethyl ether and thermocoagulation, making an enzymatic digestion that was carried out over 6 hours for experimental evaluation purposes, with a single saline precipitation and replacing the operation of tangential filtration of the hollow-fiber system with a 30 kDa cut size by the cassette system with a 30 kDa - 50 kDa cut size.

TABLE 15. CHARACTERIZATION OF THE PRODUCTION AND OBTAINING PROCESS OF F(AB')2 FRAGMENTS AT THE PILOT PLANT LEVEL

| STAGE | | SAMPLE - PRODUCT | CODE | PURITY [%] PROTEIN | | | |
|---|---|---|---|---|---|---|---|
| | | | | $F(ab')_2$ | Fab | [mg/mL] | [mg/mL$_{Plasma}$] |
| Plasma | Raw material | Plasma | A050814 | 14.39 | ND | 15.97 | 2.30 |
| Prepared plasma | Plasma preparation | Plasma + Water for injection | B050814 | 14.10 | ND | 27.43 | 7.74 |
| Digestion | Time 0 | Plasma (digested) + Water for injection + Pepsin | C050814 | 21.60 | 3.42 | 28.89 | 12.48 |
| Digestion | Time 3 | Plasma (digested) + Water for injection + Pepsin | F050814 | 29.57 | 1.78 | 16.61 | 9.82 |
| Digestion | Time 6 | Plasma (digested) + Water for injection + Pepsin | I050814 | 25.32 | 1.18 | 13.98 | 7.08 |
| Digestion | Time 9 | Plasma (digested) + Water for injection + Pepsin | L050814 | 26.49 | ND | 11.55 | 6.12 |
| Digestion | Time 12 | Plasma (digested) + Water for injection + Pepsin | O050814 | 23.23 | ND | 12.45 | 5.78 |
| Digestion | Stop | Digested mixture (Plasma + Water for injection + Pepsin | P050814 | 23.63 | ND | 12.35 | 5.84 |
| Precipitation | Salting - out | Digested mixture (Plasma + Water for injection + Pepsin + ammonium sulfate) | Q050814 | 22.25 | ND | 5.57 | 2.48 |
| Clarification I and II | Clarification precipitate | Digested mixture (Plasma + Water for injection + Pepsin + ammonium sulfate) | R050814 | 12.95 | ND | 3.13 | 0.81 |
| Tangential filtration | Concentration - Diafiltration - Concentration | Product in NaCl 9 g/L | C070814 | 97.99 | ND | 25.13 | 4.92 |
| Tangential filtration | Diafiltration | Product in NaCl 9 g/L | C080814 | 98.16 | ND | 18.88 | 3.71 |
| Tangential filtration | Concentration - Diafiltration - Concentration | Product in NaCl 9 g/L | D080814 | 99.3 | ND | 14.37 | 2.85 |
| Tangential filtration | Diafiltration | Product in NaCl 9 g/L | B110814 | 99.31 | ND | 12.05 | 2.39 |
| Tangential filtration | Concentration - Diafiltration - Concentration | Product in NaCl 9 g/L | A120814 | 99.01 | ND | 9.74 | 1.93 |
| Tangential filtration | Diafiltration | Product in NaCl 9 g/L | E120814 | 99.13 | ND | 9.05 | 1.79 |

(continued)

**TABLE 15. CHARACTERIZATION OF THE PRODUCTION AND OBTAINING PROCESS OF F(AB')2 FRAGMENTS AT THE PILOT PLANT LEVEL**

| STAGE | | SAMPLE - PRODUCT | CODE | PURITY [%] | | PROTEIN | |
|---|---|---|---|---|---|---|---|
| | | | | $F(ab')_2$ | Fab | [mg/mL] | [mg/mL$_{Plasma}$] |
| Tangential filtration | Concentration - Diafiltration - Concentration | Product in NaCl 9 g/L | C120814 | 99.06 | ND | 15.51 | 3.07 |
| Tangential filtration | Diafiltration | Product in NaCl 9 g/L | F120814 | 99.12 | ND | 13.6 | 2.70 |

**With the process of the invention cresol and phenol are removed**

Phenol and its derivatives such as cresol are preservatives, however, they are toxic substances. As with any hazardous substance, its effects on health will depend on the dose, duration and type of exposure, on the presence of other chemical substances. The North American regulation for food and drugs or FDA (regulation of the US Food and Drug Administration (FDA)), does not allow the use of cresols in pharmaceutical products. Although other regulations allow it, the ideal is to achieve its removal. Therefore, during the development of the invention we determine the clearance of phenol and cresol as explained below. According to the FEUM (Mexican Pharmacopoeia), the process for the management of plasmas implies the use of phenol (from Roodt A. R. *et al*, 2010) or cresol as a preservative of newly extracted plasma from the immunized animal. When arriving at the plant, cresol is also added according to the standards, only for products manufactured under FDA regulations no cresol has been added (it should be mentioned that in the end this never impacted the sterile nature of plasma samples thus processed). Notwithstanding the foregoing, with the dialysis process (17 steps) it was already possible to eliminate the concentration of cresol (cresol/phenol) reaching pharmaceutically acceptable levels, with the process of the invention the cresol/phenol concentration is reduced to less than 0.058 mg/vial. During the development of the invention we decided to quantify cresol and phenol in the different stages of the production and obtaining process of $F(ab')_2$ fragments, this is done by tangential ultrafiltration, using cassettes of 30 kDa and 50 kDa cut size and using process water and the concentration of cresol that is used as well as that of phenol (0.52 mg/ml and 0.45 mg/ml respectively), by means of the analytical method for the determination of cresol, it was identified that both cresol as phenol are removed since diafiltration step with 30 kDa. The results are shown in Table 16. The spectrophotometric method does not distinguish cresol from phenol, but it does deliver differentiated results, the method is designed to measure phenol, and the phenol value is obtained by applying a conversion factor according to pharmacopoeia methods (FEUM[4], 2011). The analytical method consists of a colorimetric chemical reaction with diazo solution, the azo compound in the presence of cresol and/or phenol results in a reaction that generates the p-hydroxy-azo-p nitrobenzene, a color compound that absorbs in the visible range at 550 nm. Example 14 describes the processing of this test.

**Table 16: Determination of cresol and phenol by the spectrophotometric analytical method after diafiltration by 30kDa and 50kDa cassettes**

| Description | Processed volume | Concentration | | Mass | | Reduction % |
|---|---|---|---|---|---|---|
| | ml | phenol mg/ml | cresol mg/ml | phenol mg | cresol mg | |
| SALINE SOLUTION 0.9% | 4,000 | 0.45 | 0.52 | 1,800 | 2,066 | 0.0 |
| CONCENTRATION 2X | 2,000 | 0.23 | 0.26 | 460 | 528 | 74.4 |
| DIAFILTRATION 5 30 kDa | 2,000 | 0.00 | 0.00 | 0 | 0 | 100.0 |

(continued)

**Table 16: Determination of cresol and phenol by the spectrophotometric analytical method after diafiltration by 30kDa and 50kDa cassettes**

| Description | Processed volume | Concentration | | Mass | | Reduction % |
|---|---|---|---|---|---|---|
| | ml | phenol mg/ml | cresol mg/ml | phenol mg | cresol mg | |
| DIAFILTRATION 10 30 kDa | 2,000 | 0.00 | 0.00 | 0 | 0 | 100.0 |
| DIAFILTRATION 15 30 kDa | 2,000 | 0.00 | 0.00 | 0 | 0 | 100.0 |
| DIAFILTRATION 20 30 kDa | 2,000 | 0.00 | 0.00 | 0 | 0 | 100.0 |
| CONCENTRATION 10X | 400 | 0.00 | 0.00 | 0 | 0 | 100.0 |
| DIAFILTRATION 5 50 kDa | 400 | 0.00 | 0.00 | 0 | 0 | 100.0 |
| DIAFILTRATION 10 50 kDa | 400 | 0.00 | 0.00 | 0 | 0 | 100.0 |
| DIAFILTRATION 15 50 kDa | 400 | 0.00 | 0.00 | 0 | 0 | 100.0 |
| DIAFILTRATION 20 50 kDa | 400 | 0.00 | 0.00 | 0 | 0 | 100.0 |
| DIAFILTRATION 25 50 kDa | 400 | 0.00 | 0.00 | 0 | 0 | 100.0 |

The data indicate that cresol and phenol are removed by the tangential filtration process from the first stage of concentration and they are not detectable since the number 5 wash. The aforementioned allowed to have the experimental basis to consider that the tangential filtration process with cassette format removes cresol to undetectable levels. It is worth mentioning that for these comparisons the necessary controls were carried out to prove that the method itself does not generate interference. Accordingly, the test was carried out generating a control product ("comparison target") which consisted of a 0.9% sodium chloride solution without adding cresol or phenol. We performed the same quantification analysis of cresol and phenol but applying the production process of $F(ab')_2$ viperine (antiviperine) and scorpion antivenoms (anti-scorpion) fragments, at the pilot plant level, and starting from the antiviperine and anti-scorpion equine plasmas and during digestion with pepsin, precipitation, thermocoagulation, and the diafiltration modalities: the tangential one with hollow-fiber of 30 kDa cut size and that made with 30 kDa and 50 kDa cassette systems. Subsequent samples were also taken: the last clarified, the prefiltered formulated product, nano-filtered and finished product (FP). The results for antiviperine are shown in the graphs of Figures 2, 3 and 4. The method used in the measurements presented in Figures 5 to 8 is the spectrophotometric, so the determination involves both phenol and cresol. 20 and 30 washes were worked on in these experiments (figures 7 and 8), but later it was established that with 20 washes was enough. The data indicate that the clarification stage reduces 30% of the cresol present in the product, in the tangential filtration stage up to approximately 99% reduction of the cresol is obtained, where 80% is reached during the first concentration and the rest is achieved with washing. The difference between 20 washes and 30 washes is distinguishable when comparing the graphs of Figures 7 and 8. It should be noted that in all cases the concentration of cresol apparently increases at the formulation stage, therefore, the reduction percentage has a slight fall to a value of 97%, the explanation for this increase is the interference that the glycine excipient has. In the cresol method validation protocol, the indication that glycine interferes with the signal "The glycine solution and the analytical matrix have visual interference at 550 nanometers" is made. Conclusively there is a removal of the added cresol during the production and obtaining process of $F(ab')_2$ fragments, the highest percentage is obtained in the tangential filtration stage reaching 99% at the end of the process. In order to have a more controlled clearance of cresol and phenol, a validated chromatographic detection method (HPLC) was adapted because it is more specific. With this method it is concluded that cresol and phenol levels of less than 0.58 mg/ml per vial are achieved. It should be noted that at this experimental stage it was also concluded that the combined use of cassettes and the increasing number of washes during ultrafiltration, also determine the decrease of low molecular weight components (LMWC) without decreasing the active component content (F(ab')$_2$). With these steps, the final product characteristics exceed those required by pharmacopoeia.

**Determination of the optimal digestion time with pepsin**

The optimal pepsin digestion time was calculated later, with an experimental design in which the enzymatic kinetics were evaluated in several batches. Pepsin is used at a concentration of 6.6 g/l of plasma. The preparation of pepsin is illustrated in Example 2. For enzymatic digestion a pH of 3.5 $\pm$ 0.1 was used with control, a temperature of 37° C $\pm$ 1 ° C. The digestion time was 6 h. The samples to be considered are times 0 h (time 0), 1.5 h (time 3), 3 h (time 6), 4.5 h (time 9), 6 h (time 12). In example 3 the procedure for enzymatic digestion is described in more detail. One batch of enzymatic digestion with the antiviperine plasma (Batch: B3GALN09A) was carried out and one batch of enzymatic digestion with the anti-scorpion hyperimmune plasma (Batch: B3BAL12B) was carried out. Tables 17 to 20 show results for various batches of plasma. The highest amount of F(ab')$_2$ obtained from the plasma of all the analyzed batches was at 90 minutes. The amount of F(ab')$_2$ and Fab in the plasma, in the prepared plasma and in the time 0 digestion are not considered since they are fragments that correspond to the similar size of F(ab')$_2$ and Fab which is corroborates with the SDS - PAGE under reducing conditions. Based on these results, it is proposed for the production and obtaining process of F(ab')$_2$ fragments to carry out digestions at a pH of 3.5 $\pm$ 0.1 with control and at a temperature of 37 $\pm$ 1° C for 1.5 h (90 min), with these conditions the formation of F(ab')$_2$ and the non-formation of Fab are promoted even at HPLC detectable levels.

**TABLE 17. ENZYMATIC DIGESTION WITH ANTIVIPERINE PLASMA (BATCH: B3GALN09A).**

**pH of 3.5 ± 0.1 with control and at a temperature of 37 ± 1° C**

| Sample | Stage | | Description | Bradford (mg / mL) | SDS - PAGE | | | | HPLC (%) | |
|---|---|---|---|---|---|---|---|---|---|---|
| | | | | | IgG | Albumina | Fab | F(ab')$_2$ | Fab | F(ab')$_2$ |
| A050714 | Plasma | Raw Material | Plasma | 73.15 | + | + | - | - | ND | 26.00 |
| B050714 | Prepared plasma | Plasma preparation | Plasma + Water for injection | 36.74 | + | + | - | - | ND | 26.11 |
| C050714 | Digestion | Time 0 | Plasma (digested) + Water for injection + Pepsin | 28.17 | + | + | - | + | 10.76 | 9.85 |
| F050714 | Digestion | Time 3 | Plasma (digested) + Water for injection + Pepsin | 26.3 | + | + | - | + | ND | 31.45 |
| 1050714 | Digestion | Time 6 | Plasma (digested) + Water for injection + Pepsin | 24.16 | + | + | - | + | ND | 32.06 |
| L050714 | Digestion | Time 9 | Plasma (digested) + Water for injection + Pepsin | 23.74 | + | + | - | + | ND | 30.74 |
| 0050714 | Digestion | Time 12 | Plasma (digested) + Water for injection + Pepsin | 26.26 | + | + | - | + | ND | 28.92 |
| P050714 | Digestion | STOP | Digested mixture (Digested Plasma + Water for injection + Pepsin) | 22.99 | + | + | - | + | ND | 28.18 |

**TABLE 18. ENZYMATIC DIGESTION WITH ANTI-SCORPION HIPERINMUNE PLASMA (BATCH: B3BAL12B)**

**pH of 3.5 $\pm$ 0.1 with control and at a temperature of 37 $\pm$ 1° C**

| Sample | Stage | | Description | Bradford (mg / mL) | SDS - PAGE | | | | HF ( PLC %) | |
|---|---|---|---|---|---|---|---|---|---|---|
| | | | | | IgG | Albumina | Fab | F(ab')$_2$ | Fab | F(ab')$_2$ |
| R290714 | Plasma | Raw Material | Plasma | 71.00 | + | + | - | - | ND | 21.32 |
| A290714 | Prepared plasma | Plasma preparation | Product in NaCl 9 g/L | 32.43 | + | + | - | - | ND | 21.28 |
| B290714 | Digestion | Time 0 | Plasma (digested) + Water for injection + Pepsin | 32.06 | + | + | - | + | 2.48 | 17.22 |
| E290714 | Digestion | Time 3 | Plasma (digested) + Water for injection + Pepsin | 24.92 | + | + | + | + | 2.94 | 25.87 |
| H290714 | Digestion | Time 6 | Plasma (digested) + Water for injection + Pepsin | 23.13 | + | + | - | + | 3.28 | 24.38 |
| K290714 | Digestion | Time 9 | Plasma (digested) + Water for injection + Pepsin | 21.27 | + | + | - | + | 3.09 | 22.73 |
| N290714 | Digestion | Time 12 | Plasma (digested) + Water for injection + Pepsin | 20.83 | + | + | - | + | 2.82 | 20.39 |
| O290714 | Digestion | STOP | Digested mixture (Digested Plasma + Water for injection + Pepsin) | 20.40 | + | + | - | + | 2.57 | 21.22 |

**TABLE 19. Enzymatic digestion with the antiviperine plasma (batch: B3GALN09A) at the pilot level for the production of F(ab')2. pH of 3.5 ± 0.1 with control and at a temperature of 37 ± 1° C**

| Sample | Stage | | Description | Bradford (mg/mL) | HPLC (%) | | Fab | | F(ab')$_2$ | |
|---|---|---|---|---|---|---|---|---|---|---|
| | | | | | Fab | F(ab')$_2$ | mg/ml | Total mg | mg/ml | Total mg |
| A050714 | Plasma | Raw Material | Plasma | 73.15 | ND | 26 | 0.0 | 0.0 | 1,901.9 | 3,803,800.0 |
| B050714 | Prepared plasma | Plasma preparation | Plasma + Water for injection | 36.74 | ND | 26.11 | 0.0 | 0.0 | 959.3 | 3,837,125.6 |
| C050714 | Digestion | Time 0 | Plasma (digested) + Water for injection + Pepsin | 28.17 | 10.76 | 9.85 | 303.1 | 1,212,436.8 | 277.5 | 1,109,898.0 |
| F050714 | Digestion | Time 3 | Plasma (digested) + Water for injection + Pepsin | 26.3 | ND | 31.45 | 0.0 | 0.0 | 827.1 | 3,308,540.0 |
| I050714 | Digestion | Time 6 | Plasma (digested) + Water for injection + Pepsin | 24.16 | ND | 32.06 | 0.0 | 0.0 | 774.6 | 3,098,278.4 |
| L050714 | Digestion | Time 9 | Plasma (digested) + Water for injection + Pepsin | 23.74 | ND | 30.74 | 0.0 | 0.0 | 729.8 | 2,919,070.4 |
| O050714 | Digestion | Time 12 | Plasma (digested) + Water for injection + Pepsin | 26.26 | ND | 28.92 | 0.0 | 0.0 | 759.4 | 3,037,756.8 |
| P050714 | Digestion | STOP | Digested mixture (Digested Plasma + Water for injection + Pepsin) | 22.99 | ND | 28.18 | 0.0 | 0.0 | 647.9 | 2,591,432.8 |

**Table 20. Enzymatic digestion with anti-scorpion hyperimmune plasma (BATCH: B3BAL12B) at pilot level for the production of F(ab')2 pH of 3.5 ± 0.1 with control and at a temperature of 37 ± 1° C**

| Sample | Stage | | Description | Bradford (mg/ml) | HPLC (%) | | Fab | | F(ab')2 | |
|---|---|---|---|---|---|---|---|---|---|---|
| | | | | | Fab | F (ab')2 | mg/ml | Total mg | mg/ml | Total mg |
| R290714 | Plasma | Raw Material | Plasma | 71 | ND | 21.32 | 0.0 | 0.0 | 1,513.7 | 3,027,440.0 |
| A290714 | Prepared plasma | Plasma preparation | Plasma + Water for injection | 32.43 | ND | 21.28 | 0.0 | 0.0 | 690.1 | 2,760,441.6 |
| B290714 | Digestion | Time 0 | Plasma (digested) + Water for injection + Pepsin | 32.06 | 2.48 | 17.22 | 79.5 | 318,035.2 | 552.1 | 2,208,292.8 |
| E290714 | Digestion | Time 3 | Plasma (digested) + Water for injection + Pepsin | 24.92 | 2.94 | 25.87 | 73.3 | 293,059.2 | 644.7 | 2,578,721.6 |
| H290714 | Digestion | Time 6 | Plasma (digested) + Water for injection + Pepsin | 23.13 | 3.28 | 24.38 | 75.9 | 303,465.6 | 563.9 | 2,255,637.6 |
| K290714 | Digestion | Time 9 | Plasma (digested) + Water for injection + Pepsin | 21.27 | 3.09 | 22.73 | 65.7 | 262,897.2 | 483.5 | 1,933,868.4 |
| N290714 | Digestion | Time 12 | Plasma (digested) + Water for injection + Pepsin | 20.83 | 2.82 | 20.39 | 58.7 | 234,962.4 | 424.7 | 1,698,894.8 |
| O290714 | Digestion | STOP | Digested mixture (Digested Plasma + Water for injection + Pepsin) | 20.4 | 2.57 | 21.22 | 52.4 | 209,712.0 | 432.9 | 1,731,552.0 |

After 90 minutes the productivity of F(ab')$_2$ does not improve and also the enzyme can begin to damage the proteins of interest, so the digestion must stop at this point of kinetics. The integration of the process steps to obtain a composition of F(ab')2 antibody fragments from hyperimmune plasma are presented in Table 21.

| Table 21: Developed process to obtain a composition of F(ab')2 antibody fragments | |
| --- | --- |
| 1 | Preparation of pepsin; Clarified and sterilized by filtration. |
| 2 | Mixture of hyperimmune blood plasmas containing phenol (concentrated) in the reactor. The addition of cresol and/or ethyl ether is dispensable. |
| 3 | Enzymatic digestion by pepsin at 3.5 $\pm$ 0.1 with control and at a temperature of 37 $\pm$ 1° C for 1.5 h (90 minutes). The reaction is stopped by adjusting the pH at 4.2 + 0.2 with 5N NaOH. |
| 4 | Immediately proceed to the precipitation of unwanted protein fragments and adding 21% w/v of ammonium sulfate.<br>Do not let stand, proceed to clarification I and II.<br>Thermocoagulation is dispensable. |
| 5 | Clarification I (8 $\mu$m at 20 $\mu$m) and clarification II (0.2 $\mu$m nominal) of the digested mixture (soluble Fraction of F(ab')$_2$).<br>A partially purified F(ab')$_2$ solution ready for ultrafiltration is obtained. |
| 6 | Automated ultrafiltration of the digested mixture<br>• 30 kDa Hollow-fiber membrane.<br>• 30 and 50 kDa cassette. |
| 7 | Clarification III of the Concentrate. At this point, quality control is carried out. The potency in LD$_{50}$ neutralizing/ml is determined. |
| 8 | Product Bulk Formulation. It is diluted to obtain the potency specified for the product.<br>Excipients: glycine, sodium chloride, sucrose.<br>A prefilter at 0.1 $\mu$m is integrated, leaving the product in better conditions so that it becomes nanofiltration. |
| 9 | Nanofiltration of the product bulk, 20 nm membrane to eliminate the potential presence of viruses |
| 10 | Sterile Filtration of the Product Bulk |
| 11 | Filling the Product Bulk |
| 12 | Lyophilization, samples are reconstituted by batch for analysis certificates. |

The components of the formulation that are made to the product bulk, (glycine, sodium chloride, sucrose), confer stability to the active substance, since although the product undergoes lyophilization it does not suffer any type of alteration. The final product does not need to be stored in refrigeration nor it is unstable before exposure to light, its expiration extends for quite more than 5 years, nevertheless, due to sanitary requirements an expiration date of 4 years is assigned to it. Example 13 describes the lyophilization process.

It is important to mention that the proportion of each of the excipients (glycine, sodium chloride, sucrose), affects the times of reconstitution of the mixture. This fact in medical practice is very relevant, there are antidotes on the market that take several minutes to dissolve, thus valuable minutes that can make an important difference are lost. Since minutes count in cases of poisoning, it is best to inject the antidote to the patient as soon as possible and the product of the invention is reconstituted in a few seconds. Commercially available lyophilized antivenoms have a much longer reconstitution time (Gerringa, et al. 2013).

A solubility time study in concentrated and hyperconcentrated batches (100x) were performed by using the formulation described in example 10. Hyperconcentrated products may be required to solve more serious poisoning problems so we proceeded to concentrate them 100x during the same UF process. For these reconstitution time tests of the lyophilized product, 3 batches were manufactured: Anti-scorpion, Anti-snake and an Anti-TNF; using enzymatic digestion by pepsin at pH 3.5 $\pm$ 0.1 with control and a temperature of 37 $\pm$ 1° C for 1.5 h (90 minutes), a single precipitation with 21% w/v of ammonium sulfate. The ultrafiltered product with a 30 kDa cassette was used, each batch was divided into 2; one part was formulated, dosed and lyophilized, they are the so-called concentrates. The other part was concentrated at 100X, formulated, dosed and lyophilized; these are the so-called hyperconcentrates. Tables 22 to 25 show the results of the dissolution process and the analysis of the formulations of these concentrated and hyperconcentrated batches, the times were taken by direct observation from the vials (seen by two people (Q1 and Q2)) since the solution enters and until the particles disappear, 8 repetitions of each batch were taken. Tables 26 to 29 indicate the stability of two batch: Batch B-9H-21, produced by only one precipitation and 30 kDa hollow-fiber UF (Tables 26 and 27) and batch B-0K-15, produced by 17 steps and (Tables 28 and 29) and the stability parameters are met in both cases.

**Table 22. Results of the dissolution of the lyophilized formulated concentrates.**

| Batch | Product | Disolution | | | | Average Q1 (s) | Disolution | | | | Average | Average |
|---|---|---|---|---|---|---|---|---|---|---|---|---|
| | | Q1 (s) | Q1 (s) | Q1 (s) | Q1 (s) | | Q2 (s) | Q2 (s) | Q2 (s) | Q2 (s) | Q2 (s) | |
| DFB1608-02 | Anti-scorpion | 17.84 | 12.20 | 16.41 | 22.35 | 17.20 | 17.3 | 16.49 | 16.16 | 18.66 | 17.15 | 17.18 |
| DFB1608-04 | Anti-snake | 28,16 | 21.56 | 29.30 | 24.81 | 25.96 | 22.19 | 26.27 | 25.75 | 23.32 | 24.38 | 25.17 |
| DFB1608-05 | Anti-TNF | 19.23 | 25.30 | 28.67 | 17.21 | 22.60 | 19.55 | 20.36 | 21.82 | 25.71 | 21.86 | 22.23 |

**Table 23. Results of the dissolution of the lyophilized formulated concentrates.**

| Batch | Product | Disolution | | | | Average Q1 (s) | Disolution | | | | Average Q2 (s) | Average |
|---|---|---|---|---|---|---|---|---|---|---|---|---|
| | | Q1 (s) | Q1 (s) | Q1 (s) | Q1 (s) | | Q2 (s) | Q2 (s) | Q2 (s) | Q2 (s) | | |
| DFB1609-01 | Anti-scorpion | 359 | 271 | 311 | 166 | 276.75 | 281 | 275 | 274 | 241 | 267.75 | 272.25 |
| DFB1609-02 | Anti-snake | 244 | 135 | 196 | NA | 191.67 | 224 | 231 | 207 | NA | 220.67 | 206.17 |
| DFB1609-03 | Anti-TNF | 230 | 222 | 274 | 240 | 241.50 | 361 | 185 | 247 | 266 | 264.75 | 253.13 |

**Table 24. Analysis of lyophilized formulated concentrates.**

| Batch | Product | Protein (mg/ml) | Volume (ml) | Protein (mg) | NaCl | Glycine | sucrose | Total solids (mg) | Total solids (%) | Average dissolution |
|---|---|---|---|---|---|---|---|---|---|---|
| DFB1608-02 | Anti-scorpion | 22.40 | 50 | 1120.0 | 450.0 | 750.4 | 369.6 | 2690.0 | 5.38 | 17.18 |
| DFB1608-04 | Anti-snake | 33.02 | 50 | 1651.0 | 450.0 | 1106.2 | 544.8 | 3752.0 | 7.50 | 25.17 |
| DFB1608-05 | Anti-TNF | 18.03 | 50 | 901.5 | 450.0 | 604.0 | 297.5 | 2253.0 | 4.51 | 22.23 |

Table 25. Analysis of lyophilized formulated hyperconcentrates.

| Batch | Product | Protein (mg/ml) | Volume (ml) | Protein (mg) | NaCl | Glycine | sucrose | Total solids (mg) | Total solids (%) | Average dissolution |
|---|---|---|---|---|---|---|---|---|---|---|
| DFB1609-01 | Anti-scorpion | 133.53 | 40 | 5341.2 | 360.0 | 3578.6 | 1762.6 | 11042.4 | 27.61 | 272.25 |
| DFB1609-02 | Anti-snake | 143.12 | 40 | 5724.8 | 360.0 | 3835.6 | 1889.2 | 11809.6 | 29.52 | 206.17 |
| DFB1609-03 | Anti-TNF | 140.74 | 40 | 5629.6 | 360.0 | 3771.8 | 1857.8 | 11619.2 | 29.05 | 253.13 |

EP 3 680 252 A1

| TABLE 26. Long-term stability data. Start date: 03/Sep/2009 End date: 03/Sep/2015<br>A PRECIPITATION AND UF WITH 30 kDa HOLLOW-FIBER<br>BATCH: B-9H-21 ANTISCORPION<br>CONDITIONS: 25° C / 60% RH | | | | | | | | | | |
|---|---|---|---|---|---|---|---|---|---|---|
| | | Time in Months | | | | | | | | |
| Determination | Specification | 0 | 3 | 6 | 9 | 12 | 18 | 24 | 36 | 48 |
| | | 03/Sep/09 | 03/Dic/09 | 03/Mar/10 | 03/Jun/10 | 03/Sep/10 | 03/Mar/11 | 03/Sep/11 | 03/Sep/12 | 03/Sep/13 |
| APPEARANCE LIOFILIZED CONTAINER | Powdery or porous solid appearance, fragile white to light yellow, free of foreign particles | Comply | | | | | | | | |
| pH | 6.0-7.0 | 6.66 | 6.65 | 6.65 | 6.71 | 6.73 | 6.80 | 6.81 | 6.75 | 6.82 |
| Humidity (%) | No more than 3.0% | 1.0068 | 1.7735 | 2.0057 | 2.3163 | 2.3540 | 2.2043 | 2.4859 | 2.3137 | 2.4235 |
| Disolution time | No more than 35s | 13.67 | 12.32 | 12.35 | 12.58 | 11.54 | 11.45 | 11.25 | 12.09 | 11.16 |
| Biologic potency | No less than 150 $LD_{50}$ Neutra/Fco. | 249.02 | 248.97 | 249.05 | 246.63 | 243.94 | 248.97 | 248.97 | 236.31 | 224.57 |
| Safety | Meet requirements | Comply | N/A | N/A | N/A | N/A | N/A | Comply | Comply | N/A |
| Sterility | Sterile | Comply | N/A | N/A | N/A | N/A | N/A | Comply | Comply | N/A |
| Electrophoresis SDS Page (%) | IgG + IgGT No more than 5% | < 3.0 | < 3.0 | < 3.0 | < 3.0 | < 3.0 | < 3.0 | < 3.0 | < 3.0 | < 3.0% |
| | Albumin No more than 0.5% | < 0.5 | < 0.5 | < 0.5 | < 0.5 | < 0.5 | < 0.5 | < 0.5 | < 0.5 | < 0.5 |

| ELLISA assay | It is determined by recognition of the specific antigen compared to a negative control | Comply |

UF: Ultrafiltration

N/A = Not applicable

REFERENCE: Official Mexican Standard NOM-073-SSA1-2005, Stability of drugs and medicines

ICH Guideline Q1A (R2): Stability Testing of New Drug Substances and Products

**TABLE 27. Long-term stability data. Start date: 03/Sep/2009 End date: 03/Sep/2015**
**ONE PRECIPITATION AND 30 kDA HOLLOW-FIBER UF**
**BATCH: B-9H-21 ANTISCORPION**
**CONDITIONS: 25° C / 60% RH**

| Determination | Specification | Time in Months | | | | | | | | |
|---|---|---|---|---|---|---|---|---|---|---|
| | | 0 | 3 | 6 | 9 | 12 | 18 | 24 | 36 | 48 |
| | | 03/Sep/09 | 03/Dic/09 | 03/Mar/10 | 03/Jun/10 | 03/Sep/10 | 03/Mar/11 | 03/Sep/11 | 03/Sep/12 | 03/Sep/13 |
| HPLC | % F(ab')$_2$ No les than 90% (%) | 90.58 | 90.93 | 91.90 | 90.82 | 90.68 | 90.54 | 90.75 | 96.16 | 93.13 |
| | % Fab = SPI* (%) | 3.67 | 3.72 | 3.69 | 4.00 | 4.13 | 3.19 | 3.39 | ND | 2.65 |
| | % LMWC No more than 10% | 0.85 | 0.81 | 0.13 | 1.03 | 0.95 | 0.12 | 1.72 | 1.39 | 0.71 |
| | % HMWC = SPI* | 4.89 | 4.53 | 4.27 | 3.45 | 4.15 | 6.15 | 4.14 | 2.45 | 3.51 |

EP 3 680 252 A1

OBSERVATIONS:
LMWC = Low molecular weight components
HMWC = High molecular weight components

EP 3 680 252 A1

**TABLE 28. Long-term stability data. Start date: 27/Oct/2010 End date: 27/Oct/2015**
**Processed by double salting out and dialysis (Table 1, 17 steps process)**
**BATCH:: B-0K-15 ANTISCORPION**
**CONDITIONS: 25° C / 60% RH**

| Determination | Specification | Time in Months | | | | | | | |
|---|---|---|---|---|---|---|---|---|---|
| | | 0 | 3 | 6 | 9 | 12 | 18 | 24 | 36 |
| | | 27/Oct/10 | 27/Ene/11 | 27/Abr/11 | 27/Jul/11 | 27/Oct/11 | 27/Abr/12 | 27/Oct/12 | 27/Oct/13 |
| APPEARANCE LIOFILIZED CONTAINER | Powdery or porous solid appearance, fragile white to light yellow, free of foreign particles | Cumple | | | | | | | |
| pH | 6.0-7.0 | 6.81 | 6.78 | 6.87 | 6.80 | 6.86 | 6.87 | 6.87 | 6.86 |
| Humidity (%) | No more than 3.0% | 1.0044 | 2.2623 | 1.9592 | 2.3677 | 2.1204 | 1.9859 | 2.0327 | 2.4338 |
| Disolution time | No more than 35s | 11.79 | 12.15 | 12.25 | 11.39 | 11.47 | 11.51 | 11.43 | 11.99 |
| Biologic potency | No less than 150 $LD_{50}$ Neutra/Fco. | 270.86 | 270.88 | 236.24 | 236.24 | 238.59 | 259.52 | 236.20 | 219.61 |
| Safety | Meet requirements | Cumple | N/A | N/A | N/A | N/A | N/A | Cumple | N/A |
| Sterility | Sterile | Cumple | N/A | N/A | N/A | N/A | N/A | Cumple | N/A |
| Electrophoresis SDS Page (%) | IgG + IgGT No more than 5% | < 2.0 | < 2.0 | < 3.0 | < 3.0 | < 3.0 | < 3.0 | < 3.0 | < 3.0 |

41

| | Albumin No more than 0.5% | < 0.5 | < 0.5 | < 0.5 | < 0.5 | < 0.5 | < 0.5 | < 0.5 | < 0.5 |
|---|---|---|---|---|---|---|---|---|---|
| ELLISA assay | It is determined by recognition of the specific antigen compared to a negative control | Cumple | | | | | | | |

N/A = Not applicable

**TABLE 29. Long-term stability data. Start date: 27/Oct/2010 End date: 27/Oct/2015 Processed by double salting out and dialysis (Table 1 Process of 17 steps) BATCH: B-0K-15 ANTISCORPION; CONDITIONS: 25° C / 60% RH**

| Determination | Specification | | Time in Months | | | | | | | |
|---|---|---|---|---|---|---|---|---|---|---|
| | | 0 | 3 | 6 | 9 | 12 | 18 | 24 | 36 |
| | | 27/Oct/10 | 27/Ene/11 | 27/Abr/11 | 27/Jul/11 | 27/Oct/11 | 27/Abr/12 | 27/Oct/12 | 27/Oct/13 |
| HPLC | % F(ab')$_2$ No less than 85% (%) | 88.77 | 88.30 | 89.24 | 89.43 | 89.77 | 89.58 | 91.2 | 89.01 |
| | % Fab = No more tan 7% (%) | 6.38 | 6.06 | 6.43 | 5.78 | 6.02 | 2.97 | 3.93 | 6.21 |
| | % LMWC No more than 3% | 2.05 | 2.59 | 1.3 | 1.88 | 1.64 | 2.83 | 2.96 | 1.73 |
| | % HMWC No more tan 5% | 42.80 | 3.05 | 3.0 | 2.91 | 2.56 | 3.62 | 2.54 | 3.05 |

HMWC = High molecular weight components

LMWC = Low molecular weight components

The present invention relates to a new production process of antibodies F(ab')$_2$ fragments that specifically bind to antigens present in venoms, with a preferred embodiment for antagonizing arachnid or viperine venoms. The process of the invention is characterized by the use of ultrafiltration and the reduction of unit operation stages, obtaining a safe antivenom due to its neutralizing efficacy and because it does not generate any pyrogenic or anaphylactic reaction. The process of the invention is of the highest yield and purity for the final product, this improves in its neutralizing potency and in its stability, the quality of the final product is therefore higher to that obtained with the processes described in the state of art.

It is important to note that the motivation for the changes in the process to obtain antivenoms based on $F(ab')_2$ fragments of immunoglobulins, was due to a constant change in the quality of the venoms used to immunize horses, and also due to the improvements in immunization schedules. This resulted in the obtaining of very high plasma antibody titers, so it was necessary to adapt to these new conditions that were presented year after year as evidenced below.

In the case of antivenoms against scorpion species venom, a change in the way to obtain the venoms was implemented, it was about electrically stimulating the animal's telson instead of removing the telson and macerating it. Tables 30 and 31 show the antibody titer expressed as neutralizing doses ($LD_{50}$ Neut/ml) the first (30) using scorpion venom from species *Centruroides noxius, C. limpidus limpidus, C. limpidus tecomanus* and *C. suffussus suffussus*, obtained by maceration of telson glands (Group of horses I and IV) and the second (31) using venom obtained by electrical stimulation of telson glands (Group of horses II and III). The difference in potency levels between groups A and B is therefore due to the way to obtain the scorpion venom, it must be compared by group and by year, that is: groups I and II vs groups III and IV , for example: for the year 2009, 102.78 and 95.06 vs 121.9 and 112.04; for the year 2010, 104.92 and 112.4 vs 141.8 and 116.02 and so on. From this comparison we can conclude that it has been very beneficial to obtain venom by electrically stimulating the glands instead of macerating them.

Table 30: Antibody titer in plasma expressed as $LD_{50}$ Neut/ml, annual averages 2009 to 2013. Venom obtained by maceration of telson glands. Group of horses I, II.

| ANTIBODY TITER ($LD_{50}$ Neut/ml) | | | Group I | |
|---|---|---|---|---|
| **AVERAGE (2009)** | **AVERAGE (2010)** | **AVERAGE (2011)** | **AVERAGE (2012)** | **AVERAGE (2013)** |
| 102.78 | 104.92 | 147.26 | 152.12 | 273.01 |

| ANTIBODY TITER ($LD_{50}$ Neut/ml) | | | Group II | |
|---|---|---|---|---|
| **AVERAGE (2009)** | **AVERAGE (2010)** | **AVERAGE (2011)** | **AVERAGE (2012)** | **AVERAGE (2013)** |
| 95.06 | 112.4 | 129.81 | 133.6 | 142.65 |

Table 31: Antibody titer in plasma expressed as $LD_{50}$ Neut/ml, annual averages 2009 to 2013. Venom obtained by electrical stimulation of telson glands. Group of horses III, IV.

| ANTIBODY TITER ($LD_{50}$ Neut/ml) | | | Group III | |
|---|---|---|---|---|
| **AVERAGE (2009)** | **AVERAGE (2010)** | **AVERAGE (2011)** | **AVERAGE (2012)** | **AVERAGE (2013)** |
| 121.9 | 141.8 | 189.99 | 352.25 | 369.32 |

| ANTIBODY TITER ($LD_{50}$ Neut/ml) | | | Group IV | |
|---|---|---|---|---|
| **AVERAGE (2009)** | **AVERAGE (2010)** | **AVERAGE (2011)** | **AVERAGE (2012)** | **AVERAGE (2013)** |
| 112.04 | 116.02 | 150.8 | 240.11 | 253.21 |

Also, in the case of antibody titers in antiviperine plasma, an increase has been noted year by year. As an example, some data is shown in Figure 9.

The immunization schemes have also been modified and this has also generated the increase in the antibody titer that has been seen year by year. While the increase in potencies has also been due to the increase in antibody titers, it has not been the main reason for improving yields, since the increase of titers level is not directly proportional to the increase in yields per unit of volume plasma, hence it is inferred that the obtaining process of $F(ab')_2$ is also a variable that has promoted the yield increase and concomitantly in potencies in addition to improving purities by more than 95%.

It is the main object of the invention to provide an obtaining process of antivenoms whose active ingredient is $F(ab')_2$ fragments of polyclonal antibodies from hyperimmune non-human mammalian serum. It is an embodiment of the invention to provide a process that requires fewer steps than is known in the state of the art, so that the manufacturing

## EP 3 680 252 A1

time is shorter than that required with the processes described in the state of the art, so the man-hours and machine-hours are reduced. It is an embodiment of the invention to provide a process described with 12 essential steps with greater productivity, and whose lyophilized final product has a proven stability for 48 months.

It is an embodiment of the invention the production of antivenoms against venoms of snake species or ofidios selected from the group of genus and species comprising: Cerastes genus, including the species: *C. boehmei, C. cerastes, C. gasperettii, C. vipera, Pseudocerastes fieldi,, Pseudocerastes persicus, Pseudocerastes urarachnoides;* genus Bitis, including *B. albanica, B. arietans, B. armata, B. Atropos, B. caudalis, B. cornuta, B. gabonica, B. harenna, B. heraldica, B. inornata, B. nasicornis, B. parviocula, B. peringueyi, B. rhinoceros, B. rubida, B. schneideri, B. worthingtoni, B. xeropaga* species; Crotalus genus, including species: *C. d. terrificus C. adamanteus C. angelensis C. aquilus C. armstrongi C. basiliscus C. campbelli, C. catalinensis, C. cerastes, C. cerberus, C. culminatus, C. durissus, C. enyo, C. ericsmithi, C. horridus, C. intermedius, C. lannomi, C. lepidus C. mitchellii, C. molossus, C. morulus, C. oreganus. C. ornatus, C. polystictus, C. pricei, C. pusillus, C. Pyrrhus, C. ravus, C. ruber, C. scutulatus, C. simus C. stejnegeri, C. stephensi, C. tancitarensis, C. tigris, C. tlaloci, C. totonacus, C. transversus, C. triseriatus, C. tzabcan, C. vegrandis, C. viridis, C. willardi; Calloselasma rhodostoma* species which belongs to wild Crotalinae family from the Southeast Asia from Thailand to northern Malaysia and on the island of Java; Lachesis genus, including species: *L. acrochorda, L. melanocephala, Lachesis muta, Lachesis stenophrys*; likewise, elapid species including cobras species *Naja naja siamensis* and the genus: *Acanthophi, Aipysurus, Antaioserpens, Aspidelaps Aspidomorphu, Austrelaps, Brachyurophis, Bungarus, Cacophis,Calliophis, Cryptophis, Demansia, Dendroaspis, Denisonia, Drysdalia, Echiopsis, Elapognathus, Elapsoidea, Emydocephalus, Enhydrina, Ephalophis, Furina, Hemachatus, Hemiaspis, Hemibungarus, Hoplocephalus"Hydrelaps, Hydrophis, Kolpophis, Laticauda, Loveridgelaps,Micropechis, Micruroides, Micrurus, Naja, Notechis, Ogmodon, Ophiophagus, Oxyuranus, Oxyuremus, Parahydrophis Parapistocalamus Parasut, Paroplocephalus, Pseudechis, Pseudohaje, Pseudonaja, Rhinoplocephalus, Salomonelaps, Sinomicrurus, Suta, Thalassophis, Toxicocalamus, Tropidechis, Vermicella* and *Walterinnesia.*

It is an embodiment of the invention to produce antivenoms designed against venoms of arachnid species selected from the group consisting of the Brazilian species: *Phoneutria nigriventer,* the Australian funnel spider species including the *Atrax robustus* species, and the species of the Hadronyche genus. The worldwide distribution species of Phoneutria genus, the Missulena genus and the Latrodectus genus, including the species: *L. bishopi, L. hesperus, L. mactans, L. variolus, L. antheratus, L. apicalis, L. corallinus, L. curacaviensis, L. diaguita, L. mirabilis, L. quartus, L. thoracicus, L. variegatus, L. tredecimguttatus, L. dahli, L. hystrix, L. lilianae, L. pallidus, L. revivensis, L. renivulvatus, L. tredecimguttatus, L. lugubris, L. cinctus, L. indistinctus, L. karrooensis, L. menavodi, L. obscurior, L. rhodesiensis, L. geometricus, L. elegans, L. erythromelas, L. ex laos.* Also, it is an embodiment of the invention the production of antivenoms designed against venoms of Sicariidae arachnid species (Sicariidae family) of both the Sicarius genus and the Loxosceles genus, including the species: *L. gaucho, L. intermedia, L. laeta, L. deserta, L. reclusa, L. acepta, L. adelaida, L. alamosa,, L.alicea, L. amazonica, L. anomala, L. apachea, L. aphrasta, L. aranea L. arizonica, L. aurea, L. baja, L. barbara, L. belli L. bentejui, L. bergeri, L. bettyae, L. blancasi, L. blanda, L.. candela, L. caribbaea, L. carmena, L. chapadensis, L. chinateca, L. colima, L. conococha, L. Coquimbo, L. coyote, L. cubana, L. devia, L. fontainei, L. foutadjalloni, L.francisca, L. frizzelli, L. gloria L. guajira, L. guatemala, L. harrietae, L. herreri, L. hirsuta, L. huasteca, L. hupalupa, L. immodesta, L. inca, L. insula, L. jaca, L. Jamaica, L. jarmila, L. julia, L. kaiba, L. lacroixi, L. lacta, L. lawrencei, L. lutea, L. luteola, L. mahan, L. maisi, L.manuela, L. martha, L. meruensis, L. misteca, L. mogote, L. mrazig, L. mulege, L. nahuana, L. neuvillei, L. niedeguidonae, L. olmea, L. pallidecolorata, L. palma, L. panama, L. parramae, L. pilosa, L. piura, L. pucara, L. puortoi, L. rica, L.rosana, L.rothi, L. rufescens, L. rufipes, L. russelli, L .sabina, L. seri, L. similis, L. simillima, L. smithi, L. sonora, L. spadicea, L. speluncarum, L. spinulosa, L. surca L. taeniopalpis L. taino L. tazarte L. tehuana L. Tenango, L. teresa, L. tibicena, L. tlacolula, L. unicolor, L. valdosa, L.valida, L. variegata L. virgo, L. vonwredei, L. weyrauchi, L. yucatana, L. zapoteca.*

It is an embodiment of the invention the production of antivenoms for scorpionism treatment caused by species or genera selected from the group consisting of: Androctonus genus including species: *Androctonus australis Androctonus mauretanicus mauretanicus, Androctonus crassicauda,* Buthacus genus including the species: *Buthacus macrocentrus,* Buthus genus, including the species *Buthus occitanus tunetanus,* Leiurus genus including the species *Leiurus quinquestriatus hebraeus,* Parabuthus genus including the species *Parabuthus granulatus,* Centruroides genus including the species: *Centruroides noxius, Centruroides limpidus, Centruroides suffusus,* Tityus genus, including species: *Tityus serrulatus, Tityus metuendus, Tityus matthieseni, Tityus bastosi, Tityus bahiensi.*

It is an embodiment of the invention the therapeutic applications using the antivenoms of the invention, in the example basic treatment schemes are mentioned.

It is the main object of the invention to provide a production and obtaining process of a composition of $F(ab')_2$ antibody fragments from hyperimmune plasma of a non-human mammal, capable of neutralizing venoms, characterized because it comprises:

   a) Mix of hyperimmune blood plasmas containing phenol (concentrate) in the reactor followed by enzymatic digestion by pepsin at pH 3.5 ± 0.1 with control and at a temperature of 37 ± 1° C for 1.5 h (90 minutes);

b) Stop the reaction by adjusting the pH to 4.2 + 0.2 with 5N NaOH 21% weight/volume;

c) Immediately proceed to the precipitation of unwanted protein fragments and adding 21% w/v ammonium sulfate. The soluble fraction of F(ab')$_2$ is obtained, which is the digested mixture. Where precipitation occurs as ammonium sulfate is diluted;

d) Immediately, proceed to clarification I and II, of the digested mixture obtained in iv, where clarification I is carried out with a pore size in a range of 8 µm to 20 µm and clarification II with a 0.2 µm nominal pore size, a solution of partially purified F(ab')$_2$ is obtained ready for ultrafiltration;

e) Proceed with automated ultrafiltration of the digested mixture

    a. By 30 kDa hollow-fiber membrane.
    b. By kDa 30 cassette and by 50 kDa cassette

f) Proceed with clarification II of the concentrate, determine the potency in LD$_{50}$ neutralizing/ml;

g) Formulate the product bulk. Adjust by dilution to obtain the specified potency for the product;

h) Add excipients: glycine, sodium chloride, sucrose;

i) Integrate a prefiltrate at 0.1 µm that leaves the product in better conditions to pass nanofiltration;

j) Eliminate the potential presence of virus by nanofiltration of the product bulk with a 20 nm membrane;

k) Proceed to sterile terminal filtration of the product bulk;

l) Proceed to the filling;

m) Lyophilize.

It is an embodiment of the process of the invention to dispense with the addition of cresol and/or ether. With the production process of F(ab')$_2$ antibody fragments claimed, the yield exceeds at least 15 times that obtained when purification is based on a process of double saline precipitation and dialysis. In an embodiment of the production process of F(ab')$_2$ antibody fragments, precipitation of unwanted protein fragments, step (c) above, is performed by adding 21% w/v ammonium sulfate at room temperature, during while the solubilization of ammonium sulfate lasts, and immediately after this step, clarification I and II of step (d) above are carried out. In another embodiment of the production and obtaining process of a composition of F(ab')$_2$ antibody fragments, the precipitation of unwanted protein fragments from step (c) above, is performed by adding 21% w/v ammonium sulfate to the time in which the temperature is increased to 54° C, during the time that the solubilization of the ammonium sulfate lasts, and immediately after this step, clarification I and II of the previous step (d) is carried out. In another embodiment of the process, the tangential filtration by 30 kDa hollow-fiber is carried out keeping an average τ of 4.06 Pa. In another embodiment, in step (e) the tangential filtration by 30 kDa cassette is carried out keeping an average τ of 2.05 Pa and 20 washes are performed and the subsequent tangential filtration by 50 kDa cassette is carried out keeping a τ of 0 and 20 washes are performed. It is an embodiment of the invention that in step (h) of the above production process said excipients are integrated in the following proportions depending on the amount of protein:

a)

$$[\text{volume (ml)}] \times [0.009 \ (\text{g/ml})] = \text{amount of sodium chloride (g)}$$

b)

$$[\text{amount of protein (g)}] \times [0.33] = \text{amount of sucrose (g)}$$

c)

$$[\text{amount of protein (g)}] \times [0.67] = \text{amount of glycine (g)}$$

Embodiments of the invention are the compositions obtained by said process in such a way that a degree of purity ≥ 95%, a percentage of Fab from 0 to 3%, a percentage of low molecular weight components from 0 to 0.90% and a percentage of high molecular weight components from 0 to 4.2% is obtained. Embodiments of the invention are compositions of the F(ab')$_2$ antibody fragments obtained by said process such that they are capable of neutralizing venoms with a potency that exceeds at least 6-fold that obtained when purification is based in a process of double saline precipitation and dialysis. The following examples are presented in an illustrative and non-limiting manner of the invention and refer to essential methods that enable the manufacturer based on processing a minimum representative volume of hyperimmune plasma to carry out each filtration process and thus the calculation for scaling to the volumes required for industrial production is made possible, for the entire obtaining and purifying process of the $F(ab')_2$ fraction of the invention. These characterizations can then be considered as embodiments of the invention.

### EXAMPLE 1: Immunization to obtain hyperimmune plasma.

Immunization schedules have been variable. An immunization example is to apply doses of venoms alone or in cocktails, native or recombinant, ranging between 3 and 150 $LD_{50}$ per horse over 12 immunizations given for 5 to 6 weeks for the base schemes and from 70 to 450 $LD_{50}$ per horse for 5 immunizations for 3 weeks. For reinforcement schemes, depending on the type of venom applied. Freund's complete and incomplete adjuvants can be used, as well as an isotonic saline solution, using a total of 5, 10 or 20 ml in the different inoculations. The source of antibodies used in the present invention can be obtained by plasmapheresis: bleeding at a rate from 6 to 10 liters per horse per bleeding, executing two bleeds during the 15 days following the last inoculation of each scheme the blood erythro-sediments and the entire cell pack is returned, thus reducing the stress associated with the fall of blood cells and producing the least possible impact on the animal's health despite being subjected to the production of antibodies.

Plasma is only preserved as the source of antibodies for the method of the present invention, it can be a mixture of blood plasmas from different animals immunized with the same antigen.

### EXAMPLE 2: Preparation of the pepsin solution to produce $F(ab')_2$ fragments.

To prepare 166.6 mL of the 1 mM HCl pepsin solution. 13.3 g of pepsin are weighed. 166.6 mL of the 1 mM HCl solution is added to a 500 mL bottle with magnetic stirrer, 13.3 g of pepsin are added thereto, then dissolved, and everything is at room temperature. This quantity is to process in a 5-liter reactor, 2 liters of plasma diluted with the same volume of process water. Pepsin is 6.6 g/l of plasma.

The pepsin solution is clarified, for which a membrane with a pore size from 1.0 to 3.0 $\mu$m is used. In order to sterilize, the membrane with a 0.2 $\mu$m pore size (absolute) previously wetted with process water, and flow adjustment as recommended by the equipment protocol.

### EXAMPLE 3: Enzymatic digestion of plasma to produce $F(ab')_2$ fragments.

It is carried out by integrating plasma and process water in a 50/50 % v/v ratio. For example, for 2 liters of plasma a 5-liter reactor can be used to which they are transferred using a peristaltic pump, the agitation speed of the reactor is adjusted to 300 rpm. 2 liters of process water are transferred to the 5-liter reactor with peristaltic pump. The 5-liter reactor agitation speed is adjusted to 300 rpm.

The temperature of the plasma + process water mixture is increased to 37° C $\pm$ 2° C. The pH is adjusted to 3.5 $\pm$ 0.2 with 5 N HCl. 166.6 mL of the sterile pepsin solution is added to the plasma + process water mixture located in the 5-liter reactor at a temperature of 37° C and a pH of 3.5 $\pm$ 0.2. The plasma + process water + sterile pepsin solution mixture located in the 5-liter reactor is maintained at a temperature of 37° C $\pm$ 2° C and a pH of 3.5 $\pm$ 0.2 for 90 minutes under constant stirring. To stop the enzymatic reaction, the pH is adjusted to 4.2 $\pm$ 0.2 of the digested plasma + process water + filtered pepsin solution mixture located in the 5-liter reactor at a temperature of 37° C $\pm$ 2° C with 5N NaOH.

### EXAMPLE 4: Salting-Out to obtain $F(ab')_2$ fragments.

Ammonium sulfate is added to the digested plasma + process water + pepsin solution mixture. For a 5-liter reactor, 840 g of ammonium sulfate is added to the digested plasma + process water + sterile pepsin solution mixture located in the 5-liter reactor at a temperature of 37° C $\pm$ 2° C, pH of 4.2 $\pm$ 0.2 and constant agitation.

### EXAMPLE 5: Clarification I and II of the supernatant from the precipitation (Salting-Out) to obtain $F(ab')_2$ fragments.

These clarifications aim to remove particles that damage the membranes of tangential filtration systems. In the clarifications the feed and pump flows are adjusted taking into account the performances of each membrane, the volumes and the areas, following mathematical relationships known in the state of the art. These examples are presented. For clarification I the membrane is with pore size from 8.0 to 20.0 $\mu$m, the system is fed with peristaltic pump, the feed flow is kept constant, the pressure increase to 25 psi indicates that the process must stop at this time, a density of the product to be processed of 1.2 mg/ml was considered, and a volume of 150 g (125 ml) was worked, the approximate flow: 6-7 ml/min, with a yield of 56 liters/m$^2$. Scaling to filter 60 and 120 liters the calculated area of the appropriate filter is 1.05 m$^2$ or 2.1 m$^2$, respectively. With an area of 1 m$^2$ the process takes 30 minutes applying a flow of 2,000 ml/min. This example is scalable.

Clarification II is carried out with a 0.2 $\mu$m pore size membrane. For a volume of 836 ml, the calculated feed flow is 12 ml/min and the Flux (LHM) of 956, the area of the membrane is 0.00138 m$^2$, in performance is 605 liters/m$^2$. Scaling to a 60-liter batch a membrane with 0.099 m$^2$ of area can be used. Scaling to a 120-liter batch a membrane with 0.198 m$^2$ of area can be used.

If a filtration area of 0.21 m$^2$ and a 0.2 $\mu$m nominal pore size, commercially available, is used, the theoretical process time is 30 minutes with a flow of 2,000 ml/min, but this is adjustable depending on the availability of commercial membranes or the volume to be filtered.

**EXAMPLE 6: Tangential filtration of the clarified to obtain F(ab')$_2$ fragments by 30 kDa hollow-fiber membrane.**

The membrane is polyester sulfone with 30 kDa cut size, the operating conditions (feed flow, recirculation time, volume/area ratio, transmembrane pressure, initial volume concentration factor, diafiltration volume and final volume concentration factor), were tested and selected internally. The purity obtained, the protein concentration and yield, is presented in table 13, it is the average of 7 tests where 500 ml per test were filtered in a 0.14 m$^2$ membrane, thus a volume/area ratio of 4 liters/m$^2$ is considered, the initial volume concentration factor is 3 X and 5 X final volume, the diafiltration volume is 20 and the process time is 4 hours. It works with an average $\tau$ value of 4.06 Pa; this allows the expected separation of the molecules, without causing the formation of aggregates or filter plugging.

**EXAMPLE 7: Tangential filtration of the clarified to obtain F(ab')$_2$ fragments per 30 kDa cassette.**

For 4 liters of clarified: 360 g of sodium chloride are weighed, 360 g of sodium are added and dissolved in 40 of process water. 4 liters of clarified are taken and recirculated for 5 min through the tangential filtration system (cassette with a 30 kDa cut size and a 0.5 m$^2$ area).

The product to ultrafiltrate is concentrated to 2 liters (2x). Diafiltration is done keeping the constant volume of 2 liters by adding a 0.9% sodium chloride solution with another pump until completing 40 liters of 0.9% sodium chloride (20 washes). The operating conditions (feed flow, recirculation time, volume/area ratio, transmembrane pressure, initial volume concentration factor, diafiltration volume and final volume concentration factor), were tested and selected internally, this allows the expected separation of the molecules, without causing the formation of aggregates or plugging of the cassette. It works with an average $\tau$ value of 2.05 Pa.

**EXAMPLE 8: Tangential filtration of the ultrafiltrate by 30 kDa to obtain F(ab')$_2$ fragments per 50 kDa cassette.**

To filter a volume of ultrafiltrate I, 0.4 liters are recirculated for 5 min through the tangential filtration system (50 kDa cut size cassette and a 0.5 m$^2$ area). Diafiltration is done keeping the constant volume of 0.4 liters by adding process water with another pump until complete 10 liters of process water (20 washes). The operating conditions (feed flow, recirculation time, volume/area ratio, transmembrane pressure, initial volume concentration factor, diafiltration volume and final volume concentration factor), were tested and selected internally, this allows the expected separation of the molecules, without causing the formation of aggregates or plugging of the cassette.

**EXAMPLE 9: Clarification III of the concentrate.**

A 0.2 $\mu$m pore size membrane (absolute) is prepared, moistening it with process water at a permeate and feed flow calculated according to its performance, volume and area of the system. For the example of the 5 L plasma processing, the pump flow setting is approximately 17 ml/min and the capsule is wetted with this membrane with 100 ml of process water. To filter the solution of F(ab')$_2$ fragments, the pump flow is adjusted to approximately 10 ml/min and the solution is clarified through the capsule with this membrane. Everything is done at room temperature.

Table 32.

| Flux$_{permeated}$ [LHM] | Volume solution [L] | Time clarification III [min] | Max Time clarification III [min] | Average Time clarification III [min] | Area process [m$^2$] | Feed flow fragment solution F(ab')2 [L/h] |
|---|---|---|---|---|---|---|
| 565 | 0.4 | 30 | 45 | 38 | 0.002 | 0.631 |

**EXAMPLE 10: Formulation of the F(ab')$_2$ fragments solution.**

From the potency results of the concentrate (clarified III of the formulation of the F(ab')$_2$ fragments solution and the described formulas, the final volume and amounts of excipients are calculated: sodium chloride, sucrose and glycine.

The dilution factor, % of excipients and their concentration are obtained:

$$\frac{\text{Concentrated potency } (\frac{\text{LD}_{50}}{\text{mL}})}{\text{Specified neutralizing potency } (\frac{\text{LD}_{50}}{\text{mL}})} = \text{Dilution factor}$$

$$[\text{Dilution factor}] \cdot [\text{Concentrated volume (mL)}] = \text{Final volume (mL)}$$

$$\frac{\text{Protein concentration of the concentrated}}{\text{Dilution factor}} = \text{Final protein concentration}$$

$$7\% \text{ total solids} - \text{Final protein concentration (\%)} = \text{Excipients(\%)}$$

The sum of proteins and excipients:

$$\frac{[\text{Final volume (mL)}] \cdot [\text{Excipients (\%)}]}{100 \%} = \text{Excipients(g)}$$

$$[\text{Volume (mL)}] \cdot [0.009] = \text{Sodium chloride (g)}$$

$$\text{Excipients (g)} - \text{Sodium chloride (g)} = (\text{Sucrose} + \text{Glycine})(\text{g})$$

$$[(\text{Sucrose} + \text{Glycine})(\text{g})] \cdot [0.33] = (\text{Sucrose})(\text{g})$$

$$[(\text{Sucrose} + \text{Glycine})(\text{g})] \cdot [0.67] = (\text{Glycine})(\text{g})$$

Depending on the amount of protein:

$$[\text{Protein concentration g/ml}] \cdot \text{Volume (ml)} = \text{Protein amount (g)}$$

$$[\text{volume (ml)}] \cdot \left[0.009 \left(\frac{\text{g}}{\text{ml}}\right)\right] = \text{Sodium chloride (g)}$$

$$[\text{Protein amount (g)}] \cdot [0.33] = \text{Sucrose amount (g)}$$

$$[\text{Protein amount (g)}] \cdot [0.67] = \text{Glycine amount (g)}$$

The amount of sodium chloride calculated is weighed. The amount of sucrose calculated is weighed. The amount of glycine calculated is weighed. The sodium chloride, sucrose and glycine calculated in 50% of the volume of process water is dissolved to reach the final volume in the formulation container. This last operation is omitted if it is not necessary to dilute because of the concentrate volume and then proceed to directly add the sodium chloride, sucrose and glycine. The volume of the formulated sodium chloride, sucrose and glycine is mixed with the volume of the concentrate to reach the final calculated volume of the formulation. All this step is done at room temperature.

It should be noted that it is feasible to adjust the pH of the by-product at any time from step 4 of table 21. At this point it is possible to adjust the pH to 6.9 $\pm$ 0.1 with borate buffer (pH 12-13).

**EXAMPLE 11: Prefiltration of the formulated F(ab')$_2$ fragment solution and final nanofiltration.**

Prefiltration and nanofiltration were integrated in Table 21 as steps 8 and 9 of the process of the invention. To obtain the F(ab')$_2$ fragments solution as a finished product, it is required that the formulated bulk be prefiltered and then subjected to a viral clarification by nanofiltration. The prefiltrate membrane is 0.1 $\mu$m (absolute).

For this example, used to characterize the prefiltering stage 8 (not limiting), for a volume of 165 ml, the average flow is 15 ml/min and the Flux (LHM) is 478, the membrane area is 0.00138 m$^2$, the yield is 119.8 liters/m$^2$. Scaling for 40 liters batches a membrane with 0.335 m$^2$ of area can be used. If a filtration area of 0.33 m$^2$ available on the market is used, the theoretical time of the process is 12 minutes with a 10 psi pressure. But this is adjustable depending on the availability of commercial membranes.

This example characterizes the nanofiltration stage 9 but is not limiting. A modified polyvinylidene difluoride (PVDF) hydrophilic membrane with a 20 nm pore size (absolute) is used. It was characterized for a volume to be filtered of 45 ml using a pressure of 45 psi; three samples were tested as well and with this it was observed that the flows show variations from the beginning to the end of the process, the process behavior was averaged; at the beginning the average is 0.64 ml/min (LHM: 34.9), in the end the average is 0.29 ml/min (LHM: 16). To scale to 40-liter batches, a membrane with 0.986 m$^2$ of area can be used. The system can be adapted with a filtration area of 1 m$^2$ available in the market to process 40 liters. All this step is done at room temperature.

**EXAMPLE 12: Sterile terminal filtration of the formulated F(ab')$_2$ fragment solution.**

In order to obtain the F(ab')$_2$ fragment solution as a finished product, it is required that the formulated bulk be sterilized by membrane (step 10 of table 21). A 0.2 $\mu$m sterilizing grade membrane is used. The system is powered by a peristaltic pump, the system pressure increase is 2 psi. For this example, used to characterize this stage (not limiting), for a volume of 495 ml, the flow is 12.4 ml/min and the Flux (LHM) is 538, the membrane area is 0.00138 m$^2$. Climbing to a 40-liter batch, a membrane with 0.111 m$^2$ area can be used. If a 0.15 m$^2$ filtration area available on the market is used, the theoretical time of the process is 30 minutes with a flow of 1,300 ml/min, but this is adjustable depending on the availability of commercial membranes.

**EXAMPLE 13: Lyophilization.**

The product bottles are frozen for 8 hours at -70° C and 1000 mBar, the main drying is done at -20° C (plate temperature), 0 mBar for 63 hours and the final drying at 30° C (plate temperature), 0 mBar for 8 hours. The main drying and the final drying are carried out with the freeze-drying chamber at a temperature of -60° C. These conditions are established for an operating volume of 125 ml (25 vials).

**Example 14: Verification of cresol and phenol removal for each of the obtaining process stages of F(ab')$_2$ fragments at the pilot plant level.**

Equine plasma samples were subjected to the pilot plant process, samples were taken from each stage: digestion with pepsin, precipitation, thermocoagulation, and diafiltration embodiments: the tangential with hollow-fiber of 30 kDa cut size and the one made with 30 kDa and 50 kDa cassette systems. Subsequent samples were also taken: the last clarified, the prefiltered formulated product, the nanofiltered and the finished product (PT). For each modality, samples were taken at each stage and at each 2X concentration, diafiltrates 5, 10, 15 and 20 washes and samples were taken in the 10X concentration, diafiltrates 5, 10, 15 and 20 washes.

As a control, or comparison blank, cresol and phenol quantification was performed from the tangential filtration test with cassette systems with a 30 kDa and 50 kDa cut size with 0.9% sodium chloride washes from a solution of 0.9% sodium chloride and on the other hand using process water. For which samples were taken in the 2X concentration, diafiltrates 5, 10, 15 and 20 washes and samples were taken in the 10X concentration, diafiltrates 5, 10, 15 and 20 washes. All samples were analyzed by the validated analytical method for cresol determination.

**EXAMPLE 15: Therapeutic applications**

The antivenoms against scorpion venom can be used according to the degree of poisoning under the following scheme (table 33):

Table 33:

| Degree of poisoning | Clinical data | Treatment schemei.v. |
|---|---|---|
| I mild | Pain at the site of the bite<br>Local paresthesia<br>Profuse salivation | 1 vial at any age |
| II moderate | General paresthesia<br>Nose and throat itching | 2 vials under 15<br>1 vial over 15 |
| III severe | • Sensation of an object in the throat<br>• Nistagmus<br>• Abdominal distension<br>• Lingual fasciculations<br>• Convulsions<br>• Vomiting<br>• Transient blindness<br>• Dyspnea<br>• Retrosternal pain arterial hypertension | 3 vials under 15<br>2 vials over 15 |

Antivenoms against snake venom (Elapidae) can be used according to the degree of poisoning under the following scheme (table 34):

Table 34.

| Degree of poisoning | Clinical data | Treatment in children i.v. | | Treatment in adults i.v. | |
|---|---|---|---|---|---|
| | | Initial | Maintenance | Initial | Maintenance |
| I mild | Bite history<br>Fang marks<br>Local pain<br>Bleeding from the wound<br>Local paresthesia<br>Local edema | Observation | | Observation | |
| II Moderate | Weakness<br>Adimia<br>Blepharoptosis<br>Blurry vision<br>Ophthalmoplegia<br>Diplopia<br>Dyspnea | 6 to 10 vials | 5 vials | 3 to 5 vials | 5 vials |
| III Severe | Pain in the lower jaw<br>Profuse salivation<br>Absence of deep reflexes<br>Flaccid paralysis<br>Moderate dyspnea<br>Dysphagia<br>Respiratory faliure | 15 vials | 5 vials | 6 to 10 vials | 5 vials |

(continued)

| Degree of poisoning | Clinical data | Treatment in children i.v. | | Treatment in adults i.v. | |
|---|---|---|---|---|---|
| | | Initial | Maintenance | Initial | Maintenance |
| | Coma | | | | |

The present invention is a high-performance process for obtaining a safe and effective antidote to act against the toxic activity of venoms. The antidote is a composition based on F(ab')$_2$ fragments of IgG antibodies, which when administered in mammals produce passive immunity. The process of the present invention allows to obtain the F(ab')$_2$ fragments of polyclonal origin and are characterized by their potency and purity.

With the process of the present invention, the biological activity and the purity of the final products are increased, in addition, it is also a better performance process, so that man hours are saved, and the production costs and times are reduced for composition of this nature.

References

Ahrer K., Buchacher A., Iberer G., Jungbauer A. (2006). Effects of ultra-/diafiltration conditions on present aggregates in human immunoglobulin G preparations. Journal of Membrane Science 274, 108-115

Alvarenga, L. M., Zahid, M., di Tommaso, A., Juste, M. O., Aubrey, N., Billiald, P., Et al. (2014). Engineering Venom's Toxin-Neutralizing Antibody Fragments and Its Therapeutic Potential. Toxins (6), 2541-2537.

Calmette. (1896). The treatment of animals poisoned with snake venom by the injection of antivenomous serum. Br. Med. J., 2, pp. 399-400.

Calvete, J.J., Juárez, P. y Sanz, L. (2007) Snake venomics. Strategy and applications. J. Mass Spectrom. 42, 1405-1414.

CN 103864930 (2014). Zheng Ying, Et al.

CN 101816789 (2010). Quanshui Fan, Et al.

Chen X1, Jensen PE. The role of B lymphocytes as antigen-presenting cells. Arch Immunol Ther Exp (Warsz). 2008 Mar-Apr;56(2):77-83. Epub 2008 Mar 31.

Chen, Jensen P. E. (2008). The role of B lymphocytes as antigen-presenting cells. Arch Immunol Ther Exp (Warsz). 2008 Mar-Apr;56(2):77-83.

Chippaux JP (1998). Snake-bites: appraisal of the global situation. Bulletin of the World Health Organization, 76(5), 515-24.

De Lima M.E., Martin-Eauclaire M.F., Chavez-Olortegui C., Diniz C.R., Granier C. (1993). Tityus serrulatus scorpion venom toxins display a complex pattern of antigenic reactivity. Toxicon. Feb;31(2):223-7.

ES 2549690 (2015). Lopez de Silanes.

FEUM0 Farmacopea de los Estados Unidos Mexicanos, Secretaría de Salud, 10a ed Mexico 2011. Tomo 1 y 2, Pág. 279-293, 2367-2369. Y Especificación interna del Instituto Bioclon SA de CV.

FEUM1 Farmacopea de los Estados Unidos Mexicanos, Secretaría de Salud, 10a ed Mexico 2011. Tomo 1 y 2, Pág. 449, 450, 2316, 2367-2369. USP 34 and NF 29 Pág. 115-116.

FEUM2 Farmacopea de los Estados Unidos Mexicanos, Secretaría de Salud, 10a ed Mexico 2011. Tomo 2, Pág. 2292, 2293, 2316, 2367-2369.

FEUM3 Farmacopea de los Estados Unidos Mexicanos, Secretaría de Salud, 10a ed Mexico 2011. Tomo 2, Pág. 2367-2369.

FEUM4 Farmacopea de los Estados Unidos Mexicanos, Secretaría de Salud, 10a ed Mexico 2011. Pág. 2435. MPB0260. Determinación de cresol y fenol en productos biologicos.

Fox S, Rathuwithana AC, Kasturiratne A, Lalloo DG, de Silva, HJ (2006). Underestimation of snakebite mortality by hospital statistics in the Monaragala District of Sri Lanka. Transactions of the Royal Society of Tropical Medicine and Hygiene, 100(7):693-5.

Gerringa D., Kingb T.R., Brantonc, R. (2013). Validating a faster method for reconstitution of Crotalidae Polyvalent Immune Fab (ovine). Toxicon. Volume 69, July 2013, Pages 42-49

Garavito, A.; Espíndola, M. (1995). Proceso de Concentración de Proteína de Suero de Leche por Ultrafiltración. Proyecto de Grado para optar al título de Ingeniero Químico. Planta de Vegetales - ICTA, Universidad Nacional de Colombia.

Ghosh, R., Cui, Z.F. (2000). Protein purification by ultrafiltration with pre-treated membrane. Journal of Membrane Science. Volume 167, Issue 1, 14 March 2000, Pages 47-53.

Gutiérrez JM, Theakston RDG, Warrell DA (2006). Confronting the neglected problem of snake bite envenoming: the need for a global partnership. PLoS-Medicine Jun 6;3(6):0727-0731.e150.

Gwee, M.C.E., Nirthanan, S., Khoo, H.E., Gopalakrihnakone, P., Kini, R.M., Cheah, L.S., (2002). Autonomic effects of some scorpion venoms and toxins. Clin. Exp. Pharm. Physiol. 29, 795-801.

Harms A.J. The purification of antitoxic plasmas by enzyme treatment and heat denaturation. Biochem J. 1948;42:390-397.

Heinen T. E. y Gorini da Veiga A. B. (2011) Review Arthropod venoms and cancer. Toxicon 57, 497-511.

Hemke, V. Journal of Cell and Tissue research Vol. 13(1) 3479-3484.

Landon J. et al. (2014). Single-reagent one-step procedures for the purification of ovine IgG, F(ab')2 and Fab antivenoms by caprylic acid. Journal of Immunological Methods 402 (2014) 15-22.

Landon y Jones (2002). Enhanced pepsin digestion: a novel process for purifying antibody F(ab')2 fragments in high yield from serum. Journal of Immunological Methods 263 (2002) 57-74.

Landon y Jones (2003). A protocol for 'enhanced pepsin digestion': a step by step method for obtaining pure antibody fragments in high yield from serum Journal of Immunological Methods 275 (2003) 239-250.

Lourenço, W.R., (1994). Diversity and endemism in tropical versus temperate scorpion communities. Biogeographica 70, 155-160.

Martin-Eauclaire, M.F., Couraud, F., (1995). Scorpion neurotoxins: effects and mechanisms. In: Chang, L.W., Dyer, R.S. (Eds.), Handbook of Neurotoxicology. Marcell and Dekker, New York, pp. 683-716.

MintonJr. S. A. Common Antigens in Snake Venoms. Capítulo: Venenos de serpientes. Volumen 52 de la serie Handbook of Experimental Pharmacology pp 847-862.

Mitchell, Silas Weir, and Edward T. Reichert. Researches Upon the Venoms of Poisonous Serpents. Washington: Smithsonian Institution, 1886.

Pope C.G. (1938). Dissagregation of proteins by enzymes. Br J Exp Path: 245-251.

Pope, G.G., (1939a). The action of proteolytic enzymes on antitoxins and proteins of immune sera. I. True digestion of the protein. Br. J. Exp. Pathol. 20, 132-149.

Pope, G.G., (1939b). The action of proteolytic enzymes on antitoxins and proteins of immune ser. II. Heat denaturation after partial enzyme action. Br. J. Exp. Pathol. 20, 201-212.

Possani, L.D., Merino, E., Corona, M., Bolivar, F., Becerril, B., (2000). Peptides and genes coding for scorpion toxins that affect ion-channels. Biochimie 82, 861-868.

Pugh RN, Theakston RD, Reid HA (1980). Malumfashi Endemic Diseases Research Project, XIII. Epidemiology of human encounters with the spitting cobra, Naja nigricollis, in the Malumfashi area of northern Nigeria. Annals of Tropical Medicine and Parasitology, 74(5):523-30.

Rodriguez-Pinto. (2005). Review. B cells as antigen presenting cells Cellular Immunology. 238. 67-75 www.elsevier.com/locate/ycimm

Saetang, T., Treamwattana, N., Suttijitpaisal, P., Ratanabanangkoon, K., (1997). Quantitative comparison on the refinement of horse antivenom by salt fractionation and ion-exchange chromatography. J. Chromatogr., B: Biomed. Sci. Appl. 700, 233.

Sharma SK et al. (2004). Impact of snake bites and determinants of fatal outcomes in southeastern Nepal. American Journal of Tropical Medicine and Hygiene, 2004, 71(2):234-238.

Simard, M.J., Watt, D.D., (1990). Venoms and toxins. In: Polis, G.A. (Ed.), The Biology of Scorpions. Stanford University Press, Standford, pp. 414-444. Smith, K.J.

Snow RW et al. (1994). The prevalence and morbidity of snake bite and treatment-seeking behaviour among a rural Kenyan population. Annals of Tropical Medicine and Parasitology, 88(6):665-71.

US 4,814,433 (1989). Fredrickson R.

US 4,849,352 (1989). Sullivan et al.

US 5,733,742 (1998). Landon et al,

US 6,709,655 (2004). Lopez de Silanes, et al.

US 8,048,414 (2011). Sullivan et al

Warrell DA, Arnett C (1976). The importance of bites by the saw-scaled or carpet viper (Echis carinatus): epidemiological studies in Nigeria and a review of the world literature. Acta Tropica, 33(4):307-41.

Wolfenden, R. N. (1886). On the nature and action of the venom of poisonous snakes. J. Physiol. (London.) 7, 326-324.

Williams, D., Gutiérrez, J.-M., Calvete, J., Wüster, W., Ratanabanangkoon, K., Paiva, O., y otros. (2011). Ending the drought: New strategies for improving the flow of affordable, effective antivenoms in Asia and Africa. Journal of Proteomics (74), 1735-1767.

**Claims**

1. Production and obtaining process of a composition of F(ab')$_2$ antibody fragments capable of neutralizing poisons from non-human mammal hyperimmune plasma, wherein it comprises:

   a) mix of hyperimmune blood plasma containing phenol in the reactor followed by enzymatic digestion with pepsin at pH 3.5 $\pm$ 0.1 with control and a temperature of 37 $\pm$ 1° C for 90 minutes;
   b) stop the reaction by adjusting the pH at 4.2 + 0.2 with 5N NaOH 21% w/v;
   c) immediately proceed to the precipitation of unwanted protein fragments and adding 21% w/v of ammonium sulfate; the soluble fraction of F(ab')$_2$ is obtained, which is the digested mixture; where precipitation occurs as ammonium sulfate is diluted;
   d) immediately proceed to clarification I and II of the digested mixture obtained in (c), where clarification I is carried out with a pore size in a range from 8 $\mu$m to 20 $\mu$m and clarification II with a nominal 0.2 $\mu$m pore size; a solution of partially purified F(ab')$_2$ is obtained ready for ultrafiltration;
   e) proceed with automated ultrafiltration of the digested mixture

   - By a 30 kDa hollow-fiber membrane.
   - By a 30 kDa cassette and 50 kDa cassette

   f) proceed with clarification II of the concentrate, determine the potency in LD$_{50}$ neutralizing/ml;
   g) formulate the bulk of the product and adjust by dilution to obtain the specified potency for the product;
   h) add excipients: glycine, sodium chloride, sucrose;
   i) integrate a pre-filtrate at 0.1 $\mu$m which leaves the product in better conditions for passing to nanofiltration;
   j) remove the potential presence of virus by nanofiltration of the bulk of the product with a 20 nm membrane;
   k) proceed with a sterile terminal filtration of the bulk of the product;
   l) proceed to filling;
   m) lyophilize.

2. The production and obtaining process of a composition of F(ab')$_2$ antibody fragments of claim 1 or 2, wherein cresol and/or ethyl ether addition is expendable.

3. The production and obtaining process of a composition of F(ab')$_2$ antibody fragments of claim 1, 2 or 3, wherein the precipitation of unwanted protein fragments from step (c) is carried out by adding 21% w/v of ammonium sulfate at room temperature, during the time the ammonium sulfate solubilization lasts, and immediately at this step, clarification I and II of step (d) are carried out.

4. The production and obtaining process of a composition of F(ab')$_2$ antibody fragments of claim 1, 2 or 3, wherein the precipitation of unwanted protein fragments from step (c) is carried out by adding 21% w/v of ammonium sulfate at room temperature, at the time the temperature is increased to 54° C, during the time the ammonium sulfate solubilization lasts, and immediately at this step, clarification I and II of step (d) are carried out.

5. The production and obtaining process of a composition of F(ab')$_2$ antibody fragments of claim 1, 2, 3, or 5, wherein in step (e), the tangential filtration by 30 kDa hollow-fiber is carried out keeping an average $\tau$ of 4.06 Pa.

6. The production and obtaining process of a composition of F(ab')$_2$ antibody fragments of claim 1, 2, 3, 4 or 5, wherein in step (e) tangential filtration by 30 kDa cassette is performed keeping an average $\tau$ of 2.05 Pa and 20 washes are performed and the subsequent tangential filtration by 50 kDa cassette is performed keeping a $\tau$ of 0 and 20 washes are performed.

7. The process according to any of claims 1 to 6, wherein further in step (h) said excipients are integrated in the following proportions depending on the amount of protein:

   a)

$$[\text{volume (ml)}] \times [0.009 \text{ (g/ml)}] = \text{Amount of sodium chloride (g)}$$

b)

$$[\text{amount of protein (g)}] \times [0.33] = \text{amount of sucrose (g)}$$

c)

$$[\text{amount of protein (g)}] \times [0.67] = \text{amount of glycine (g)}$$

8. The production and obtaining process of a composition of F(ab')$_2$ antibody fragments of any of claims 1 to 7, wherein the degree of purity obtained is $\geq$ 95%.

9. The production and obtaining process of a composition of F(ab')$_2$ antibody fragments of any of claims 1 to 8, wherein the percentage of obtained Fab is from 0 to 3%, the percentage of obtained low molecular weight components ranges from 0 to 0.90% and the percentage of obtained high molecular weight components ranges from 0 to 4.2%.

10. The production and obtaining process of a composition of F(ab')$_2$ antibody fragments of any of claims 1 to 9, wherein the yield exceeds at least 15 times that obtained when purification is based on a process of double saline precipitation and dialysis.

11. The production and obtaining process of a composition of F(ab')$_2$ antibody fragments capable of neutralizing poisons from non-human mammal hyperimmune plasma, of any of claims 1 to 10, wherein the potency exceeds at least 6 times that obtained when purification is based on a process of double saline precipitation and dialysis.

12. A composition of F(ab')$_2$ antibody fragments capable of neutralizing poisons from non-human mammal hyperimmune plasma, wherein it is obtained by the process of any of claims 1, 2, 3, 4, 5, 6 or 7 and further wherein:

n) the degree of purity obtained is > 95%.
o) the percentage of obtained Fab is from 0 to 3%,
p) the percentage of obtained low molecular weight components ranges from 0 to 0.90%
q) the percentage of obtained high molecular weight components ranges from 0 to 4.2%.

13. The composition of F(ab')$_2$ antibody fragments capable of neutralizing poisons from non-human mammal hyperimmune plasma of claim 12, wherein further depending on the amount of protein it contains the following proportions of excipients:

r)

$$[\text{volume (ml)}] \times [0.009 \text{ (g/ml)}] = \text{amount of sodium chloride (g)}$$

s)

$$[\text{amount of protein (g)}] \times [0.33] = \text{amount of sucrose (g)}$$

t)

$$[\text{amount of protein (g)}] \times [0.67] = \text{amount of glycine (g)}.$$

Figure 1

Figure 2

SAMPLE INFORMATION

| | | | |
|---|---|---|---|
| Sample Name: | Q050814 | Acquired By: | System |
| Sample Type: | Unknown | Sample Set Name: | fraccionamientos_Plasma |
| Vial: | 21 | Acq. Method Set: | Isocratico 075 mL MS |
| Injection #: | 1 | Processing Method | ApexTrack_BioSuite_Precol_IgG |
| Injection Volume: | 75.00 ul | Channel Name: | 2487Channel 1 |
| Run Time: | 32.0 Minutes | Proc. Chnl. Descr.: | 280 nm |
| Date Acquired: | 8/12/2014 11:57:07 PM CDT | | |
| Date Processed: | 8/13/2014 8:44:35 AM CDT | | |

| | Peak Name | RT | Area | % Area | Height |
|---|---|---|---|---|---|
| 1 | | 8.053 | 100068 | 0.18 | 1908 |
| 2 | | 10.897 | 90629 | 0.16 | 1763 |
| 3 | IgG | 11.735 | | | |
| 4 | F(ab')2 | 12.813 | 12514254 | 22.25 | 164964 |
| 5 | Fab | 14.836 | | | |
| 6 | | 16.531 | 1794404 | 3.19 | 29030 |
| 7 | | 18.645 | 20574981 | 36.59 | 168800 |

**Figure 3A**

SAMPLE   INFORMATION

| | | | |
|---|---|---|---|
| Sample Name: | R050814 | Acquired By: | System |
| Sample Type: | Unknown | Sample Set Name: | fraccionamientos_Plasma |
| Vial: | 22 | Acq. Method Set: | Isocratico 075 mL MS |
| Injection #: | 1 | Processing Methoc | ApexTrack_BioSuite_Precol_IgG |
| Injection Volume: | 75.00 ul | Channel Name: | 2487Channel 1 |
| Run Time: | 32.0 Minutes | Proc. Chnl. Descr.: | 280 nm |
| | | | |
| Date Acquired: | 8/13/2014 12:30:44 AM CDT | | |
| Date Processed: | 8/13/2014 8:44:35 AM CDT | | |

| | Peak Name | RT | Area | % Area | Height |
|---|---|---|---|---|---|
| 1 | | 8.234 | 5234 | 0.01 | 145 |
| 2 | | 10.236 | 2855 | 0.00 | 169 |
| 3 | | 10.811 | 18105 | 0.03 | 524 |
| 4 | IgG | 11.735 | | | |
| 5 | F(ab')2 | 12.815 | 7865628 | 12.95 | 113934 |
| 6 | Fab | 14.836 | | | |
| 7 | | 16.524 | 2439934 | 4.02 | 34969 |

| | Peak Name | RT | Area | % Area | Height |
|---|---|---|---|---|---|
| 8 | | 18.587 | 25587788 | 42.13 | 245060 |
| 9 | | 20.725 | 2977854 | 4.90 | 83520 |
| 10 | | 21.171 | 5890576 | 9.70 | 80455 |
| 11 | | 24.371 | 15945156 | 26.25 | 375899 |

**Figure 3B**

## SAMPLE INFORMATION

| | | | |
|---|---|---|---|
| Sample Name: | A050814 | Acquired By: | System |
| Sample Type: | Unknown | Sample Set Name: | fraccionamientos_Plasma |
| Vial: | 19 | Acq. Method Set: | Isocratico 075 mL MS |
| Injection #: | 1 | Processing Methoc | ApexTrack_BioSuite_Precol_IgG |
| Injection Volume: | 75.00 ul | Channel Name: | 2487Channel 1 |
| Run Time: | 32.0 Minutes | Proc. Chnl. Descr.: | 280 nm |

Date Acquired: 8/12/2014 10:49:51 PM CDT
Date Processed: 8/13/2014 8:44:35 AM CDT

| | Peak Name | RT | Area | % Area | Height |
|---|---|---|---|---|---|
| 1 | | 8.338 | 3149061 | 5.15 | 91894 |
| 2 | | 10.152 | 1552167 | 2.54 | 31729 |
| 3 | IgG | 11.698 | 35655470 | 58.26 | 531644 |
| 4 | F(ab')2 | 13.005 | 8806351 | 14.39 | 186167 |
| 5 | Fab | 14.836 | | | |
| 6 | | 17.938 | 441276 | 0.72 | 14917 |
| 7 | | 19.477 | 305207 | 0.50 | 8406 |
| 8 | | 21.808 | 99327 | 0.16 | 2847 |
| 9 | | 24.459 | 11188136 | 18.28 | 274689 |

**Figure 4a**

## SAMPLE INFORMATION

| | |
|---|---|
| Sample Name: | B050814 |
| Sample Type: | Unknown |
| Vial: | 9 |
| Injection #: | 1 |
| Injection Volume: | 75.00 ul |
| Run Time: | 32.0 Minutes |

| | |
|---|---|
| Acquired By: | System |
| Sample Set Name: | fraccionamientos_Plasma |
| Acq. Method Set: | Isocratico 075 mL MS |
| Processing Method | ApexTrack_BioSuite_Precol_IgG |
| Channel Name: | 2487Channel 1 |
| Proc. Chnl. Descr.: | 280 nm |

| | |
|---|---|
| Date Acquired: | 8/12/2014 4:06:05 PM CDT |
| Date Processed: | 8/13/2014 8:44:35 AM CDT |

| | Peak Name | RT | Area | % Area | Height |
|---|---|---|---|---|---|
| 1 | | 8.349 | 677940 | 3.94 | 21679 |
| 2 | | 10.158 | 422082 | 2.45 | 8515 |
| 3 | IgG | 11.694 | 10143508 | 58.94 | 151150 |
| 4 | F(ab')2 | 13.035 | 2427404 | 14.10 | 50966 |
| 5 | Fab | 14.836 | | | |
| 6 | | 17.943 | 171566 | 1.00 | 5060 |
| 7 | | 19.480 | 107974 | 0.63 | 2552 |

| | Peak Name | RT | Area | % Area | Height |
|---|---|---|---|---|---|
| 8 | | 20.376 | 14340 | 0.08 | 428 |
| 9 | | 21.774 | 28444 | 0.17 | 668 |
| 10 | | 23.603 | 11955 | 0.07 | 404 |
| 11 | | 24.677 | 3204776 | 18.62 | 81269 |

**Figure 4b**

## SAMPLE INFORMATION

| | | | |
|---|---|---|---|
| Sample Name: | C050814 | Acquired By: | System |
| Sample Type: | Unknown | Sample Set Name: | fraccionamientos_Plasma |
| Vial: | 20 | Acq. Method Set: | Isocratico 075 mL MS |
| Injection #: | 1 | Processing Methoc | ApexTrack_BioSuite_Precol_IgG |
| Injection Volume: | 75.00 ul | Channel Name: | 2487Channel 1 |
| Run Time: | 32.0 Minutes | Proc. Chnl. Descr.: | 280 nm |
| | | | |
| Date Acquired: | 8/12/2014 11:23:30 PM CDT | | |
| Date Processed: | 8/13/2014 8:44:35 AM CDT | | |

| | Peak Name | RT | Area | % Area | Height |
|---|---|---|---|---|---|
| 1 | | 8.290 | 4203275 | 26.87 | 107429 |
| 2 | IgG | 11.771 | 2809575 | 17.96 | 41260 |
| 3 | F(ab')2 | 12.835 | 3379023 | 21.60 | 42750 |
| 4 | Fab | 14.502 | 534640 | 3.42 | 8352 |
| 5 | | 15.873 | 193166 | 1.23 | 4563 |
| 6 | | 16.426 | 145540 | 0.93 | 4407 |
| 7 | | 17.940 | 719852 | 4.60 | 10020 |

| | Peak Name | RT | Area | % Area | Height |
|---|---|---|---|---|---|
| 8 | | 19.390 | 320357 | 2.05 | 5045 |
| 9 | | 20.594 | 129754 | 0.83 | 2448 |
| 10 | | 21.801 | 111789 | 0.71 | 2356 |
| 11 | | 23.465 | 20493 | 0.13 | 516 |
| 12 | | 24.646 | 3078027 | 19.67 | 78076 |

**Figure 4c**

SAMPLE INFORMATION

| | | | |
|---|---|---|---|
| Sample Name: | F050814 | Acquired By: | System |
| Sample Type: | Unknown | Sample Set Name: | fraccionamientos_Plasma |
| Vial: | 10 | Acq. Method Set: | Isocratico 075 mL MS |
| Injection #: | 1 | Processing Methoc | ApexTrack_BioSuite_Precol_IgG |
| Injection Volume: | 75.00 ul | Channel Name: | 2487Channel 1 |
| Run Time: | 32.0 Minutes | Proc. Chnl. Descr.: | 280 nm |
| | | | |
| Date Acquired: | 8/12/2014 4:39:44 PM CDT | | |
| Date Processed: | 8/13/2014 8:44:35 AM CDT | | |

| | Peak Name | RT | Area | % Area | Height |
|---|---|---|---|---|---|
| 1 | | 8.365 | 1384485 | 6.28 | 20327 |
| 2 | | 10.693 | 356709 | 1.62 | 6113 |
| 3 | IgG | 11.735 | | | |
| 4 | F(ab')2 | 12.860 | 6520425 | 29.57 | 81119 |
| 5 | Fab | 15.288 | 393212 | 1.78 | 8316 |
| 6 | | 16.437 | 608509 | 2.76 | 11991 |
| 7 | | 18.006 | 2970731 | 13.47 | 43339 |

| | Peak Name | RT | Area | % Area | Height |
|---|---|---|---|---|---|
| 8 | | 18.906 | 2846428 | 12.91 | 35429 |
| 9 | | 21.077 | 1074081 | 4.87 | 17835 |
| 10 | | 21.703 | 785703 | 3.56 | 13866 |
| 11 | | 24.609 | 5111988 | 23.18 | 124919 |

**Figure 4d**

| | | | | SAMPLE INFORMATION | | | |

| | SAMPLE | INFORMATION | |
|---|---|---|---|
| Sample Name: | I050814 | Acquired By: | System |
| Sample Type: | Unknown | Sample Set Name: | fraccionamientos_Plasma |
| Vial: | 11 | Acq. Method Set: | Isocratico 075 mL MS |
| Injection #: | 1 | Processing Methoc | ApexTrack_BioSuite_Precol_IgG |
| Injection Volume: | 75.00 ul | Channel Name: | 2487Channel 1 |
| Run Time: | 32.0 Minutes | Proc. Chnl. Descr.: | 280 nm |
| | | | |
| Date Acquired: | 8/12/2014 5:13:23 PM CDT | | |
| Date Processed: | 8/13/2014 8:44:35 AM CDT | | |

| | Peak Name | RT | Area | % Area | Height |
|---|---|---|---|---|---|
| 1 | | 8.313 | 2316322 | 8.66 | 48870 |
| 2 | | 10.626 | 279288 | 1.04 | 5414 |
| 3 | IgG | 11.735 | | | |
| 4 | F(ab')2 | 12.861 | 6770519 | 25.32 | 83707 |
| 5 | Fab | 15.271 | 314573 | 1.18 | 8753 |
| 6 | | 16.435 | 770183 | 2.88 | 14173 |
| 7 | | 18.037 | 3922294 | 14.67 | 57739 |

| | Peak Name | RT | Area | % Area | Height |
|---|---|---|---|---|---|
| 8 | | 18.843 | 3819602 | 14.28 | 46482 |
| 9 | | 20.976 | 1300904 | 4.86 | 24241 |
| 10 | | 21.755 | 1208992 | 4.52 | 20285 |
| 11 | | 24.587 | 6042017 | 22.59 | 145462 |

**Figure 4e**

64

SAMPLE INFORMATION

Sample Name: C070814
Sample Type: Unknown
Vial: 24
Injection #: 1
Injection Volume: 75.00 ul
Run Time: 32.0 Minutes

Date Acquired: 8/13/2014 2:11:43 AM CDT
Date Processed: 8/13/2014 8:44:35 AM CDT

Acquired By: System
Sample Set Name: fraccionamientos_Plasma
Acq. Method Set: Isocratico 075 mL MS
Processing Method ApexTrack_BioSuite_Precol_IgG
Channel Name: 2487Channel 1
Proc. Chnl. Descr.: 280 nm

| | Peak Name | RT | Area | % Area | Height |
|---|---|---|---|---|---|
| 1 | | 8.070 | 32066 | 0.20 | 1674 |
| 2 | | 8.410 | 68815 | 0.42 | 1993 |
| 3 | | 9.456 | 59844 | 0.37 | 1187 |
| 4 | | 10.836 | 134188 | 0.82 | 2723 |
| 5 | IgG | 11.735 | | | |
| 6 | F(ab')2 | 12.757 | 15953995 | 97.99 | 241324 |
| 7 | Fab | 14.836 | | | |

**Figure 4f**

65

## SAMPLE INFORMATION

| | | | |
|---|---|---|---|
| Sample Name: | C080814 | Acquired By: | System |
| Sample Type: | Unknown | Sample Set Name: | fraccionam |
| Vial: | 5 | Acq. Method Set: | Isocratico 075 mL MS |
| Injection #: | 1 | Processing Method | ApexTrack_BioSuite_Precol_IgG |
| Injection Volume: | 75.00 ul | Channel Name: | 2487Channel 1 |
| Run Time: | 32.0 Minutes | Proc. Chnl. Descr.: | 280 nm |
| | | | |
| Date Acquired: | 8/13/2014 11:33:47 PM CDT | | |
| Date Processed: | 8/14/2014 7:06:29 AM CDT | | |

| | Peak Name | RT | Area | % Area | Height |
|---|---|---|---|---|---|
| 1 | | 8.050 | 16814 | 0.10 | 947 |
| 2 | | 8.437 | 63344 | 0.38 | 1782 |
| 3 | | 9.477 | 63693 | 0.38 | 1249 |
| 4 | | 10.848 | 144160 | 0.87 | 2827 |
| 5 | IgG | 11.735 | | | |
| 6 | F(ab')2 | 12.769 | 16354582 | 98.16 | 247045 |
| 7 | Fab | 14.836 | | | |

| | Peak Name | RT | Area | % Area | Height |
|---|---|---|---|---|---|
| 8 | | 18.500 | 5028 | 0.03 | -269 |
| 9 | | 19.210 | 4476 | 0.03 | 148 |
| 10 | | 23.614 | 9313 | 0.06 | 199 |

**Figure 4g**

**Figure 4h**

<table>
<tr><th>SAMPLE INFORMATION</th></tr>
</table>

| | SAMPLE INFORMATION | |
|---|---|---|

Sample Name: B110814    Acquired By: System
Sample Type: Unknown    Sample Set Name: fraccionamientos_Plasma
Vial: 28    Acq. Method Set: Isocratico 075 mL MS
Injection #: 1    Processing Method ApexTrack_BioSuite_Precol_IgG
Injection Volume: 75.00 ul    Channel Name: 2487Channel 1
Run Time: 32.0 Minutes    Proc. Chnl. Descr.: 280 nm

Date Acquired: 8/13/2014 4:26:17 AM CDT
Date Processed: 8/13/2014 8:44:35 AM CDT

| | Peak Name | RT | Area | % Area | Height |
|---|---|---|---|---|---|
| 1 | | 8.288 | 7647 | 0.05 | 241 |
| 2 | | 10.845 | 68807 | 0.42 | 1629 |
| 3 | IgG | 11.735 | | | |
| 4 | F(ab')2 | 12.759 | 16286082 | 99.31 | 245547 |
| 5 | Fab | 14.836 | | | |
| 6 | | 18.500 | 6083 | 0.04 | -359 |
| 7 | | 20.427 | 4011 | 0.02 | 151 |
| 8 | | 21.539 | 5369 | 0.03 | 155 |
| 9 | | 23.543 | 20646 | 0.13 | 346 |

**Figure 4i**

**Figure 4j**

**Figure 4k**

| | Peak Name | RT | Area | % Area | Height |
|---|---|---|---|---|---|
| 1 | | 10.832 | 98518 | 0.66 | 2236 |
| 2 | IgG | 11.735 | | | |
| 3 | F(ab')2 | 12.761 | 14842207 | 99.06 | 220584 |
| 4 | Fab | 14.836 | | | |
| 5 | | 18.500 | 5756 | 0.04 | -310 |
| 6 | | 19.151 | 4913 | 0.03 | 119 |
| 7 | | 23.595 | 17376 | 0.12 | 336 |
| 8 | | 24.824 | 14182 | 0.09 | 350 |

**Figure 4I**

| | | SAMPLE INFORMATION |
|---|---|---|

| | | | |
|---|---|---|---|
| Sample Name: | F120814 | Acquired By: | System |
| Sample Type: | Unknown | Sample Set Name: | fraccionam |
| Vial: | 8 | Acq. Method Set: | Isocratico 075 mL MS |
| Injection #: | 1 | Processing Method | ApexTrack_BioSuite_Precol_IgG |
| Injection Volume: | 75.00 ul | Channel Name: | 2487Channel 1 |
| Run Time: | 32.0 Minutes | Proc. Chnl. Descr.: | 280 nm |
| | | | |
| Date Acquired: | 8/14/2014 1:48:23 AM CDT | | |
| Date Processed: | 8/14/2014 7:06:29 AM CDT | | |

| | Peak Name | RT | Area | % Area | Height |
|---|---|---|---|---|---|
| 1 | | 8.320 | 7221 | 0.05 | 303 |
| 2 | | 10.837 | 96626 | 0.64 | 2254 |
| 3 | IgG | 11.735 | | | |
| 4 | F(ab')2 | 12.760 | 14890285 | 99.12 | 221295 |
| 5 | Fab | 14.836 | | | |
| 6 | | 18.497 | 8169 | 0.05 | -379 |
| 7 | | 23.581 | 20634 | 0.14 | 349 |

**Figure 4m**

**Figure 5**

**Figure 6**

**Figure 7**

**Figure 8**

**Figure 9**

# INTERNATIONAL SEARCH REPORT

International application No.

PCT/MX2017/000115

## A. CLASSIFICATION OF SUBJECT MATTER

*C07K16/18* (2006.01)

According to International Patent Classification (IPC) or to both national classification and IPC

## B. FIELDS SEARCHED

Minimum documentation searched (classification system followed by classification symbols)

C07K

Documentation searched other than minimum documentation to the extent that such documents are included in the fields searched

Electronic data base consulted during the international search (name of data base and, where practicable, search terms used)

EPODOC, INVENES, WPI, MEDLINE, NPL, EMBASE, BIOSIS

## C. DOCUMENTS CONSIDERED TO BE RELEVANT

| Category* | Citation of document, with indication, where appropriate, of the relevant passages | Relevant to claim No. |
|---|---|---|
| X | WHO Guidelines for the Production, Control and Regulation of Snake Antivenom Immunoglobulins. WHO Expert Committee on Biological Standardization, 59th meeting, Geneva (13 to 17 October 2008), 2010 pages 47, 48, 54, 64 | 1-7, 12, 13 |
| X | SOLANO RODRÍGUEZ L. Purificación del suero antiviperino polivalente utilizando ácido caprílico (AC) solo and/o en combinación con cromatografía de intercambio iónico y su comparación con el método Birmex tradicional. Tesis para obtener el grado de maestra en ciencias de la salud con área de concentración en vacunología, 12/2012 pages 10 and 11 | 1-7, 12, 13 |

☒ Further documents are listed in the continuation of Box C.      ☒ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance. | |
| "E" earlier document but published on or after the international filing date | |
| "L" document which may throw doubts on priority claim(s) or which is cited to establish the publication date of another citation or other special reason (as specified) | "X" document of particular relevance; the claimed invention cannot be considered novel or cannot be considered to involve an inventive step when the document is taken alone |
| "O" document referring to an oral disclosure use, exhibition, or other means. | "Y" document of particular relevance; the claimed invention cannot be considered to involve an inventive step when the document is combined with one or more other documents , such combination being obvious to a person skilled in the art |
| "P" document published prior to the international filing date but later than the priority date claimed | |
| | "&" document member of the same patent family |

| Date of the actual completion of the international search | Date of mailing of the international search report |
|---|---|
| 18/05/2018 | **(29/05/2018)** |

| Name and mailing address of the ISA/ | Authorized officer |
|---|---|
| OFICINA ESPAÑOLA DE PATENTES Y MARCAS<br>Paseo de la Castellana, 75 - 28071 Madrid (España)<br>Facsimile No.: 91 349 53 04 | M. Cumbreño Galindo<br><br>Telephone No. 91 3496880 |

Form PCT/ISA/210 (second sheet) (January 2015)

## INTERNATIONAL SEARCH REPORT

| | International application No. |
|---|---|
| | PCT/MX2017/000115 |

| C (continuation). | | DOCUMENTS CONSIDERED TO BE RELEVANT | |
|---|---|---|---|
| Category * | Citation of documents, with indication, where appropriate, of the relevant passages | | Relevant to claim No. |
| X | BURNOUF T. et al. Assessment of the viral safety of antivenoms fractionated from equine plasma. Biologicals, 2004, Vol. 32, Pages 115-128 page 120; table 2 | | 1-7, 12, 13 |
| X | HARMS A. J. The Purification of Antitoxic Plasmas by Enzyme Treatment and Heat Denaturation. Biochem J, 1948, Vol. 42, N° 3, Pages 390-397 the whole document | | 1-7, 12, 13 |
| X | ES 2549690 A2 (INOSAN BIOPHARMA S A) 30/10/2015, the whole document | | 1-7, 12, 13 |
| A | ES 2345245T T3 (INST BIOCLON S A DE CV) 20/09/2010, the whole document | | 1-7, 12, 13 |

Form PCT/ISA/210 (continuation of second sheet) (January 2015)

## INTERNATIONAL SEARCH REPORT

Information on patent family members

International application No.

PCT/MX2017/000115

| Patent document cited in the search report | Publication date | Patent family member(s) | Publication date |
|---|---|---|---|
| ES2549690 A2 | 30.10.2015 | US2016368969 A1 | 22.12.2016 |
| | | FR2997084 A1 | 25.04.2014 |
| | | MX2012012132 A | 08.12.2013 |
| ES2345245T T3 | 20.09.2010 | HK1063057 A1 | 16.12.2011 |
| | | US2012064063 A1 | 15.03.2012 |
| | | US8512706 B2 | 20.08.2013 |
| | | DK1374895T T3 | 11.10.2010 |
| | | AT472337T T | 15.07.2010 |
| | | US2009142356 A1 | 04.06.2009 |
| | | US8075893 B2 | 13.12.2011 |
| | | US2002164327 A1 | 07.11.2002 |
| | | US6709655 B2 | 23.03.2004 |
| | | US2004166107 A1 | 26.08.2004 |
| | | US7485303 B2 | 03.02.2009 |
| | | MXPA03007992 A | 29.09.2004 |
| | | WO02068475 A2 | 06.09.2002 |
| | | WO02068475 A3 | 31.10.2002 |
| | | CA2443391 A1 | 06.09.2002 |
| | | CA2443391 C | 24.04.2012 |
| | | AU2002237586 A1 | 12.09.2002 |
| | | AU2002237586B B2 | 21.12.2006 |
| | | EP1374895 A2 | 02.01.2004 |
| | | EP1374895 B1 | 30.06.2010 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 6709655 B
- US 4849352 A
- US 8048414 B, Sullivan
- US 5733742 A, Landon
- US 4814433 A
- CN 103864930
- CN 101816789

- ES 2549690
- US 8512706 B
- US 7485303 B
- US 8075893 B
- US 7537916 B, Jones
- US 6706655 B
- ES 25496902015, Lopez de Silanes

### Non-patent literature cited in the description

- **HEINEN, A.B.** *Gorini da Veiga / Toxicon,* 2011, vol. 57, 497-511
- **CALVETE, JJ et al.** *Bitis gabonica gabonica,* 2007
- **AHRER K. ; BUCHACHER A. ; IBERER G. ; JUNG-BAUER A.** Effects of ultra-/diafiltration conditions on present aggregates in human immunoglobulin G preparations. *Journal of Membrane Science,* 2006, vol. 274, 108-115
- **ALVARENGA, L. M. ; ZAHID, M. ; DI TOMMASO, A. ; JUSTE, M. O. ; AUBREY, N. ; BILLIALD, P. et al.** Engineering Venom's Toxin-Neutralizing Antibody Fragments and Its Therapeutic Potential. *Toxins,* 2014, vol. 6, 2541-2537
- **CALMETTE.** The treatment of animals poisoned with snake venom by the injection of antivenomous serum. *Br. Med. J.,* 1896, vol. 2, 399-400
- **CALVETE, J.J. ; JUÁREZ, P. ; SANZ, L.** Snake venomics. Strategy and applications. *J. Mass Spectrom.,* 2007, vol. 42, 1405-1414
- **CHEN X1 ; JENSEN PE.** The role of B lymphocytes as antigen-presenting cells. *Arch Immunol Ther Exp (Warsz),* March 2008, vol. 56 (2), 77-83
- **CHEN, JENSEN P. E.** The role of B lymphocytes as antigen-presenting cells. *Arch Immunol Ther Exp (Warsz),* March 2008, vol. 56 (2), 77-83
- **CHIPPAUX JP.** Snake-bites: appraisal of the global situation. *Bulletin of the World Health Organization,* 1998, vol. 76 (5), 515-24
- **DE LIMA M.E. ; MARTIN-EAUCLAIRE M.F. ; CHAVEZ-OLORTEGUI C. ; DINIZ C.R. ; GRANIER C.** Tityus serrulatus scorpion venom toxins display a complex pattern of antigenic reactivity. *Toxicon,* February 1993, vol. 31 (2), 223-7

- FEUM0 Farmacopea de los Estados Unidos Mexicanos, Secretaría de Salud. Y Especificación interna del Instituto Bioclon SA de CV, 2011, 279-293, 2367-2369
- FEUM1 Farmacopea de los Estados Unidos Mexicanos, Secretaría de Salud. 2011, 115-116, 449, , 450, , 2316, , 2367-2369
- FEUM2 Farmacopea de los Estados Unidos Mexicanos, Secretaría de Salud. 2011, 2292, , 2293, , 2316, , 2367-2369
- FEUM3 Farmacopea de los Estados Unidos Mexicanos, Secretaría de Salud. 2011, 2367-2369
- MPB0260. Determinación de cresol y fenol en productos biologicos. FEUM4 Farmacopea de los Estados Unidos Mexicanos, Secretaría de Salud. 2011, 2435
- **FOX S ; RATHUWITHANA AC ; KASTURIRATNE A ; LALLOO DG ; DE SILVA, HJ.** Underestimation of snakebite mortality by hospital statistics in the Monaragala District of Sri Lanka. *Transactions of the Royal Society of Tropical Medicine and Hygiene,* 2006, vol. 100 (7), 693-5
- **GERRINGA D. ; KINGB T.R. ; BRANTONC, R.** Validating a faster method for reconstitution of Crotalidae Polyvalent Immune Fab (ovine). *Toxicon,* July 2013, vol. 69, 42-49
- **GARAVITO, A. ; ESPÍNDOLA, M.** Proceso de Concentración de Proteína de Suero de Leche por Ultrafiltración. Proyecto de Grado para optar al título de Ingeniero Químico. *Planta de Vegetales - ICTA, Universidad Nacional de Colombia,* 1995
- **GHOSH, R. ; CUI, Z.F.** Protein purification by ultrafiltration with pre-treated membrane. *Journal of Membrane Science,* 14 March 2000, vol. 167 (1), 47-53

- **GUTIÉRREZ JM ; THEAKSTON RDG ; WARRELL DA.** Confronting the neglected problem of snake bite envenoming: the need for a global partnership. *PLoS-Medicine,* 06 June 2006, vol. 3 (6

- **GWEE, M.C.E. ; NIRTHANAN, S. ; KHOO, H.E. ; GOPALAKRIHNAKONE, P. ; KINI, R.M. ; CHEAH, L.S.** Autonomic effects of some scorpion venoms and toxins. *Clin. Exp. Pharm. Physiol.,* 2002, vol. 29, 795-801

- **HARMS A.J.** The purification of antitoxic plasmas by enzyme treatment and heat denaturation. *Biochem J.,* 1948, vol. 42, 390-397

- **HEINEN T. E. ; GORINI DA VEIGA A. B.** Review Arthropod venoms and cancer. *Toxicon,* 2011, vol. 57, 497-511

- **HEMKE, V.** *Journal of Cell and Tissue research,* vol. 13 (1), 3479-3484

- **LANDON J. et al.** Single-reagent one-step procedures for the purification of ovine IgG, F(ab')2 and Fab antivenoms by caprylic acid. *Journal of Immunological Methods,* 2014, vol. 402, 15-22

- **LANDON ; JONES.** Enhanced pepsin digestion: a novel process for purifying antibody F(ab')2 fragments in high yield from serum. *Journal of Immunological Methods,* 2002, vol. 263, 57-74

- **LANDON ; JONES.** A protocol for 'enhanced pepsin digestion': a step by step method for obtaining pure antibody fragments in high yield from serum. *Journal of Immunological Methods,* 2003, vol. 275, 239-250

- **LOURENÇO, W.R.** Diversity and endemism in tropical versus temperate scorpion communities. *Biogeographica,* 1994, vol. 70, 155-160

- Scorpion neurotoxins: effects and mechanisms. **MARTIN-EAUCLAIRE, M.F. ; COURAUD, F.** Handbook of Neurotoxicology. Marcell and Dekker, 1995, 683-716

- Common Antigens in Snake Venoms. Capítulo: Venenos de serpientes. **MINTONJR. S. A.** Handbook of Experimental Pharmacology. vol. 52, 847-862

- **MITCHELL ; SILAS WEIR ; EDWARD T. REICHERT.** Researches Upon the Venoms of Poisonous Serpents. *Washington: Smithsonian Institution*

- **POPE C.G.** Dissagregation of proteins by enzymes. *Br J Exp Path,* 1938, 245-251

- **POPE, G.G.** The action of proteolytic enzymes on antitoxins and proteins of immune sera. I. True digestion of the protein. *Br. J. Exp. Pathol.,* 1939, vol. 20, 132-149

- **POPE, G.G.** The action of proteolytic enzymes on antitoxins and proteins of immune ser. II. Heat denaturation after partial enzyme action. *Br. J. Exp. Pathol.,* 1939, vol. 20, 201-212

- **POSSANI, L.D. ; MERINO, E. ; CORONA, M. ; BOLIVAR, F. ; BECERRIL, B.** Peptides and genes coding for scorpion toxins that affect ion-channels. *Biochimie,* 2000, vol. 82, 861-868

- **PUGH RN ; THEAKSTON RD ; REID HA.** Malumfashi Endemic Diseases Research Project, XIII. Epidemiology of human encounters with the spitting cobra, Naja nigricollis, in the Malumfashi area of northern Nigeria. *Annals of Tropical Medicine and Parasitology,* 1980, vol. 74 (5), 523-30

- **RODRIGUEZ-PINTO.** *Review. B cells as antigen presenting cells Cellular Immunology,* 2005, vol. 238, 67-75, www.elsevier.com/locate/ycimm

- **SAETANG, T. ; TREAMWATTANA, N. ; SUTTIJITPAISAL, P. ; RATANABANANGKOON, K.** Quantitative comparison on the refinement of horse antivenom by salt fractionation and ion-exchange chromatography. *J. Chromatogr., B: Biomed. Sci. Appl.,* 1997, vol. 700, 233

- **SHARMA SK et al.** Impact of snake bites and determinants of fatal outcomes in southeastern Nepal. *American Journal of Tropical Medicine and Hygiene,* 2004, vol. 71 (2), 234-238

- **SIMARD, M.J. ; WATT, D.D.** The Biology of Scorpions. Stanford University Press, 1990, 414-444

- **SNOW RW et al.** The prevalence and morbidity of snake bite and treatment-seeking behaviour among a rural Kenyan population. *Annals of Tropical Medicine and Parasitology,* 1994, vol. 88 (6), 665-71

- **WARRELL DA ; ARNETT C.** The importance of bites by the saw-scaled or carpet viper (Echis carinatus): epidemiological studies in Nigeria and a review of the world literature. *Acta Tropica,* 1976, vol. 33 (4), 307-41

- **WOLFENDEN, R. N.** On the nature and action of the venom of poisonous snakes. *J. Physiol. (London.),* vol. 7, 326-324

- **WILLIAMS, D. ; GUTIÉRREZ, J.-M. ; CALVETE, J. ; WÜSTER, W. ; RATANABANANGKOON, K. ; PAIVA, O.** Ending the drought: New strategies for improving the flow of affordable, effective antivenoms in Asia and Africa. *Journal of Proteomics,* 2011, vol. 74, 1735-1767